# EUROPÄISCHE PATENTSCHRIFT

(11) **EP 1 948 196 B1**
(45) Veröffentlichungstag und Bekanntmachung des Hinweises auf die Patenterteilung: **26.12.2012**
(21) Anmeldenummer: 06818051.2
(22) Anmeldetag: 02.11.2006
(51) Int. Cl.: A61K 31/695, A61K 31/7028, A61P 31/04, A61P 31/12, A61P 35/00

(54) **VERWENDUNG VON TETRAORGANOSILIZIUM-VERBINDUNGEN**
USE OF TETRAORGANOSILICON COMPOUNDS
UTILISATION DE COMPOSES TETRAORGANOSILICIUM

(30) Priorität: 03.11.2005 DE 102005053011; 09.12.2005 DE 102005059750; 03.04.2006 US 744191 P
(43) Veröffentlichungstag der Anmeldung: 30.07.2008
(73) Patentinhaber: Salama, Zoser B., 13465 Berlin (DE)
(72) Erfinder: Salama, Zoser B., 13465 Berlin (DE)
(74) Vertreter: Boeckh, Tobias
(86) Internationale Anmeldenummer: PCT/DE2006/001948
(87) Internationale Veröffentlichungsnummer: WO 2007/051462

(56) Entgegenhaltungen:
- EP-A1- 0 483 465

## Beschreibung

Die Erfindung betrifft die Verwendung von Tetraorganosilizium-Verbindungen der allgemeinen Formel 1 zur Behandlung von Tumor-, Bakterien - und/oder Viruserkrankungen.

Die Erfindung betrifft insbesondere eine Anspruch1 und Kombinations-Erfindung, bei der aus jeweils zwei bzw. drei Listen spezielle Paare so ausgewählt und kombiniert werden, dass ein unerwarteter Effekt auftritt, wobei Tetraorganosilizium-Verbindungen zur Behandlung von Erkrankungen ausgewählt werden, die durch eine einheitliche Wirkung der molekularen Muster eines Pathogens ausgewählt aus der Gruppe der entarteten Zellen, Viren oder Bakterien bzw. durch den einheitlichen Effekt der Reaktion des Immunsystems hierauf charakterisiert werden und wobei die ausgewählten Tetraorganosilizium-Verbindungen als Immuntherapeutikum, bevorzugt mit Verbindungen ausgewählt aus der Gruppe von Substanzen, die an Nukleinsäuren binden und die Synthese dieser - insbesondere die DNA-Synthese oder RNA-Synthese in einem Organsismus beispielsweise in einer Krebszelle oder die DNA bzw. RNA-Synthese in einem Virus oder Bakterium - hemmen, umfassend Oxoplatin, Arsen-Oxide, Foscamet und/oder Antisense-Moleküle verwendet werden.

Seit mehreren Jahrzehnten geht die Fachwelt davon aus, dass sich die Nebenwirkungen von Arzneistoffen reduzieren lassen, wenn diese im Körper möglichst lange stabil verbleiben und sicher und zielgerichtet an ihren Wirkort verbracht werden können. Vor 50 Jahren begann man, Mischungen von Phospholipiden und Wasser herzustellen, die geschlossene Vesikel darstellten. Erste Arzneistoffe, die in Vesikeln eingeschlossen wurden, sind bereits kommerziell erhältlich. Wenn Vesikel aus einem wässrigen Kern und mindestens einer Hülle aus Phospholipid-Doppelmembranen bestehen, werden sie als Liposomen bezeichnet. Die Liposomen weisen allerdings den Nachteil auf, dass sie wegen der mangelnden Stabilität der Doppelmembran zahlreiche Anteile des Arzneimittels verlieren und so einer Modifikation des Arzneimittels innerhalb des Körpers nicht entgegenwirken. Weiterhin hat sich gezeigt, dass sich die ursprüngliche Hoffnung, dass das Arzneimittel und die Vesikel eine Krankheit auf verschiedene Art und Weise lindern - d. h. also indem die Vesikel ebenfalls eine therapeutische Wirkung haben - nicht erfüllt. Die Liposomen sind aber nicht nur innerhalb des Körpers instabil, sondern auch außerhalb des Körpers, so beispielsweise bei einer thermischen Behandlung. In der Praxis bedeutet das, dass ein Liposom, welches eine Arzneistoffzubereitung umfasst, nicht mehr autoklaviert werden kann. Aus diesem Grunde gibt es im Stand der Technik Bestrebungen, Partikel bereitzustellen, die eine verbesserte Lagerstabilität sowie eine erhöhte thermische Stabilität aufweisen und die weiterhin eine eigenständige therapeutische Wirkung besitzen. Im Stand der Technik gab es, um derartige Nachteile zu beheben, Versuche, Selen-, Silizium-, Germanium-, Phosphor- oder Kohlenstoff-haltige Systeme bereitzustellen. Derartige Systeme können als small unilamellar vesicles, als multilammelar vesicles, als large unilamellar vesicles bzw. als oligolamellar vesicles oder multivesicular liposomes bereitgestellt werden, wobei diese Systeme entweder gemäß ihrer Größe als auch nach der Anzahl an bimolekularen Membranen charakterisiert werden können. Verbindungen, die Systeme auf Selen-, Silizium-, Germanium-Basis sein können, sind beispielsweise Cubosomen, Novosomen, Sphingosomen, Galaktosomen, Transferosomen, Niosomen, Siosomen, Smarticles, Cagicles, Nanocapsules, Micromethason und einige Hunderte mehr, wobei sich jedes der genannten Systeme in mehrere Tausende Untersysteme aufsplitten lässt. Die genannte Verbindungen sind vor allem Gegenstand von aktuellen Forschungen in Hochschuleinrichtungen. Da für die entsprechenden Projekte Forschungsmittel beantragt werden müssen, gibt es zahlreiche Anträge, in denen vorteilhafte Wirkungen der genannten Systeme bei allen Zivilisationskrankheiten behauptet, aber in der Regel nicht konkret dargelegt werden. Standardmäßig wird ausgeführt, dass die üblichen Medikamente, die gegen Alzheimer, Parkinson, Krebs, Autoimmunerkrankungen, Fettsucht, Herz- und/oder Kreislauferkrankungen eingesetzt werden, vorteilhaft verwendet werden können, wenn diese in Cubosomen, Novosomen, Sphingosomen, Galaktosomen, Transferosomen, Niosomen, Siosomen, Smarticles, Cagicles, Nanocapsules und/oder Micromethason gegebenenfalls auf Germanium-, Silizium- oder Selen-Basis eingebracht werden. Verifizierbare oder belastbare Nachweise in Form von konkreten Verbindungen fehlen allerdings. Die genannten Lehren des Standes der Technik sind nicht nacharbeitbar, da aus dem breiten Bereich der angegebenen Systeme oder der angegebenen Mittel gegen die Zivilisationskrankheiten keine konkreten Verbindungen offenbart werden. Demgemäß fehlen auch technische Lehren mit ausgewählten Verbindungen - von der Liposomen- oder Armeimittelseite her -, die besonders gute, überraschende Eigenschaften aufweisen würden. Es ist daher auch nicht bekannt, dass der so genannte "glückliche Griff" gelungen ist, bei dem aus einer Vielzahl von Möglichkeiten eine bestimmte gewählt wird, deren Ergebnis nicht vorausgesagt werden konnte. Mit den bekannten Systemen des Standes der Technik wurde keine Leistungssteigerung, keine Vorteile, wie Verbilligung oder Vereinfachung oder Ersparnis an Zeit, Material und Arbeitsstoffen, Kosten oder schwer zu beschaffenden Arzneimittel erreicht. Auch fehlt es an einer erhöhten Zuverfässigkeit, Qualitätshebung, einer größeren Effektivität oder an wertvollen oder überlegenen Eigenschaften; im Prinzip gelang also keine Vermehrung der technischen Möglichkeiten oder die Bereitstellung von weiteren Mitteln oder die Eröffnung von weiteren alternativen Wegen auf dem Gebiet der Therapie. Weder wurden Reservemittel oder Alternativen bereitgestellt bzw. der Arzneimittelschatz bereichert. Die bisher durch Routineversuche bereitgestellten, eingeschlossenen Arzneimittel zeigen keine Verbesserung gegenüber dem Stand der Technik. Trotz der zahlreichen behaupteten positiven Wirkungen der o. g. Systeme ist es so, dass diese nach einem gewissen Zeitraum durch die Fachwelt nicht mehr aktiv bearbeitet werden, da keine besonderen Wirkungen erzielt werden können.

Aufgabe der Erfindung ist es daher, die Nachteile des Standes der Technik zu beseitigen und aus dem größeren bekannten Bereich einen nicht ausdrücklich erwähnten Teilbereich gezielt auszuwählen, für den im Vergleich zum größeren Bereich besondere Wirkungen, Eigenschaften, Vorteile und Effekte dargelegt werden können. Hierbei ist zu berücksichtigen, dass der Stand der Technik sich insbesondere durch seine Größe, Unüberschaubarkeit und Heterogenität charakterisiert. Demgegenüber soll durch eine gezielte, nicht willkürliche Auswahl, die die Fachwelt noch nicht ernsthaft erwogen hatte, ein ausgewählter Bereich mit wertvollen, überraschenden Eigenschaften offenbart und charakterisiert werden. Die überraschenden, nicht nahe liegenden Eigenschaften betreffen nicht nur den besonders sicheren und effektiven Einschluss von ausgewählten Arzneimitteln, die sich aufgrund ihrer völlig überraschenden Interaktion mit dem System besonders sicher und effektiv einschließen lassen, sondern sie betrifft auch die überraschende Wirkung der ausgewählten Systeme an sich - d. h. auch ohne ausgewählte Arzneimittel - in Bezug auf gezielt ausgewählte Erkrankungen.

Völlig überraschend hat sich gezeigt, dass durch die Auswahl aus verschiedenen Listen, die entweder verschiedene liposomale Systeme bzw. verschiedene Erkrankungen oder verschiedene ausgewählte Armeimittel betreffen, spezielle Paare aus den jeweils mindestens zwei bzw. drei Listen zusammengestellt werden können, die überraschende Eigenschaften aufweisen. Die anmeldungsgemäße Lehre ist daher auch eine Kombinationserfindung, bei der mehrere ausgewählte Elemente zur Erreichung eines technischen Gesamterfolges zusammenwirken, wobei ein unerwarteter synergistischer Effekt auftritt. Es kommt aufgrund der erfindungsgemäßen Kombination zu einer funktionellen Wechselwirkung der ausgewählten Elemente. Die in der Erfindung vereinigten, kombinierten, ausgewählten Elemente wirken gemeinsam auf ein einheitliches Ziel hin, d. h. sie kooperieren. Der einheitliche technische, überraschende Erfolg, der auf den Wirkungen der ausgewählten Elemente beruht, ist sozusagen die Kammer, die die patentfähige Kombination charakterisiert. Der Stand der Technik gab dem Durchschnittsfachmann keine Anregung, gerade die spezifisch ausgewählten Elemente zusammenwirken zu lassen. Der durch das Zusammenwirken erzielte Gesamterfolg war unerwartet, da ein synergistischer Effekt entsteht Krankheiten, die bei Menschen oder Tieren auftreten können, lassen sich unter verschiedenen Gesichtspunkten charakterisieren und in Zuordnungssysteme einordnen; beispielsweise nach der Art der Erreger, nach Art der befallenen Organe, nach dem Schweregrad der Erkrankung oder nach der Art der Therapie, mit der sie behandelt werden können bzw. durch die Art der Diagnose, die es möglich macht, sie zu detektieren (beispielsweise durch chemische Reaktionen, durch elektronenmikroskopische Beobachtungen oder durch das Messen bestimmter Parameter). Einige Krankheiten des Menschen und z. T. auch von Tieren sind mit der initialen oder adaptiven Immunabwehr so verbunden, dass das Immunsystem charakteristische Merkmale einer pathogenen Ausprägung eines Virus, eines Parasiten oder eines entarteten Gewebes erkennt und auf diese spezifisch reagiert. D. h. derartige Krankheiten (Pathogen-assoziierte molekulare Muster Krankheiten (*pathogen-associated molecular patterns diseases,* PAMPD)) sind durch ein definiertes molekulares Muster charakterisiert, welches eine bestimmte Reaktion des Immunsystems induziert. Zu derartigen Reaktionen gehören Entzündungsreaktionen in einem Organismus, die durch Viren, Bakterien oder Parasiten bzw. durch entartete Zellen hervorgerufen werden. Solche Erkrankungen sind untereinander molekular verbunden, da sie die einheitliche Idee der molekularen Muster (auch: specific pathogen-associated molecular pattern) und der Reaktion des Immunsystems darauf verwirklichen. In der phylogenetischen Entwicklung von einfachen Lebewesen bis hin zu komplexen Organismen sind diese Abwehrstrategien gegen die o. g. Erkrankungen fast unverändert übernommen worden, weil sich die spezifischen molekularen Muster in der Phylogenese kaum veränderten. Diesen Erkrankungen ist beispielsweise ein typisches Entzündungsmuster eigen, welches beim Menschen durch eine Wahrnehmung der molekularen Muster durch Reaktionen von Granulozyten, Makrophagen, dendrititschen Zellen, Epitelzellen und natürlichen Killerzellen erfolgt. Das besondere klinisch-technische Merkmal dieser Krankheiten wird durch die genannten Reaktionen im Organismus und durch die Art der Interaktion von pathogenen Erregern bzw. ihrem molekularen Muster und dem initialen oder adaptiven Immunsystem charakterisiert und gegenüber Organismen, die derartige Krankheiten nicht aufweisen können (z. B. Insekten), abgegrenzt.

Es war überraschend, dass diese Erkrankungen, die durch eine einheitliche Wirkung der molekularen Muster bzw. durch den einheitlichen Effekt der Reaktion des Immunsystems charakterisiert werden, ausgewählt aus der Gruppe umfassend Tumor-, Bakterien- und Viruserkrankungen, durch Medikamente als Immuntherapeutika behandelt werden können, die hergestellt sind durch die Verwendung von Verbindungen der allgemeinen Formel I 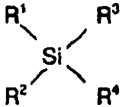 in der R¹ und R², die gleich oder verschieden sein können, jeweils einen Acyloxyrest der Formel R-COO- oder einen Peptidrest der Formel 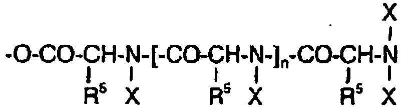 bedeuten, wobei R einen unverzweigten oder verzweigten Alkyl-, Alkenyl- oder Alkinylrest mit 5 bis 29 C-Atomen bedeutet, die durch ein bis drei Halogenatome, Alkoxyreste mit 1 bis 18 C-Atomen oder Aminogruppen substituiert sein können, die Reste R⁵, die gleich oder verschieden sein können, den nach der Entfernung der Gruppe 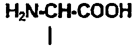 von einer in der Natur vorkommenden Aminosäure verbleibenden Rest darstellen, die Reste X, die gleich oder verschieden sein können, ein Wasserstoffatom oder eine in der Peptidchemie übliche Aminoschutz-gruppe darstellen und n eine ganze Zahl von 1 bis 12 bedeutet, und R³ und R⁴, die gleich oder verschieden sein können, jeweils einen Alkylrest mit 1 bis 6 C-Atomen, eine Arylgruppe mit 3 bis 14 C-Atomen, beispielsweise eine Phenylgruppe, den nach Entfernung eines Wasserstoffatoms verbleibenden Rest eines Monosaccharids, Disaccharids, Aminozuckers oder einer Hydroxycarbonsäure, einen Alkoxyrest mit 1 bis 5 C-Atomen, einen Acylrest, einer in der Natur vorkommenden Aminosäure mit freier oder geschützter Aminogruppe oder einen Di-, Tri- oder Tetrapeptidrest aus einer in der Natur vorkommenden Aminosäure mit freien oder geschützten Aminogruppen darstellen oder die Bedeutung R¹ und R² haben, wobei R einen unverzweigten oder verzweigten Alkyl-, Alkenyl-, oder Alkinylrest darstellt, die in einer bevorzugten Ausführungsform Verbindungen umfassen, ausgewählt aus der Gruppe von Substanzen, die an Nucleinsäuren binden und insbesndere die Synthese hemmen, umfassend Oxoplatin, Arsen-Oxide und/oder Foscamet.

Ähnliche chemische Verbindungen werden in der EP 0483465 beschrieben. Die Verbindungen gemäß EP 0483465 werden jedoch nur zur Herstellung von Vesikeln (Siosomen) verwendet. Die Verwendung solcher ähnlichen Vangkettiger silane-Verbindungen zur Behandlung von Tumor - Bakterien - und/oder Viruserkrankungen wird weder offenbart nach nahegelegt.

Es war völlig überraschend, dass die genannten Verbindungen eingesetzt werden können, um die o. g. spezifischen molekularen Muster von Krankheiten ausgewählt aus der Gruppe Tumorerkrankungen und Virenerkrankungen sowie bakterielle Erkrankungen zu behandeln. Hierbei handelt es sich um Erkrankungen, die im Sinne der Erfindung mit einer Defizienz des zellulären Immunsystems assoziiert sind.

In einer weiteren Ausführungsform der Erfindung ist es vorgesehen, dass die genannten Verbindungen verwendet werden, um Liposomen bevorzugt ohne Phospholipid bereitzustellen, die vollständig oder zum Teil aus den genannten Verbindungen gemäß Formel I bestehen. Sowohl die Verbindungen gemäß Formel I als auch liposomale Strukturen, die diese umfassen, können zur Behandlung der o. g. Krankheiten eingesetzt werden. Die Wirkung dieser Mittel scheint darauf zurückzuführen sein, dass die Reaktion des initialen und des adaptiven Immunsystems auf die o. g. molekularen Muster so verstärkt wird, dass pathogene Ereignisse, die diese Muster hervorrufen, wie z. B. entartete Zellen oder Viren bzw. Bakterien mit den Verbindungen oder Mitteln so interagieren, dass eine Therapie eines Organismus überraschend möglich ist.

Besonders überraschend war es, dass die Verbindungen gemäß Formel I bzw. vesikuläre Strukturen, die diese umfassen, insbesondere wenn sie mit Oxoplatin, Arsen-Oxiden, Foscamet und/oder Antissense-Konstrukte assoziiert sind, die Metastasierung von Tumoren verhindern. Die Wirkung der erfindungsgemäßen Verbindungen oder Systeme ist überraschenderweise viel höher, als wenn Oxoplatin, Arsen-Oxide und/oder Foscarnet bzw. Antisense-Konstrukte allein oder in Phospholipid-Vesikel eingeschlossen verwendet werden. Nur Vesikel, die Verbindungen gemäß der Formen I umfassen, haben diese besondere Wirkung auf entartete Zellen, insbesondere auf ihr Metastasierungsverhalten.

Metastasierungsverhalten bzw. Metastasierung im Sinne der Erfindung bedeutet jede Ausbildung einer Tochtergeschwulst. Im engeren Sinne bedeutet dies die Absiedlung und/oder Wanderung eines Tumors in entferntem und/oder näherem Gewebe aber auch die Streuung von entarteten Zellen, bevorzugt in verschiedene zahlreiche Organe. Auch eine direkte Nachbargeschwulst ist eine metastasierte Geschwulst im Sinne der Erfindung. Auch die Wanderung von Zellen und Zellverbänden oder das Abschilfern dieser ist Metastasierung im Sinne der Erfindung.

Vorteilhafterweise können die Verbindungen gemäß Formel I bzw. die vesikulären Systeme, die diese umfassen, in einem Organismus auch eingesetzt werden, um Viruserkrankungen zu behandeln. Überraschenderweise hat sich gezeigt, dass die Wirkung der erfindungsgemäßen Mittel deutlich erhöht werden kann, wenn diese mit einem Antisense-Molekül oder Foscarnet bzw. Arsen-Oxiden assoziiert sind. In diesem Falle lässt sich eine therapeutische Wirkung auf alle im Zusammenhang mit der erfindungsgemäßen Lehre offenbarten Krankheiten erreichen, die deutlich verbessert gegenüber einer solchen ist, die durch Antisense-Moleküle alleine hervorgerufen wird, oder wenn diese in Phospholipid-Vesikeln gemäß des Standes der Technik verpackt werden. Zu der Wirkung der Antisense-Moleküle wird folgendes ausgeführt: Die mRNA (messenger RNA) entsteht am kodierenden Strang des DNA-Matzritzenstrangs. Wird auch der komplementäre Strang abgelesen, entsteht eine zur mRNA komplementäre RNA: die Antisense-RNA. Verbinden sich Antisense-RNA und mRNA zu einem Doppelstrang, kann die mRNA nicht an Ribosomen abgelesen werden und es wird kein Protein gebildet. Darüber hinaus wird die doppelsträngige RNA von den RNA-Interferenz-Mechanismen (RNAi) rasch abgebaut Die RNA-Interferenz oder kurz RNAi ist ein Zellmechanismus, der die Expression von Genen in einer Zelle beeinflusst. Der Mechanismus wird von langen, doppelsträngigen RNA Molekülen ausgelöst und kleine (21 bis 23 Basenpaar lang) RNA-Moleküle, die man siRNA (small interfering RNA) nennt, kodieren die Information über die zu zerstörende Ziel-RNA. Es wird vermutet, dass die RNA-Interferenz unter anderem ein Schutzmechanismus der Zelle gegen "zellfremde" RNA, z.B. Virus-RNA, ist. Angriffspunkt der RNA-Interferenz ist die messenger RNA (mRNA). Taucht siRNA in der Zelle auf, reagiert die Zelle, in dem sie die gesamte mRNA zerstört, die dieselbe Nukleotid-Sequenz wie die siRNA besitzt. Dadurch werden die von dieser mRNA codierten Proteine nicht mehr produziert. Dem Fachmann sind weitere Methoden bekannt, um Viren zu bekämpfen. Alle Methoden der RNA-Interferenz (RNAi) können eingesetzt werden, um die ausgewählten Erkrankungen zu therapieren. Der Fachmann kann durch Routineversuche die spezifischen RNAi-Moleküle bereitstellen. Die Standardwerke auf diesem Gebiet sind demgemäß in den Offenbarungsgehalt dieser Lehre mit aufgenommen. Die Wirkung der Verwendung der Verbindungen gemäß der allgemeinen Formel I lässt sich verbessern durch eine Kombination mit einem weiteren Mittel ausgewählt aus der Gruppe umfassend Abacavir, Acyclovir, Adefovir, Amantadine, Amprenavir, Atazanavir, Codofovir, Darunavir, Delavirdine, Didanosine, Docosanol, Emtricitabine, Efavirenz, Enfuvirtide, Entecavir, Famciclovir, Foscamet, Fomivirsen, Fosamprenavir, Ganciclovir, Gardasil, Idoxuridine, Imiquimod, Indinavir, Interferon, Lamivudine, Lopinavir, Nevirapine, Nelfinavir, Oseltamivir, Penciclovir, Peramivir, Ribavirin, Rimantadine, Ritonavir, Saquinavir, Stavudine, Tenofovir, Tipranavir, Trifluridine, Tromantadine, Valaciclovir, Valganciclovir, Vidarabine, Viramidine, Zalcitabine, Zanamivir und/oder Zidovudine.

Beim Menschen können eine Vielzahl von Krankheiten durch Viren verursacht werden, die mit dem erfindungsgemäßen Mittel behandelt werden können. Selbstverständlich ist die Inhibierung der folgenden Viren in vivo wie in vitro möglich:

Das erfindungsgemäße Mittel kann insbesondere gegen behüllte Viren aber auch gegen unbehüllte Viren eingesetzt werden. Bei den behüllten Viren handelt es sich bevorzugt um:
**Doppelsträngige DNA-Viren = dsDNA**
   - Familie Poxviridae
      ○ Unterfamilie *Chordopoxvirinae*
         ■ Gattung *Orihopoxvirus*
            ■ Orthopox-Variola-Virus = Pockenvirus-Pocken
            ■ Orthopox-Alastrim-Virus - Weiße Pocken
         ■ Gattung *Parapoxvirus*
            ■ Parapox-Ovis-Virus = Orf-Virus - Orf = Schafpocken, bei Tieren, auf den Mensch übertragbar
         ■ Gattung *Molluscipoxvirus*
            ■ Molluscum-Contagiosum-Virus - Dellwarze *(Molluscum contagiosum*)
   - Familie Herpesviridae
      ○ Unterfamilie *Alphaherpesvirinae*
         ■ Gattung *Simplexvirus*
            ■ Herpes-simplex-Virus 1 (HSV 1) = Humanes-Herpes-Virus 1 (HHV 1) - Herpes simplex, Herpes labialis, Stomatitis aphtosa
            ■ Herpes-simplex-Virus 2 (HSV 2) = Humanes-Herpes-Virus 2 (HHV 2) - Herpes simplex, Herpes genitalis
            ■ Herpes-B-Virus = (Herpesvirus simiae)
         ■ Gattung Varicellovirus
            ■ Varizella-Zoster-Virus (VZV) = Humanes-Herpes-Virus 3 (HHV 3)-Windpocken = Varizellen (Herpes zoster), *Gürtelrose*
            ■ Pseudowut-Virus - Juckseuche = Tollkrätze, bei Tieren, auf den Menschen übertragbar!
      o Unterfamilie Betaherpesvirinae
         ■ Gattung *Cytomegalovirus*
            ■ Humanes-Zytomegalie-Virus = Humanes-Cytomegalie-Virus (HCMV) = Humanes-Herpes-Virus 5 (HHV 5) - Zytomegalie
         ■ Gattung *Reseolovirus*
            ■ Humane-Herpes-Virus 6(HHV 6) - Drei-Tage-Fieber
            ■ Humanes-Herpes-Virus 7 (HHV 7) - Drei-Tage-Fieber
      ○ Unterfamilie *Gammaherpesvirinae*
         ■ Gattung *Lymphocryptovirus*
            ■ Epstein-Barr-Virus (EBV) = Humanes-Herpes-Virus 4 (HHV 4) - Pfeiffer'sches Drüsenfieber, Burkitt-Lymphom
         ■ Gattung *Rhadinovirus*
            ■ Humanes-Herpes-Virus 8 (HHV 8)- Kaposi-Sarkom
      • Familie *Hepadnaviridae*
      ○ Gattung *Orthohepadnavirus*
         ■ Hepatitis-B-Virus (HBV) - Hepatitis B
**Einzel(+)-Strang-RNA-Viren = ss(+)RNA**
   • Familie Togaviridae
      ○ Gattung *Alphaviren -* Erreger von Arbovirosen
         ■ Chikungunya-Virus (CHIKV) - Chikungunya-Fieber
         ■ O'nyong'nyong-Virus (ONNV) - O'nyong-nyong-Fieber
      ○ Gattung *Rubiviren*
         ■ Rubivirus = Rötelvirus = Rubellavirus - Röteln
   • Familie Flaviviridae
      ○ Gattung *Hepacivirus*
         ■ Hepatitis-C-Virus (HCV) - Hepatitis C
         ■ GB-Virus-C (ohne Krankheitswert)
      ○ Gattung *Flavivirus*
         ■ West-Nil-Virus - West-Nil-Fieber
         ■ Dengue-Virus-Dengue-Fieber
         ■ Gelbfieber-Virus-Gelbfieber
         ■ Louping-ill-Virus-Louping-ill-Enzephalitis
         ■ St.-Louis-Enzephalitis-Virus - St.-Louis-Enzephalitis
         ■ Japan-B-Enzephalitis-Virus - Japanische Enzephalitis
         ■ Powassan-Virus - Powassan-Enzephalitis
         ■ RSSE-Virus - RSSE = Russian-Spring-Summer-Enzephalitis
         ■ FSME-Virus - FSME = Frühsommer-Meningoenzephalitis
   • Familie Coronaviridae - Magen-Darm-Entzündungen
      ○ Gattung *Coronavirus*
         ■ SARS-assoziiertes-Corona-Virus (SARS-CoV) - SARS = atypische Lungenentzündung = (Pneumonie).
         ■ Humanes-Corona-Virus 229E (HCoV 229E)- Erkältung
         ■ Humanes-Corona-Virus OC43 (HCoV OC43) - Erkältung
      ○ Gattung *Torovirus* - Gastroenteritis
   • Familie Retroviridae = Einzel(+)-Strang-RNA-Viren mit dsDNA-Zwischenstufe:
      ○ Unterfamilie *Orthoretrovirinae.*
         ■ Gattung *Deltaretrovirus*
            ■ Humanes-T-Zell-lymphotropes-Virus Typ I (HTLV-I) - Leukämie
            ■ Humanes_T-Zell-lymphotropes_Virus Typ II (HTLV-III) -Leukämie
         ■ Gattung *Lentivirus*
            ■ Humanes-Immundefizienz-Virus Typ 1 (HIV-1)-AIDS
            ■ Humanes-Immundefizienz-Virus Typ 2 (HIV-2) - AIDS
**Enzel(-)-Strang-RNA-Viren = ss(-)RNA**
   • Familie *Arenaviridae*
      ○ Gattung *Arenavirus*
         ■ Lassa-Virus - Lassa-Fieber
         ■ lymphozytäre-Chorio-Meningitis-Virus (LCMV)-Choriomeningitis
         ■ Tacaribe-Virus
         ■ Junin-Virus - Junin-Fieber (argentinisches hämorrhagisches Fieber)
         ■ Machupo-Virus - Machupo-Fieber (bolivianisches hämorrhagisches Fieber)
   • Familie Bornaviridae
      ○ Gattung *Bornavirus*
         ■ Virus der Boma'schen Krankheit - beim Pferd, vielleicht auch auf den Menschen übertragbar - Affektive Störungen
   • Familie Bunyaviridae - Erreger von Arbovirosen
      ○ Gattung *Orthobunyavirus*
         ■ Bunyamwera-Vrus (Serogruppe)
         ■ California-Encephalitis-Virus (Serogruppe) - Encephalitis
      ○ Gattung *Phlebovirus*
         ■ Rift-Valley-Fieber-Virus - 3 Subtypen, Rift-Tal-Fieber
         ■ Sandmückenfieber-Virus - *Sandfly fever=* Sandmückenfieber
            ■ Subtyp Toscana-Virus - Pappataci-Fieber
      ○ Gattung *Nairovirus*
         ■ Krim-Kongo-Fieber-Virus (Serogruppe):
            ■ Subtyp Krim-Kongo-hämorrhagisches-Fieber-Virus - Krim-Kongo-Fieber
            ■ Subtyp Hazara-Virus
            ■ Subtyp Khasan-Vrus
      ○ Gattung *Hantavirus*
         ■ Hantaan-Virus (4 Subtypen) hämorrhagisches Fieber, Nephritis
         ■ Seoul-Virus (Serogruppe) hämorrhagisches Fieber
         ■ Prospect-Hill-Vrus (2 Subtypen) hämorrhagisches Fieber
         ■ Puumala-Virus (Serogruppe)- hämorrhagisches Fieber, Pneumonie, Nephritis
         ■ Dobrava-Belgrad-Virus - hämorrhagisches Fieber
         ■ Tula-Virus - hämorrhagisches Fieber
         ■ Sin-Nombre-Virus (Serogruppe) - hämorrhagisches Fieber mit schwerem Lungenödem
   • Familie Filoviridae
      ○ Gattung Marburg-Virus
         ■ Lake-Victoria-Marburgvirus (Serogruppe) - Marburg-Fieber (hämorrhagisches Fieber)
      ○ Gattung Ebolavirus
         ■ Zaire-Ebolavirus (Serogruppe) - Ebola (hämorrhagisches Fieber)
         ■ Sudan-Ebolavirus - Ebola (hämorrhagisches Fieber)
         ■ Cöte d'Ivoire-Ebolavirus - Ebola (hämorrhagisches Fieber)
   • Familie Orthomyxoviridae
      ○ Gattung *Influenzavirus A* - Influenza (Grippe)
         ■ Influenzavirus A-Variante (H1 N1)- Influenza (Grippe)
         ■ Influenzavirus A-Variante (H3N2) - Influenza (Grippe)
         ■ (aviäres) Influenzavirus-A-Variante (H5N1), hoch pathogenes aviäres Influenzavirus (HPAIV) - Vogelgrippe, bei Tieren, auch auf den Mensch übertragbar, aber nicht von Mensch zu Mensch.
      ○ Gattung *Influenzavirus B* - Influenza (Grippe)
         ■ Influenzavirus B/Victoria-Linie - Influenza (Grippe)
         ■ Influenzavirus B/Yamagata-Linie - Influenza (Grippe) ○ Gattung *Influenzaviren C* - Influenza (Grippe)
   • Familie Paramyxoviridae
      ○ Unterfamilie *Paramyxovirinae*
         ■ Gattung *Avulavirus*
            ■ Parainfluenzavirus (1,3) - Parainfluenza
         ■ Gattung *Morbillivirus*
            ■ Masernvirus - Masern
         ■ Gattung *Rubulaviren*
            ■ Parainfluenzavirus (2, 4) - Parainfluenza
            ■ Mumpsvirus - Mumps
      ○ Unterfamilie *Pneumovirinae*
         ■ Gattung *Pneumovirus*
            ■ Respiratory-Syncytical-Virus (RSV) - Atemwegsinfektion
         ■ Gattung *Metapneumovirus*
            ■ Humanes Metapneumovirus (HMPV) - Atemwegsinfektion
   • Familie Rhabdoviridae
      ○ Gattung *Vesiculovirus*
         ■ Vesicular-Stomatitis-Indiana-Virus (VSV) - Stomatitis vesicularis (Mundschleimhautentzündung mit Bläschenbildung) bei Tieren, auch auf den Mensch übertragbar
      ○ Gattung *Lyssavirus*
         ■ Rabiesvirus (RABV) (ehemals Genotyp 1) = Tollwutvirus - Tollwut, bei Tieren, auch auf den Mensch übertragbar
         ■ Mokola-Virus (MOKV) (ehemals Genotyp 3) - Tollwut, bei Tieren, auch auf den Mensch übertragbar
         ■ Duvenhage-Virus (DUVV) (ehemals Genotyp 4) - Tollwut, bei Tieren, auch auf den Mensch übertragbar
         ■ Europäisches-Fledermaus-Lyssa-Virus 1 + 2 (EBLV 1, 2) (ehemals Genotypen 5 und 6) - Tollwut, bei Tieren, auch auf den Mensch übertragbar
         ■ Australisches-Fledermaus-Lyssa-Virus (ABLV) (ehemals Genotyp 7) - Tollwut, bei Tieren, auch auf den Mensch übertragbar

Bei den unbehüllten Viren handelt es sich insbesondere um:
**Doppelsträngige DNA-Viren = dsDNA**
   - Adenoviridae
      ○ Humane Adenoviren A-F (51 Subtypen) - Schnupfen, Erkältungen, Durchfall
   - Papovaviridae
      o Papovaviren
         ■ Humane-Papilloma-Viren
            ■ diverse Humane-Papilloma-Viren (HPV)-Warzen
            ■ Kondyloma-Virus 6 (HPV 6)- Feigwarzen
            ■ Kondyloma-Virus 11 (HPV 11)- Feigwarzen
            ■ Humanes-Papilloma-Virus, low-risk Typen (HPV 40,42,43,44,54,61,70,72,81 und CP6108; high-risk Typen (HPV 16, 18, 31 und 33, aber auch 35, 39, 45, 51, 52, 56, 58, 59, 68, 73 und 82)- Zervixkarzinom = Gebärmutterhalstumor/-Krebs
         ■ Polyomaviren
**Einzelsträngige DNA-Viren = ssDNA**
   • Panroviridae
      ○ Parvovirinae
         ■ Dependovirus
            ■ Adenoassoziiertes-Virus 2 (AAV 2)
            ■ Adenoassoziiertes-Virus 3 (AAV 3)
            ■ Adenoassoziiertes-Virus 5 (AAV 5)
         ■ Erythrovirus
            ■ Parvovirus B19 - Ringelröteln
**Doppelsträngige RNA-Viren = dsRNA**
   • Reoviren
      ○ Rotaviren - Gastroenteritis = Durchfall
      ○ Orbiviren
         ■ Colorado-Tick-Virus-Colorado-Tick-Fieber
         ■ Epizootic hemorrhagic disease virus (EHDV) - Enzootische Hämorrhagie der Hirsche
**Einzel(+)-Strang-RNA-Viren = ss(+)RNA**
   • Picomaviridae
      ○ Rhinoviren
         ■ Humanes-Rhino-Virus (HRV), 1A, 1B-100 - Schnupfen, Erkältungen
      ○ Aphthoviren
         ■ Maul-und-Klauenseuche-Virus - Maul- und Klauenseuche beim Tier, auch in milder Form auf den Menschen übertragbar
      ○ Enteroviren
         ■ Poliovirus (1-3) - Kinderlähmung
         ■ Coxsackie-Virus-A 1-22,24 (CVA 1-22,24) Erkältungen, virale Meningitis, Myokarditis
         ■ Coxsackie-Virus-B1-6 (CVB 1-6) - Erkältungen, virale Meningitis, Myokarditis
         ■ Echoviren- Erkältungen, Gastroenteritis = Durchfall, Meningoenzephalitis
         ■ Humane Enteroviren - Erkältungen, Gastroenteritis = Durchfall
         ■ SVD-Viren (Vesikuläre Schweinekrankheit)
      ○ Cardioviren
         ■ Enzephalo-Myocarditis-Virus (EMCV) - Enzephatomyocarditis
         ■ Mengo-Virus-Enzephalomyocarcitis
         ■ Theiler-Murines-Enzephalomyelitis-Virus (TMEV) - Enzephalomyelitis
         ■ Vlyuisk-Humanes-Enzephalomyelitis-Virus(VHEV)-Enzephalomyelitis
      ○ Hepatoviren
         ■ Hepatitis-A-Virus (HAV) - Hepatitis A
   • Hepeviridae
      ○ Hepevirus
         ■ Hepatitis-E-Virus (HEV) Hepatitis E
   • Caliciviridae
      ○ Caliciviren
         ■ SRSV = small rounded structured viruses
            ■ Norwalk-Virus - Gastroenteritis = Durchfall
            ■ Noroviren - Gastroenteritis = Durchfall
            ■ Sapoviren - Gastroenteritis = Durchfall
            ■ Vesiviren
            ■ Lagoviren
   • Astroviridae
      ○ Astroviren
         ■ Humanes-Astro-Virus - Gastroenteritis = Durchfall

Da die Verwendung der an sich bekannten Verbindung gemäß Formel I zu einer Verstärkung der Reaktion des Immunsystems auf Entzündungen, hervorgerufen durch pathogen-assoziierte molekulare Muster, führt, ist eine bevorzugte erfindungsgemäße Verwendung der Verbindung gemäß der allgemeinen Formel I die als Immunstimulator bzw. Immuntherapeutikum oder Immunmodulator. Dieser Aspekt der Erfindung, d. h. die Verwendung der ausgewählten Verbindungen gemäß der allgemeinen Formel I, als Immuntherapeutikum bzw. Immunstimulator betrifft insbesondere die o. g. Verwendung in der Krebstherapie, als antivirales und/oder antibakterielles Mittel.

Es war weiterhin völlig überraschend, dass die genannten Verbindungen und Systeme die Blut-/Hirnschranke überwinden und daher bevorzugt mit ZNS-aktiven Substanzen eingesetzt werden können.

Es war außerdem völlig überraschend, dass die Auswahl aus selen-, silizium-, germanium-, phosphor- oder kohlenstoffhaltigen Systemen dazu führte, dass die ausgewählten siliziumhaltigen Glykolipide bzw. Glykosphingolipide, die auch als langkettige Di(acyloxy)dialkylsilane, Di(acyloxy)diarylsilane, Di(acyloxy)-dialkoxysilane und Tetra(acyloxy)silane gemäß der genannten Formel I aufgefasst werden können, eine immunstimulatorische und immunmodulierende Wirkung auf die o. g. ausgewählten Krankheiten dergestalt haben, dass sie therapeutisch eingesetzt werden können. Diese Wirkung betrifft jede Modulation des Immunsystems, die dazu führt, dass die Krankheit gelindert oder therapiert werden kann.

Der Fachmann konnte nicht erwarten, dass bei dem heterogenen Stand der Technik der Cubosomen, Novosomen, Sphingosomen, Galaktosomen, Transferosomen, Niosomen, Siosomen, Smarticles, Cagicles, Nanocapsules und Micromethason auf Selen-, Silizium- und/oder Germanium-Basis die Auswahl von Glykolipiden auf Silizium-Basis zu völlig überraschenden Wirkungen bei der Stimulierung des Immunsystems führt, wobei die Stimulierung des Immunsystems insbesondere die Metastasierung von Krebszellen bzw. die Ausbreitung von Viren oder Bakterien in einem Organismus verhindert.

In einer besonders bevorzugten Ausführungsform der Erfindung wird vorgeschlagen, als die genannten Arsen-Oxide im Sinne der Erfindung Monomethyl-Arsensäuren und Dimethyl-Arsensäuren sowie Monomethyl-Verbindungen von Arsen-Trioxiden sowie Dimethyl-Verbindungen von Arsen-Trioxiden einzusetzen, insbesondere als Antikrebsmittel, bevorzugt als antimetastasierende Agenzien. Die genannten Verbindungen können beispielsweise als Salze, wie als Natrium-, Kalium-, Kalzium-, Magnesium- oder andere Salze, eingesetzt werden. Weiterhin kann es verteilhaft sein, die genannten Arsensäuren oder die genannten Methylverbindungen von Arsentrioxid als Komplexverbindungen, wie z. B. Chelate aus EDTA oder PEG einzusetzen. Weiterhin ist es möglich, organische Verbindungen, wie alkylierte Formen, oder andere Verbindungen für die genannten Verwendungen einzusetzen. Aber auch ionische Verbindungen, wie z. B. Mono- oder Dimethyl-Verbindungen von Arsentrioxid gelöst in Natriumhydroxid bzw. Kalium- oder Bariumhydroxid können bevorzugt eingesetzt werden. In einer weiteren bevorzugten Ausführungsform ist es bevorzugt, Metaboliten der genannten Arsenverbindungen einzusetzen. Bei den Metaboliten kann es sich beispielsweise um Stoffe handeln, die dadurch entstehen, dass ein humaner Patient die genannten Arsenverbindungen aufnimmt und diese in metabolisch veränderter Form nach einiger Zeit mit dem Urin, Blut oder anders ausscheidet. Diese ausgeschiedenen Metaboliten können ebenfalls zur Tumorbekämpfung, insbesondere als Antimetastasierungsmittel eingesetzt werden. Neben der Verwendung dieser Substanzen als eingeschlossene Substanzen - beispielsweise in Liposomen - kann es auch vorgesehen sein, dass diese Verbindungen selbst dazu verwandt werden, um die genannten Krankheiten zu therapieren. Die genannten Verbindungen können auch als "shortening agent" bei Telomeren eingesetzt werden. Hierbei führt der Einsatz der genannten Verbindungen dazu, dass die Telomere verkürzt werden, was zur Folge hat, dass eine Apoptose induziert wird. Besonders bevorzugt können eingesetzt werden: Arsentrioxid, Natriumrnetaarsenit, Arsenic-Säure-Triethylester und Kakodylsäure, die ebenfalls wie die o. g. Verbindungen Arsen-Oxide im Sinne der Erfindung sind.

Besonders bevorzugt ist die Verwendung von CH₃AsO(OH)₂, HAsO₂, H₃[As(OH)₆], deren Salzen bevorzugt Arseniten und/oder Arsenaten, Methylarsenoxiden, Methylarsensäure, Kakodyl, CH₃AsO(OH)₂, (CH₃)₂As(O)OH, Mono- und/oder Dimethyl-Verbindungen von Arsentrioxiden - die Arsen-Oxide im Sinne der Erfindung sind - zur Herstellung eines Mittels zur Behandlung von bevorzugt Tumoren, insbesondere als Antimetastasierungsmittel.

Weitere bevorzugte Ausgestaltungen der Erfindung ergeben sich aus den Ansprüchen.

Die Verbindungen der allgemeinen Formel I, die erfindungsgemäß zur Behandlung von ausgewählten Erkrankungen verwendet werden, enthalten zwei an das Si-Atom gebundene langkettige und zwei ebenfalls an das Si-Atom gebundene verhältnismäßig kurzkettige Reste. Die langkettigen, in der Formel I mit R¹ und R² bezeichneten Reste können Acyloxyreste der Formel R-COO- oder Peptidreste der Formel

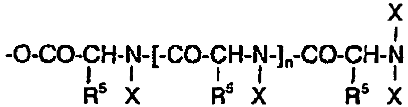

Die Acyloxyreste der Formel R-COO- weisen 6 bis 30 C-Atome auf, d.h. die Reste R enthalten 5 bis 29 C-Atome. Bevorzugt sind Alkyl-, Alkenyl- und Alkinylreste mit 6 bis 18 C-Atomen.

Die Reste R können 1 bis 3 Substituenten tragen, nämlich Halogenatome, Alkoxyreste mit 1 bis 18 C-Atomen oder Aminogruppen. Die Reste R selbst können unverzweigt oder verzweigt sein.

In einer zweiten Ausführungsform bedeuten die Reste R¹ und R² Peptidreste der vorstehend angegebenen Formel. In dieser Formel ist der Rest 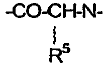 der Rest einer in der Natur vorkommenden Aminosäure der allgemeinen Formel

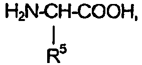

R⁵ also beispielsweise von Isoleucin, Leucin, Lysin, Methionin, Phenylalanin, Threonin, Tryptophan, Valin, Alanin, Arginin, Asparagin, Cystein, Glutamin, Glutaminsäure, Glycin, Hydroxylysin, Hydroxyprolin, Prolin, Serin oder Thyrosin.

Die Peptidreste der vorstehend angegebenen Formel in der Bedeutung von R¹ und R² können Homopolymere sein, d.h. alle Reste R⁵ haben die gleiche Bedeutung, oder Copolymere, bei denen die Reste R⁵ unterschiedliche Bedeutung haben, oder Sequenzcopolymere, in denen die aufeinanderfolgenden Aminosäureeinheiten regelmäßig angeordnet sind.

In den Peptidresten der vorstehend angegebenen Formel können die Aminogruppen in freier Form vorliegen, d.h. X hat die Bedeutung Wasserstoffatom, oder sie können die auf dem Gebiet der Peptidchemie üblichen Schutzgruppen tragen, beispielsweise Alkyl-oderAcylreste.

Die verhältnismäßig kurzkettigen Reste R³ und R⁴ in der Formel I können in einer Ausführungsform Alkylreste mit 1 bis 6, vorzugsweise 1 bis 4 C-Atomen oder Arylgruppen mit 3 bis 14 Atomen, vorzugsweise Phenylgruppen sein. In einer weiteren Ausführungsform können diese Reste die nach Entfernung eines Wasserstoffatoms verbleibenden Reste eines Monosaccharids, Disaccharids, Aminozuckers oder einer Hydroxycarbonsäure sein.

Spezielle Beispiele für geeignete Monosaccharide sind Pentosen, wie Arabinose, Ribose und Xylose, sowie Hexosen, wie Glucose, Mannose, Galactose und Fructose. Beispiele für geeignete Disaccharide sind Saccharose, Lactose und Maltose. Beispiele für geeignete Aminozucker sind Glucosamin und Galactosamin. Ein Beipiel für eine geeignete Hydroxycarbonsäure ist Glucuronsäure. Die Hydroxygruppen der Hydroxycarbonsäuren können dabei in freier, teilderivatisierter oder vollständig derivatisierter Form (Schutzgruppen) vorliegen.

Es war überraschend, dass von den zahlreichen getesteten selen-, silizium- und/oder germaniumhaltigen Systemen, insbesondere Glykolipid-haltigen Systemen, nur die folgenden siliziumhaltigen Glykolipide eine Wirkung bei der Therapie haben: langkettige Di(acyloxy)dialkylsilane, Di(acyloxy)diarylsilane, Di(acyioxy)-dialkoxysilane und Tetra(acyloxy)silane gemäß der o. g. allgemeinen Formel I.

Ein Aspekt der Erfindung ist also die Verwendung der genannten Verbindungen der allgemeinen Formel I als Immuntherapeutikum zur Behandlung von Tumorerkrankungen, insbesondere als Antimetastasierungsmittel und als antivirales Arzneimittel. Bevorzugt ist die Verwendung der Verbindungen gemäß der allgemeinen Formel I zur Herstellung von Vesikeln auf Nicht-Phospholipid-Basis als Therapeutikum gegen die genannten Krankheiten. Besonders bevorzugt ist der Einschluss von Oxoplatin, Foscamet und den genannten Arsen-Verbindungen oder von Antisense-Molekülen in die Vesikel auf Nicht-Phospholipid-Basis als bevorzugt Antimetastasierungsmittel, antivirales oder antibakterielles Mittel.

Es war beispielsweise völlig überraschend, dass die konkreten Vesikel auf Nicht-Phospholipid-Basis, die mit Verbindungen gemäß der allgemeinen Formel I hergestellt wurden, besonders überraschende antitumorale - und hierbei insbesondere antimetastasierende - Wirkungen aufweisen, wenn sie Oxoplatin beinhalten. Im Stand der Technik war lediglich bekannt, dass Cubosomen, Novosomen, Sphingosomen, Galaktosomen, Transferosomen, Niosomen, Siosomen, Smarticles, Cagicles, Nanocapsules und/oder Micromethason Antikrebsmittel wie z. B. Oxoplatin aufnehmen können. Doch auch wenn allgemein bekannt war, das z. B. Siosomen als Träger für Oxoplatin eingesetzt werden können, offenbarte kein Dokument des Standes der Technik, welche der Tausenden möglichen Verbindungen besonders gute, überraschende Eigenschaften aufweisen. Es ist das Verdienst der Erfinder, gezeigt zu haben, dass nur Systeme, die mit Verbindungen gemäß der allgemeinen Formel I hergestellt werden durch Verbindung mit Oxoplatin (d. h. durch den Einschluss von Oxoplatin) völlig überraschende Wirkungen bei der Antimetastasierung in einem Organismus haben. Werden beispielsweise Niosomen oder Siosomen verwendet, die aus zu Verbindungen gemäß der allgemeinen Formel 1 verschiedenen Substanzen hergestellt wurden, so fehlen die überraschenden Eigenschaften. Aber auch die Wirkung als Antimetastasierungsmittel war - selbst im Lichte der offenbarten antitumoralen Wirkung - überraschend.

Unter antitumoraler Wirkung wird ein durchschnittlicher Fachmann die Wirkung auf das Wachstum eines Tumors oder die Remission des Tumors verstehen. Hierzu ist eine Redifferenzierung des Tumorgewebes erforderlich. Die molekularen Mechanismen der Rediffierenzierung sind völlig verschieden von den Mechanismen, die eine Metastasierung von entarteten Zellen verhindern sollen.

Gegenstand der Erfindung ist somit auch die Verwendung von Verbindungen der allgemeinen Formel I 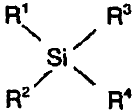 in der R¹, R², R³ und R⁴, die gleich oder verschieden sein können oder Teile eines zwei- oder mehrzähnigen Liganden, durch den ein Chelatkomplex gebildet wird, die jeweils einen oder mehrere Alkylreste R, eine oder mehrere Alkoxyreste R-CO-, einen oder mehrere Acyloxyreste der Formel R-COO- oder Glukosiolreste wie Monosaccharide, Di-Saccharide, Polysaccharide (Zuckersilane), Hydroxygruppen oder einen Peptidrest der Formel 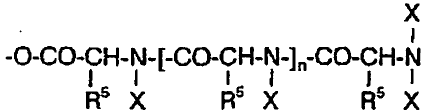 bedeuten, wobei R¹ bis R⁴ einen unverzweigten oder verzweigten Alkyl-, Alkenyl-, Alkinylrest, Arylrest oder Heterocyclus oder einen cyclischen oder aliphatischen Rest oder einen heterocyclischen oder heteroaliphatischen Rest bedeuten, der durch ein bis drei Halogenatome, eine oder mehrere Hydroxygruppen, eine oder mehrere Carboxyl- oder Carboxylatgruppen, Akoxyeste, Aminogruppen, die substituiert oder unsubstituiert sind oder als Ammoniumsalz vorliegen, eine oder mehrere Ketogruppen, Aldehydgruppen, eine oder mehrere Estergruppen substituiert sein kann, die Reste R⁵, die gleich oder verschieden sein können, den nach Entfernung der Gruppe 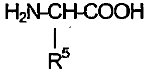 von einer in der Natur vorkommenden oder synthetisierten Aminosäure verbleibenden Rest darstellen, die Reste X, die gleich oder verschieden sein können, ein Wasserstoffatom oder eine in der Peptidchemie übliche Aminoschutzgruppe darstellen und n eine ganze Zahl von 0 bis 12 bedeutet.

R¹, R², R³ und R⁴ können ferner Monosaccharide, Disaccharide, Oligosaccharide, Polysaccharide (Homoglycane, Heteroglycane), Reste eines Aminozuckers, einer Hydroxycarbonsäure, Polyhydroxycarbonsäure oder eines entsprechenden Salzes darstellen oder einen Acylrest einer in der Natur vorkommenden und/oder synthetichen Aminosäure mit freier oder geschützter Aminogruppe, ein Lipoprotein, Glucoprotein, Glycerid, Wirkstoffe wie z. B. Vitamine, Enzyme, Coenzyme, Antioxidantien, Antimetabolite, Hormone, DNA "Desoxyribonucleinsäure", Nucleoside und Arzneistoffe darstellen.

R¹, R², R³ und R⁴ können ferner Rezeptoren und/oder Rezeptorenblocker für z.B. Viren, Bakterien, Hormone sein, Kofaktoren, Substrate, Agonisten und /oder Antagonisten, die über eine Si-O, eine Si-N oder eine Si-C Bindung an das Silicium-Zentralatom gebunden sind, darstellen.

Die zwei- oder mehrzähnigen Liganden können Zucker, Aminosäuren, Pepitde, Dicarbonsäuren, Aminocarbonsäuren, Polyhydroxydicarbonsäuren, Aminozucker, sowie alle geeigneten chemischen Verbindungen sein, die als Chelatliganden eingesetzt werden können.

In einer bevorzugten Ausführungsform der Erfindung ist vorgehesen, dass die genannten Verbindungen der allgemeinen Formel I, in der R¹, R², R³ und/oder R⁴, die gleich oder verschieden sein können, den nach Entfernung eines Wasserstoffatoms verbleibenden Rest eines Monosaccharids, Disaccharids, Oligosaccharids, Polysaccharids (Homoglycane, Heteroglycane), Aminozuckers, einer Hydroxycarbonsäure, Polyhydroxy-carbonsäure oder eines entsprechenden Salzes darstellen oder ein Lipoprotein, Glucoprotein, Glycerid, die über eine Si-O, eine Si-N oder eine Si-C Bindung an das Slicium-Zentralatom gebunden sind, darstellen.

In einer bevorzugten Ausführungsform der Erfindung ist vorgehesen, dass die genannten Verbindungen der allgemeinen Formel I, in der R¹, R², R³ und/oder R⁴ die gleich oder verschieden sein können, eine Polypeptidkette der Formel 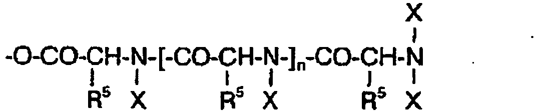 bedeuten, in der n einen Wert von 0 bis 12 aufweist und die Reste R⁵, die gleich oder verschieden sein können, den nach Entfernung der Gruppe 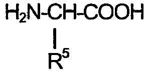 von einer in der Natur vorkommenden Aminosäure und/oder synthetisierte Aminosäure verbleibenden Rest darstellt, die Reste X, die gleich oder verschieden sind, ein Wasserstoffatom oder eine in der Peptidchemie übliche Aminoschutzgruppe darstellen.

In einer bevorzugten Ausführungsform der Erfindung ist vorgehesen, dass die genannten Verbindungen der allgemeinen Formel I, in der R¹ und R² durch einen zwei- oder mehrzähnigen Liganden gebildet werden, wobei die zwei- oder mehrzähnigen Liganden Zucker, Aminosäuren, Pepitde, Dicarbonsäuren, Aminocarbonsäuren, Polyhydroxydicarbonsäuren, Aminozucker, sowie alle geeigneten chemischen Verbindungen, die als Chelatliganden eingesetzt werden können, sein können.

In einer bevorzugten Ausführungsform der Erfindung ist vorgehesen, dass die genannten Verbindungen der allgemeinen Formel I, in der R¹, R², R³ und/oder R⁴, die gleich oder verschieden sein können, Wirkstoffe wie z.B. Vitamine, Enzyme, Coenzyme, Antioxidantien, Antimetabolite, Antisense Moleküle/Reste Hormone oder Arzneistoffe, die über eine Si-O, eine Si-N oder eine Si-C Bindung an das Silicium-Zentralatom gebunden sind, darstellen.

In einer bevorzugten Ausführungsform der Erfindung ist vorgehesen, dass die genannten Verbindungen der allgemeinen Formel I, in der R¹ R², R³ und/ oder R⁴, die gleich oder verschieden sein können, Rezeptoren und/oder Rezeptorenblocker für z.B. Viren, Bakterien, Hormone sind, Kofaktoren, Substrate, Agonisten und /oder Antagonisten, die über eine Si-O, eine Si-N oder eine Si-C Bindung an das Silicium-Zentralatom gebunden sind, darstellen.

Gegenstand der Erfindung ist auch die Verwendung der genannten Verbindungen in monomerer Form, oligomer oder polymer, als Aggregate oder in Form von Vesikeln.

In einer bevorzugten Ausführungsform werden die genannten Verbindungen als Rezeptoren und/oder Rezeptorenblocker (für z.B. Viren, Bakterien, Hormonen), Kofaktoren, Substrate, Agonisten, Antagonisten verwendet.

In einer weiteren bevorzugten Ausführungsform ist die Verwendung der genannten Verbindungen als Suspensionen, als Dispersionen, als Mischung und/oder Lösung, als multilamellare Vesikel, unilamellare Vesikel und heterolamellare Vesikel, Reverse-phase Evaporation Vesicles, große einschichtige Vesikel, heterogene Vesikel (multilamellare, unilamellare, heterolamellare, kleine, große) vorgesehen.

In einer weiteren bevorzugten Ausführungsform der Erfindung ist vorgesehen, dass die genannten Verbindungen bei der Vorbereitung, Herstellung, und Applikation von parenteralen Arzneiformen, rektaten und vaginalen Arzneiformen, perkutanen, subkutanen und transdermalen Arzneiformen, Arzneiformen zur Anwendung am Auge, Arzneiformen zur Anwendung in der Nase und dem Ohr, perorale Arzneiformen mit langsamer Wirkstoffireisetzung, perorale Arzneiformen mit kontrollierter Wirkstofffreisetzung, dentale Applikation und topische Applikalionsformen verwendet werden.

In einer weiteren bevorzugten Ausführungsform ist die Verwendung der genannten Verbindungen als Bestandtetl oder in Mischung mit natürlichen und/oder synthetischen Polymeren aller Art wie Stärke, Stärkehydrolyse und andere Stärkeabbauprodukte, Polyethyienglycol und andere lösliche, schwerlösliche und unlösliche Polymere, vorgesehen.

In einer weiteren bevorzugten Ausführungsform ist die Verwendung der genannten Verbindungen als Wirkstoff, Pre-Wirkstoff, Pre-Pre-Wirkstoff, für die Vorbereitung, Herstellung, und Anwendung von Antigenen, Antigenkonjugaten und Antigenpräparaten, Antikörpern, Antikörperkonjugaten und Antikörperpräparaten, Haptenen, Haptenkonjugaten und Haptenpräparaten, Bakterien, Bakterienkonjugaten und Bakterienpräparaten, Virus, Viruskonjugaten und Viruspräparaten, Heparin, Heparinkonjugaten, Heparinformulierungen und Präparaten, Immunostimulantien, Immunostimulantienkonjugaten und Präparaten, Humanarzneistoffen. Tierarzneistoffen, Impfstoffen, Enzymen, Coenzymen, Isoenzymen, Vitaminen, Hormonen, Proteinen, Peptiden, Kohlenhydraten, Naturstoffen, Kosmetika, Zahnpflegemitteln, Haut- und Haarpflegemitteln, vorgesehen, so lange, dass diese in Zusammenhang mit der Behandlung von Tumor-, Bakterien-und/oder Viruserkrankungen verabreicht werden.

In einer weiteren bevorzugten Ausführungsform ist vorgesehen, dass die genannten Verbindungen der allgemeinen Formel 1, in der R¹, R², R³ und/oder R⁴, die gleich oder verschieden sein können, als Wirkstoff der Behandlung von Tumoren, Krebs und/oder Leukämien.

In einer weiteren bevorzugten Ausführungsform ist die Verwendung der genannten Verbindungen zur Hemmung oder Verhinderung eines Tumorwachstums, einer Tumorausbreitung, einer Tumor-. Angiogenese, einer Tumor-Invasion, einer Tumor-Infiltration und/oder eine Tumor-Metastasierung vorgesehen.

In einer weiteren bevorzugten Ausführungsform ist die Verwendung der genannten Verbindungen in pharmazeutischen Mitteln vorgesehen, die in Gesamtmengen von 0,05 bis 1000 mg pro kg, bevorzugt von 5 bis 450 mg pro kg Körpergewicht, je 24 Stunden eingesetzt werden.

Gegenstand der Erfindung sind ferner Mischvesikel aus siliciumorganischen Verbindungen der angegebenen allgemeinen Formel, mit neutralen oder geladenen Phospholipiden zur Herstellung geladener oder ungeladener biofiner Siosomen, sowie Mischvesikel mit quartemären Ammoniumsalzen mit mindestens einen längeren Alkylrest n (n mit 10 < n < 18).

Die Verwendung einer mit einem Kohlenhydratrest und einem Glucolipid-oder Fettsäurerest sowie zwei weiteren Resten substituierten Tetraorganosilicium-Verbindung als Rezeptor für Viren oder Bakterien, der in der Zellmembran verankert ist, wird in Zeichnung 1 schematisch dargestellt.

Der Erfindung liegt die Annahme zugrunde, dass entweder die Siosomen oder einzelne Silanmoleküle aufgrund ihrer Molekülstruktur und ihrer Amphiphilie über Adhäsions- oder Assoziationsmechanismen an Membranen binden oder geladene, biofine Mischsiosomen mit Zellmembranen fusionieren und somit eine pharmakologische Wirkung ausüben könnten.

Es war überraschend, dass die anmeldungsgemäßen Verbindungen prophylaktisch und therapeutisch sowie zur Unterstützung biologischer Aktivitäten insbesondere zur Tumorbehandlung eingesetzt werden können. Therapeutisch können die Verbindungen, insbesondere in Form einer Kombination von Verbindungen und pharmazeutisch akzeptablem Träger als pharmazeutisches Mittel auch eingesetzt werden, um chronische Infektionen, septische Infektionen, atopische Ekzeme, Neurodermitis, Psoriasis und anderen Immunerkrankungen eingesetzt werden. Eine prophylaktische Indikation der Verbindungen ist beispielsweise die Gabe der Verbindungen bei einem Patienten, der mit einer Strahlentherapie oder einer Chemotherapie mit Zytostatika behandelt wird, um die Immunsupression, die durch die Art der Therapie initiiert wird, zu verhindern beziehungsweise abzumildern. Aber auch in der präoperativen Phase von Patienten ist eine prophylaktische Gabe der erfindungsgemäßen Verbindungen sinnvoll, beispielsweise wenn eine Bluttransfusion zu einer zellulären Intoleranz geführt hat, die den Immunstatus des Patienten nachteilig verändert. Selbstverständlich kann der Immunstatus eines Patienten auch nach Unfällen, größerer oder kleinflächiger Verletzungen, aber auch nach Traumata nachteilig verändert sein, so dass eine unmittelbare Therapie nicht sofort möglich ist. In diesem Falle können die erfindungsgemäßen Verbindungen ebenfalls prophylaktisch eingesetzt werden, um den Zustand des Patienten so zu stabilisieren, dass die Verletzungen oder pathogenen Veränderungen ursächlich therapiert werden können.

Eine weitere Verwendungsmöglichkeit ist die Aktivierung von Thymozytenpopulationen (Th 1) oder die Freisetzung von Zytokinen und Interleukinen. Weiterhin ist es möglich, den Kalzium-Ionen-Transport durch die Zellmembran zu aktivieren oder den oxidativen Metabolismus in Zellen von wichtigen Stoffwechselorganen wie zum Beispiel der Leber oder der Nieren zu verbessern. Aber auch regulatorische Supressions-mechanismen von immunologischen Kaskaden können aktiviert werden.

Selbstverständlich ist es möglich, dass die erfindungsgemäßen Verbindungen übliche Hilfsstoffe, bevorzugt Träger, Adjuvantien und/oder Vehikel umfassen. Bei den Trägern kann es sich beispielsweise um Füllmittel, Streckmittel, Binde-mittel, Feuchthaltemittel, Sprengmittel, Lösungsverzögerer, Resorptionsbeschteuniger, Netzmittel, Adsorptionsmittel und/oder Gleitmittel handeln. In diesem Fall werden die Verbindungen insbesondere als Arzneimittel oder pharmazeutisches Mittel bezeichnet

In einer weiteren bevorzugten Ausführungsform der Erfindung wird das erfindungsgemäße Mittel als Gel, Puder, Pulver, Tablette, Retard-Tablette, Premix, Prodrug, Emulsion, Aufgussformulierung, Tropfen, Konzentrat, Granulat, Sirup, Pellet, Boli, Kapsel, Aerosol, Spray und/oder Inhalat zubereitet und/oder in dieser Form angewendet Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, dass sie den oder die Wirkstoffe nur oder bevorzugt in einem bestimmten Teil des Intestinaltraktes gegebenenfalls verzögert abgeben, wobei als Einbettungsmassen zum Beispiel Polymersubstanzen und Wachse verwendet werden können.

Die Arzneimittel dieser Erfindung können beispielsweise zur oralen Verabreichung in einer beliebigen oral verträglichen Dosierungsform verwendet werden, die Kapseln, Tabletten und wässrige Suspensionen und Lösungen einschließt, aber nicht darauf beschränkt ist. Im Fall von Tabletten zur oralen Verwendung schließen Träger, die häufig verwendet werden, Lactose und Maisstärke ein. Gleitmittel, wie Magnesium-stearat, können typischerweise zugesetzt werden. Zur oralen Verabreichung in Kapselform werden verwendbare Verdünnungsmittel wie Lactose und getrocknete Maisstärke eingesetzt. Wenn wässrige Suspensionen oral verabreicht werden, wird der Wirkstoff mit Emulgier- und Suspendiermitteln kombiniert. Falls gewünscht, können bestimmte Süßmittel und/oder Geschmacksstoffe und/oder Farbmittel zugesetzt werden.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, zum Beispiel Polyethylenglycole, Fette, zum Beispiel Kakaofett und höhere Ester (zum Beispiel C₁₄₋Alkohol mit C₁₆-Fettsäure) oder Gemische dieser Stoffe.

Salben, Pasten, Cremes und Gele können neben dem oder den Wirkstoffen die üblichen Trägerstoffe enthalten, zum Beispiel tierische und pflanzliche Fette, Wachse, Paraffine, Stärke, Tragant, Cellulosederivate, Polyethylenglycole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, zum Beispiel Milchzucker, Talkum, Kieselsäure, Aluminiumhydroxid, Calciumsilikat und Polyamidpulver oder Gemische dieser Stoffe. Sprays können zusätzlich die üblichen Treibmittel, zum Beispiel Chlorfluorkohlenwasserstoffe, enthalten.

Lösungen und Emulsionen können neben den Wirkstoffen, das heißt der erfindungsgemäßen Verbindungen, die üblichen Trägerstoffe wie Lösungsmittel, Lösungsvermittler und Emul-gatoren, zum Beispiel Wasser, Ethylalkohol, Isopropylalko-hol, Ethylcarbonat, Ethylacetat, Benzyialkohol, Benzylbenzoat, Propylenglykol, 1,3-Butylenglykol, Dimethytformamid, Öle, insbesondere Baumwollsaatöl, Erdnussöl, Maiskeimöl, Olivenöl, Rizinusöl und Sesamöl, Glycerin, Glycerinformal, Tetrahydofurfurylalkohol, Polyethylenglycole und Fettsäureester des Sorbitans oder Gemische dieser Stoffe enthalten. Zur parenteralen Applikation können die Lösungen und Emulsionen auch in steriler und blutisotonischer Form vorliegen.

Suspensionen können neben den Wirkstoffen die üblichen Trägerstoffe wie flüssige Verdünnungsmittel, zum Beispiel Wasser, Ethylalkohol, Propylenglykol, Suspendiermittel, zum Beispiel ethoxilierte Isostearylalkohole, Polyoxyethylen-sorbit- und Sorbitan-Ester, mikrokristalline Cellulose, Aluminiummetahydroxid, Bentonit, Agar-Agar und Tragant oder Gemische dieser Stoffe enthalten.

Die Arzneimittel können in Form einer lyophilisierten sterilen injizierbaren Zubereitung, zum Beispiel als sterile injizierbare wässrige oder ölige Suspension, vorliegen. Diese Suspension kann auch mit im Fachgebiet bekannten Verfahren unter Verwendung geeigneter Dispergier- oder Netzmittel (wie zum Beispiel Tween 80) und Suspendiermittel formuliert werden. Die sterile injizierbare Zubereitung kann auch eine sterile injizierbare Lösung oder Suspension in einem ungiftigen parenteral verträglichen Verdünnungs- oder Lösungsmittel, zum Beispiel eine Lösung in 1,3-Butandiol, sein. Zu den verträglichen Vehikeln und Lösungsmitteln, die verwendet werden können, gehören Mannit, Wasser, Ringer-Lösung und isotonische Natriumchloridlösung. Außerdem werden üblicherweise sterile, nichtflüchtige Öle als Lösungsmittel oder Suspendiermedium verwendet. Zu diesem Zweck kann ein beliebiges mildes nichtflüchtiges Öl einschließlich synthetischer Mono- oder Diglyceride verwendet werden. Fettsäuren, wie Ölsäure und ihre Glyceridderivate sind bei der Herstellung von Injektionsmitteln verwendbar, wie es natürliche pharmazeutisch verträgliche Öle, wie Olivenöl oder Rizinusöl, insbesondere in ihren polyoxyethylierten Formen sind. Diese Öllösungen oder Suspensionen können auch einen langkettigen Alkohol oder einen ähnlichen Alkohol als Verdünnungs- oder Dispergiermittel enthalten.

Die genannten Formulierungsformen können auch Färbemittel, Konservierungsstoffe sowie geruchs- und geschmadcsverbes-serte Zusätze, zum Beispiel Pfefferminzöl und Eukalyptusöl und Süßmittel, zum Beispiel Saccharin, enthalten. Die erfindungsgemäßen Verbindungen sollen in den aufgeführten pharmazeutischen Zubereitungen vorzugsweise in einer Konzentration von etwa 0,01 bis 99,9, vorzugsweise von etwa 0,05 bis 99 Gew.-% der Gesamtmischung vorhanden sein.

Die aufgeführten pharmazeutischen Zubereitungen können außer den Verbindungen weitere pharmazeutische Wirkstoffe enthalten, aber neben weiteren pharmazeutischen Wirkstoffen auch Salze, Puffer, Vitamine, Zuckerderivate, insbesondere Saccaride, Enzyme, Pflanzenextrakte und andere. Die Puffer und Zudcerderivate reduzieren mit Vorteil den Schmerz bei subkutaner Applikation und Enzyme wie beispielsweise Hyaluronidase erhöhen die Wirksamkeit Die Herstellung der oben aufgeführten pharmazeutischen Zubereitungen erfolgt in üblicher Weise nach bekannten Methoden, zum Beispiel durch Mischen des oder der Wirkstoffe mit dem oder den Trägerstoffen.

Die genannten Zubereitungen können bei Mensch und Tier entweder oral, rektal, parenteral (intravenös, intra-muskulär, subkutan), intracisternal, intravaginal, intraperitoneal, lokal (Puder, Salbe, Tropfen) und zur Therapie der unten genannten Krankheiten angewendet werden. Als geeignete Zubereitung kommen Injektionslösungen, Lösungen und Suspensionen für die orale Therapie, Gele, Aufgussformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen. Ferner können die Arzneimittel in andere Trägermaterialien wie zum Beispiel Kunststoffe - Kunststoffketten zur lokalen Therapie -, Kollagen oder Knochenzement eingearbeitet werden.

In einer weiteren bevorzugten Ausführungsform der Erfindung sind die Verbindungen in einer Konzentration von 0,1 bis 99,5, bevorzugt von 0,5 bis 95, besonders bevorzugt von 20 bis 80 Gew.-% in einer pharmazeutischen Zubereitung eingebracht Das heißt, die Verbindungen sind in den oben aufgeführten pharmazeutischen Zubereitungen, zum Beispiel Tabletten, Pillen, Granulaten und anderen, vorzugsweise in einer Konzentration von 0,1 bis 99,5 Gew.-% der Gesamtmischung vorhanden. Die Wirkstoffmenge, das heißt die Menge der erfindungsgemäßen Verbindungen, die mit den Trägermaterialien kombiniert wird, um eine einzige Dosierungsform zu erzeugen, wird von dem Fachmann in Abhängigkeit von dem zu behandelnden Patienten und der besonderen Verabreichungsart variieren können. Nach Besserung des Zustandes des Patienten kann der Anteil der wirksamen Verbindung in der Zubereitung so geändert werden, dass eine Erhaltungsdosis vorliegt, die die Krankheit zum Stillstand bringt. Anschließend kann die Dosis oder Frequenz der Verabreichung oder beides als Funktion der Symptome auf eine Höhe verringert werden, bei der der verbesserte Zustand beibehalten wird. Wenn die Symptome auf das gewünschte Niveau gelindert worden sind, sollte die Behandlung aufhören. Patienten können jedoch eine Behandlung mit Unterbrechung auf Langzeitbasis nach beliebigem Wiederauftreten von Erkrankungssymptomen benötigen. Demgemäß ist der Anteil der Verbindungen, das heißt ihre Konzentration, in der Gesamtmischung der pharmazeutischen Zubereitung ebenso wie ihre Zusammensetzung oder Kombination variabel und kann vom Fachmann aufgrund seines Fachwissens modifiziert und angepasst werden.

Dem Fachmann ist bekannt, dass die erfindungsgemäßen Verbindungen mit einem Organismus, bevorzugt einem Menschen oder einem Tier, auf verschiedenen Wegen in Kontakt gebracht werden können. Weiterhin ist dem Fachmann bekannt, dass insbesondere die pharmazeutischen Mittel in verschiedenen Dosierungen appliziert werden können. Die Applikation sollte hierbei so erfolgen, dass die Erkrankung möglichst effektiv bekämpft wird bzw. der Ausbruch einer Krankheit in einer prophylaktischen Gabe verhindert wird. Die Konzentration und die Art der Applikation können vom Fachmann durch Routineversuche eruiert werden. Bevorzugte Applikationen der erfindungsgemäßen Verbindungen sind die orale Applikation in Form von Pulver, Tabletten, Saft, Tropfen, Kapseln oder ähnlichem, die rektale Applikation in Form von Zäpfchen, Lösungen und ähnlichem, parenteral in Form von Injektionen, Infusionen und Lösungen sowie lokal in Form von Salben, Pflastern, Umschlägen, Spülungen und ähnlichem. Bevorzugt erfolgt das In-Kontakt-Bringen der erfindungsgemäßen Verbindungen prophylaktisch oder therapeutisch.

Die Eignung der gewählten Applikationsformen wie auch der Dosis, des Applikationsschemas, der Adjuvantswahl und dergleichen kann beispielsweise durch Entnahme von Serum-Alliquoten aus dem Patienten, das heißt dem Mensch oder dem Tier, und dem Testen auf das Vorhandensein von Krankheits-indikatoren im Verlauf des Behandlungsprotokolls bestimmt werden. Alternativ und begleitend hierzu kann der Zustand der Nieren, der Leber o.a., aber auch die Menge von T-Zellen oder anderen Zellen des Immunsystems, auf herkömmliche Weise begleitend bestimmt werden, um einen Gesamtüberblick über die immunologische Konstitution des Patienten und insbesondere die Konstitution von stoffwechselwichtigen Organen, zu erhalten. Zusätzlich kann der klinische Zustand des Patienten auf die gewünschte Wirkung hin beobachtet werden. Wenn eine unzureichende therapeutische Effektivität erzielt wird, kann der Patient mit erfindungsgemäßen Mitteln ggf. mit anderen bekannten Medikamenten modifiziert weiterbehandelt werden, von denen eine Verbesserung der Gesamtkonstitution erwartet werden kann. Selbstverständlich ist es auch möglich, die Träger oder Vehikeln des pharmazeutischen Mittels zu modifizieren oder den Verabreichungsweg zu variieren.

Neben der oralen Aufnahme kann es dann zum Beispiel vorgesehen sein, dass Injektionen beispielsweise intramuskulär oder subkutan oder in die Blutgefäße ein weiterer bevorzugter Weg für die therapeutische Verabreichung der erfindungsgemäßen Verbindungen sind. Zeitgleich kann auch die Zufuhr über Katheter oder chirurgische Schläuche angewendet werden; beispielsweise über Katheter, die direkt zu bestimmten Organen wie den Nieren, der Leber, der Milz, dem Darm, der Lunge etc. führen.

Die erfindungsgemäßen Verbindungen können in einer bevorzugten Ausführungsform in einer Gesamtmenge von bevorzugt 0,05 bis 500mg/kg Körpergewicht je 24 Stunden eingesetzt werden, bevorzugt von 5 bis 100mg/kg Körpergewicht Hierbei handelt es sich vorteilhafterweise um eine therapeutische Menge, die verwendet wird, um die Symptome einer Störung oder responsiven, pathologisch physiologischen Kondition zu verhindern oder zu verbessern.

Selbstverständlich wird die Dosis vom Alter, der Gesundheit und dem Gewicht des Empfängers, dem Grad der Krankheit, der Art einer notwendigen gleichzeitigen Behandlung, der Häufigkeit der Behandlung und der Art der gewünschten Wirkungen und der Nebenwirkungen abhängen. Die tägliche Dosis von 0,05 bis 500mg/kg Körpergewicht kann einmalig oder mehrfach angewendet werden, um die gewünschten Ergebnisse zu erhalten. Typischerweise werden insbesondere pharmazeutischen Mittel zur etwa 1- bis 10-maligen Verabreichung pro Tag oder alternativ oder zusätzlich als kontinuierliche Infusion verwendet Solche Verabreichungen können als chronische oder akute Therapie angewendet werden. Die Wirkstoffmengen, die mit den Trägermaterialien kombiniert werden, um eine einzelne Dosierungsform zu erzeugen, können in Abhängigkeit von dem zu behandelnden Wirt und der besonderen Verabreichungsart selbstverständlich variieren. Bevorzugt ist es, die Tagesdosis auf 2 bis 5 Applikationen zu verteilen, wobei bei jeder Applikation zum Beispiel 1 bis 2 Tabletten mit einem Wirkstoffgehalt von 0,05 bis 500mg/kg Körpergewicht verabreicht werden. Selbstverständlich ist es möglich, den Wirkstoffgehalt auch höher zu wählen, beispielsweise bis zu einer Konzentration bis 5000mg/kg. Die Tabletten können auch retardiert sein, wodurch sich die Anzahl der Applikationen pro Tag auf 1 bis 3 vermindert. Der Wirkstoffgehalt der retardierten Tabletten kann 3 bis 3000mg betragen. Wenn der Wirkstoff - wie ausgeführt - durch eine Injektion verabreicht wird, ist es bevorzugt, 1- bis 10-mal pro Tag beziehungsweise durch Dauerinfusion den Wirt mit den erfindungsgemäßen Verbindungen in Kontakt zu bringen, wobei Mengen von 1 bis 4000mg pro Tag bevorzugt sind. Die bevorzugten Gesamtmengen pro Tag haben sich in der Humanmedizin und in der Veterinärmedizin als vorteilhaft erwiesen. Es kann erforderlich sein, von den genannten Dosierungen abzuweichen und zwar in Abhängigkeit von der Art und dem Körpergewicht des zu behandelnden Wirts, der Art und der Schwere der Erkrankung, der Art der Zubereitung der Applikation des Arzneimittels sowie dem Zeitraum bzw. dem Intervall, innerhalb welchem die Verabreichung erfolgt. So kann es in einigen Fällen bevorzugt sein, den Organismus mit weniger als den genannten Mengen in Kontakt zu bringen, während in anderen Fällen die angegebene Wirkstoffmenge überschritten werden muss. Die Festlegung der jeweils erforderlichen optimalen Dosierungen und der Applikationsart der Wirkstoffe kann durch den Fachmann aufgrund seines Fachwissens erfolgen.
In einer weiteren besonders bevorzugten Ausführungsform der Erfindung wird das pharmazeutische Mittel in einer Einzelgabe von 1 bis 100, insbesondere von 2 bis 50mg/kg Körpergewicht eingesetzt. Wie auch die Gesamtmenge pro Tag (siehe oben) kann auch die Menge der Einzelgabe pro Applikation von dem Fachmann aufgrund seines Fachwissens variiert werden. Die erfindungsgemäß verwendeten Verbindungen können in den genannten Einzelkonzentrationen und Zubereitungen zusammen mit dem Futter bzw. mit Futterzubereitungen oder mit dem Trinkwasser auch in der Veterinärmedizin gegeben werden. Eine Einzeldosis enthält vorzugsweise die Menge Wirkstoff, die bei einer Applikation verabreicht wird, und die gewöhnlich einer ganzen, einer halben Tagesdosis oder einem Drittel oder einem Viertel einer Tagesdosis entspricht Die Dosierungseinheiten können demgemäß bevorzugt 1, 2, 3 oder 4 oder mehrere Einzeldosen oder 0,5, 0,3 oder 0,25 einer Einzeldosis enthalten. Bevorzugt wird die Tagesdosis der erfindungsgemäßen Verbindungen auf 2 bis 10 Applikationen verteilt, bevorzugt auf 2 bis 7, besonders bevorzugt auf 3 bis 5 Applikationen. Selbstverständlich ist auch eine Dauerinfusion der erfindungsgemäßen Mittel möglich.

In einer besonders bevorzugten Ausführungsform der Erfindung werden bei jeder oralen Applikation der erfindungsgemäßen Verbindungen 1 bis 2 Tabletten gegeben. Die erfindungsgemäßen Tabletten können mit dem Fachmann bekannten Überzügen und Hüllen versehen sein und auch so zusammengesetzt werden, dass sie den oder die Wirkstoffe nur bei bevorzugten, in einem bestimmten Teil des Wirts freigeben.

Bevorzugt ist es in einer weiteren Ausführungsform der Erfindung, dass die einzelnen Bestandteile der Verbindungen gegebenenfalls miteinander assoziiert oder mit einem Träger verbunden in Liposomen eingeschlossen sind, wobei der Einschluss in Liposomen im Sinne der Erfindung nicht zwingend bedeuten muss, dass die Verbindungen im Inneren der Liposomen vorliegen. Ein Einschluss im Sinne der Erfindung kann auch bedeuten, dass die Verbindungen mit der Membran der Liposomen assoziiert sind, beispielsweise so, dass diese auf der äußeren Membran verankert sind. Eine solche Darstellung der erfindungsgemäßen Verbindungen in oder auf den Liposomen ist verteilhaft, wenn der Fachmann die Liposomen so auswählt, dass sie eine immunstimulierende Wirkung haben. Dem Fachmann sind aus der DE 198 51 2 82 verschiedene Möglichkeiten bekannt, die immunstimulierende Wirkung von Liposomen zu modifizieren. Bei den Lipiden kann es sich auch um einfache Lipide handeln, wie beispielsweise Ester und Amide oder um komplexe Lipide wie zum Beispiel um Glycolipide wie Cerebroside oder Ganglioside, um Sphingolipide oder um Phospholipide.

Bevorzugte Erkrankungen sind im Sinne der Erfindung: AIDS, Akne, Albuminurie (Proteinurie), Alkoholentzugssyndrom, Allergien, Alopezie (Haarausfall), ALS (Amyotrophe Lateralsklerose), Alzheimer Erkrankung, AMD (Altersbedingte Makuladegeneration), Anämie, Angststörungen, Anthrax (Milzbrand), Aortensklerose, arterielle Verschlusskrankheit, Arterienverkalkung, Arterienverschluss, Arteritis temporalis, Arteriosklerose, Arteriovenöse Fisteln, Arthritis, Arthrose, Asthma, Ateminsuffizienz, Autoimmunerkrankung, AV-Block, Azidose, Bandscheibenvorfall, Bauchfellentzündung, Bauchspeicheldrüsenkrebs, Becker Muskeldystrophie, Benigne Prostata Hyperplasie (BPH), Blasenkarzinom, Bluterkrankheit (Hämophilie), Bronchialkarzinom, Brustkrebs, BSE, Budd-Chiari-Syndrom, Bulimia nervosa, Bursitis (Schleimbeutelentzündung), Byler-Syndrom, Bypass, Chlamydien-Infektion, Chronische Schmerzen, Cirrhose, Commotio cerebri (Gehirnerschütterung), Creutzfeld-Jakob-Erkrankung, Darmkarzinom, Darmkrebs, Darmtuberkulose, Depression, Diabetes insipidus, Diabetes mellitus, Diabetes mellitus juvenilis, Diabetische Retinopathie, Duchenne-Muskeldystrophie, Duodenalkarzinom, Dystrophia musculorum progressiva, Dystrophie, Ebola, Ekzem, Erektile Dysfunktion, Fettsucht, Fibröse, Gebärmutterhalskrebs, Gebärmutterkrebs, Gehimblutung, Gehirnentzündung, Haarausfall, Halbseitenlähmung, Hämolytische Anämie, Hämophilie, Haustierallergie (Tierhaarallergie), Hautkrebs, Herpes zoster, Herzinfarkt, Herzinsuffizienz, Herzklappenentzündung, Hirnmetastase, Hirnschlag, Hirntumor, Hodenkrebs, Ischämie, Kahler-Krankheit (Plasmozytom), Kinderlähmung (Poliomyelitis), Knochenschwund, Kontaktekzem, Lähmung, Leberzirrhose, Leukämie, Lungenfibrose, Lungenkrebs, Lungenödem, Lymphdrüsenkrebs (Morbus Hodgkin), Lymphogranulomatose, Lymphom, Lyssa, Magenkarzinom, Mammakarzinom, Meningitis, Milzbrand, Mukoviszidose (zystrische Fibröse), Multiple Sklerose (MS), Myokardinfarkt Neurodermitis, Neurofibro-matose, Neuronale Tumoren, Nierenkrebs (Nierenzellkarzinom), Osteoporose, Pankreaskarzinom, Pneumonie, Polyneuropathien, Potenzstörungen, Progressive Systemische Sklerose (PSS), Prostatakrebs, Quaddelauschlag (Urtikaria), Querschnitts-syndrom, traumatisches, Rektumkarzinom, Rippenfellentzündung, Schädel-Him-Trauma, Scheidenkrebs (Vaginalkarzinom), Sinusitis, Speiseröhrenkrebs, Tremor, Tuberkulose, Tumorschmerz, Vaginalkarzinom, Verbrennung, Verbrühung, Vergiftungen, Virale Meningitis, Wechseljahre, Weichteilsarkom, Weichteiltumor, Zerebrale Durchblutungsstörungen und/oder ZNS-Tumore. Hierbei handelt es sich im Sinne der Erfindung um Erkrankungen, die mit einer Defizienz des zellulären Immunsystems assoziiert sind.

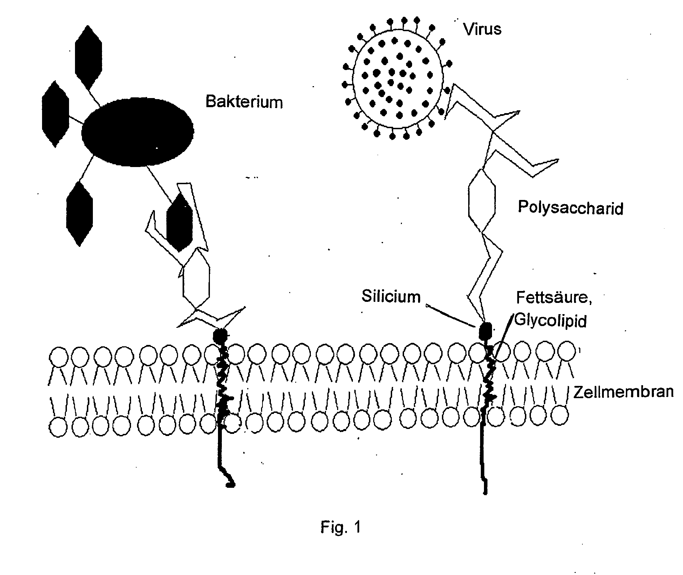

Selbstverständlich ist es auch möglich, die erfindungsgemäßen Tetraorganosilizium-Verbindungen beispielsweise in Form von Zuckersilanen als antivirales Mittel oder antibakterielles Mittel einzusetzen. In diesem Falle werden diese Verbindungen entweder in Vesikel eingebaut oder in nicht eingebauter Form appliziert. Die Interaktion zwischen einem Virus oder einem Bakterium und den genannten Verbindungen, wenn sie in Vesikeln eingebaut sind, ist in Abb. 1 gezeigt. Demgemäß betrifft die Erfindung die Verwendung der Tetraorganosilizium-Verbindungen, insbesondere von Zuckersilanen, zur Prophylaxe, Therapie, Verlaufskontrolle und/oder Nachbehandlung von durch Protozoen, Bakterien, Viren und/oder Parasiten hervorgerufenen Krankheiten. Die Verlaufskontrolle kann bevorzugt eine Überwachung der Wirksamkeit einer Antitumorbehandlung sein.

In einer bevorzugten Ausführungsform der Erfindung ist diese Krankheit insbesondere eine bakterielle Krankheit ausgewählt aus der Gruppe umfassend Affenpocken, AIDS, Anthrax (Bacillus anthracis, Milzbrand), Aviäre Influenza (Geflügelpest, Vogelgrippe), Borreliose, Borrelia recurrentis (Läuserückfallfieber), Botulismus (Clostridium botulinum), Brucellose, Campylobacter-Infektionen, Chlamydiose, Cholera (Vibrio cholerae), Creutzfeldt-Jakob-Krankheit, Coxiella burnetii (Q-Fieber), Cryptosporidium parvuum (Kryptosporidiose), Dengue-Fieber, Diphtherie, Ebolavirus-Infektionen, Echinokokkose (Fuchsbandwurm, Hundebandwurm), EHEC-Infektionen (STEC-Infektionen, VTEC-Infektionen), Enteroviren, Fleckfieber (Rickettsia prowazeckii), Francisella tularensis (Tularämie), Frühsommer-Meningoenzephalitis (FSME), Gelbfieber, Giardiasis, Gonorrhoe, Grippe (Influenza), Haemophilis influenzae, Hantavirus, Helicobacter pylori, Hepatitis A, Hepatitis B, Hepatitis C, Hepatitis D, Hepatitis E, Herpes, HUS (hämolytisch urämisches Syndrom), Keratoconjunctivitis epidemica, Keuchhusten (Pertussis), Kinderlähmung (Poliomyelitis), Kopflausbefall, Krätzemilbenbefall, Krim-Kongo-Fieber, Lassa-Fieber, lebensmittelbedingte Krankheiten, Legionellose, Leishmaniose, Lepra, Leptospirose, Listeriose, Lyme-Borreliose, Lymphogranuloma venereum, Malaria (Plasmodien-Infektionen), Marburgvirus-Infektionen, Masern, Meliodose, Meningokokken-Erkrankungen, MRSA (Staphylokokken), Mumps, Mykosen (Pilzinfektionen), neu und vermehrt auftretende Infektionskrankheiten, Noroviren, Ornithose (Papageienkrankheit), Papillomaviren, Paratyphus, Pest (Yersinia pestis), Pneumokokken-Infektionen (Streptococcus pneumoniae), Pocken, reiseassoziierte Infektionskrankheiten, Rinderbandwurm-Infektion beim Menschen, Rotaviren, Röteln, RSV-Infektionen, Salmonellose, Scharlach, Schweres Akutes Respiratorisches Syndrom (SARS), sexuell übertragbare Infektionen, Shigellose, Syphilis, Tetanus, Tollwut, Toxoplasmose, Thrichinellose, Tuberkulose, Typhus, Varizellen (Windpocken), Variante Creutzfeld-Jakob-Krankheit, virale hämorrhagische Fieber, West-Nil-Fieber, Yersiniose und/oder Zecken-übertragene Erkrankungen. Die Mittel können auch membrandestabilisierend oder anders wirken. Im Sinne der Erfindung ist es bevorzugt wesentlich, dass die Pathogenität der Erreger herabgesetzt wird.

In einer weiteren bevorzugten Ausführungsform ist die Krankheit insbesondere eine Viruserkrankung ausgewählt aus der Gruppe umfassend Influenza, Schnupfen, Husten, Masern, Mumps, Röteln, Ringelröteln, Drei-Tage-Fieber, Windpocken, Pfeiffer'sches Drüsenfieber, SARS, Zytomegalie, Durchfall, Hepatitis, Kinderlähmung, Herpes labialis, Warzen, Tollwut, Lassa-Fieber, Ebola, Marburg-Fieber, Hanta-Virus-Fieber, FSME, RSSE, Louping-ill-Enzephalitis, Powassan-Enzephalitis, Kyasanur forest-Fieber, Omsk-hämorrhagisches-Fieber, Colorado-tick-Fieber, Gelbfieber, Dengue-Fieber, Japanische Enzephalitis, West-Nil-Fieber, Chikungunya-Fieber, O'nyong-nyong-Fieber, Rift-Tal-Fieber, Sandmücken-Fieber, Ross-River-Fieber, Sindbis-Fieber, Mayaro-Fieber, Murray-Valley-Enzephalitis, St. Louis-Enzephalitis, Rocio-Enzephalitis, California-Enzephalitis, Bunyamwera-Fieber, Oropouche-Fieber, AIDS, Herpes genitalis und/oder Herpes simplex

In einer besonders bevorzugten Ausführungsform ist die Erkrankung einer Hepatitis-Erkrankung ausgewählt aus der Gruppe umfassend Hepatitis A, Hepatitis B, Hepatitis C, Hepatitis D, Hepatitis E, Hepatitis F, Hepatitis G und/oder autoimmune Hepatitis.

Und ganz besonders bevorzugt ist die Viruserkrankung AIDS ausgewählt aus der Gruppe umfassend
(a) asymptomatische oder symptomatische HIV-Infektionen,
(b) bazilläre Angiomatosen, Entzündungen des kleinen Beckens, besonders bei Komplikationen eines Tuben- oder Ovarialabszesses, ausgedehnter oder rezidivierender Herpes zoster, thrombozytopene Purpura, lang anhaltendes Fieber oder Diarrhoen, die länger als einen Monat anhalten, Listeriose, orale Harrleukoplakie, oropharyngeale Candidiosen, chronische oder schwer zu therapierende vaginale Candidosen, zervikale Dysplasien, Carcinoma in situ, periphere Neuropathie und/oder
(c) Candidosen der Atemwege oder der Speiseröhre, Cytomegalievirus-Infektionen, CMV-Retinitis, HIV-bedingte Enzephalopathie, Herpes simplex mit chronischen Ulzera (>1 Monat) oder durch Herpes simplex bedingte Bronchitis, Pneumonie oder Ösophagitis, chronische Histoplasmose, itestinale Isosporiasis, Kaposi-Sarkom, disseminierte oder extrapulmonale Kokzidiomykose, extrapulmonale Kryptokokkose, chronisch intestinale Kryptosporidiose, immunoblastisches, primär zerebrales oder Burkitt Lymphom, extrapulmonale Mykobakterien, Pneumocystis-Pneumonie, rezidivierende bakterielle Pneumonie, progressive multifokale Leukenzephalopathie, rezidivierende Salmonellen-Septikämie, Tuberkulose, zerebrale Toxoplasmose, Wasting-Symdrom und/oder invasives Zervixkarzinom.

HIV-Erkrankungen werden in der Regel nach einer CDC-Klassifikation eingeteilt, die auf der Einteilung in drei verschiedenen Kategorien basieren, die sich aus dem klinischen Bild [(a) bis (c)] ergeben unter Einteilung des CD4-T-T Hetferzellstatus (JAMA, 269, Nr. 6,1993, S. 729 - 730).

Das klinische Bild gemäß (a) bezeichnet insbesondere eine asymptotische HIV-Infektion.

Im Sinne der Erfindung werden unter Kategorie (b) AIDS-Erkrankungen zusammengefasst, die im Zusammenhang mit dem CD4-T-Helferzellstatus stehen; zu diesen gehören bazilläre Angiomatosen, Entzündungen des kleinen Beckens, besonders bei Komplikationen eines Tuben- oder Ovarialabszesses, ausgedehnter oder rezidivierender Herpes zoster, thrombozytopene Purpura, lang anhaltendes Fieber oder Diarrhoen, die länger als einen Monat anhalten, Listeriose, orale Harrleukoplakie, oropharyngeale Candidiosen, chronische oder schwer zu therapierende vaginale Candidosen, zervikale Dysplasien, Carcinoma in situ, periphere Neuropathie.

Im Sinne der Erfindung werden unter Kategorie (c) die AIDS-Erkrankungen zusammengefasst, die das klinische Erscheinungsbild dieser Krankheit definieren; zu diesen gehören Candidosen der Atemwege oder der Speiseröhre, Cytomegalievirus-Infektionen, CMV-Retinitis, HIV-bedingte Enzephalopathie, Herpes simplex mit chronischen Ulzera (>1 Monat) oder durch Herpes simplex bedingte Bronchitis, Pneumonie oder Ösophagitis, chronische Histoplasmose, itestinale Isosporiasis, Kaposi-Sarkom, disseminierte oder extrapulmonale Kokzidiomykose, extrapulmonale Kryptokokkose, chronisch intestinale Kryptosporidiose, immunoblastisches, primär zerebrales oder Burkitt Lymphom, extrapulmonale Mykobakterien, Pneumocystis-Pneumonie, rezidivierende bakterielle Pneumonie, progressive multifokale Leukenzephalopathie, rezidivierende Salmonellen-Septikämie, Tuberkulose, zerebrale Toxoplasmose, Wasting-Symdrom und/oder invasives Zervixkarzinom.

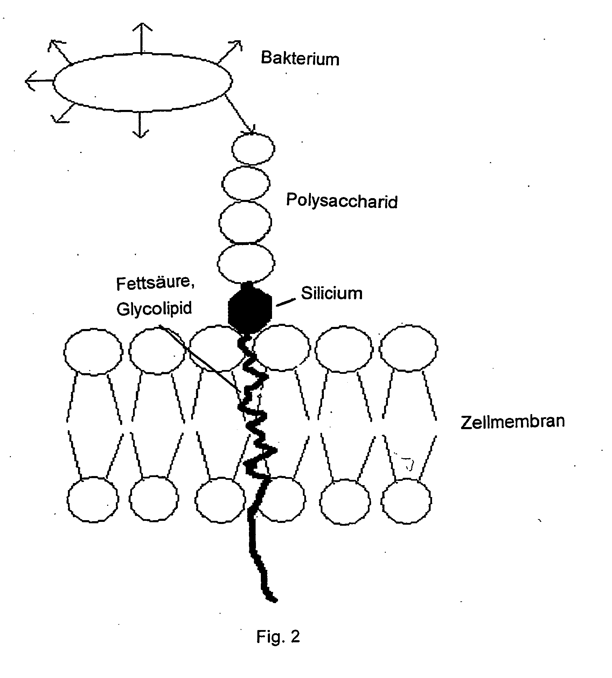

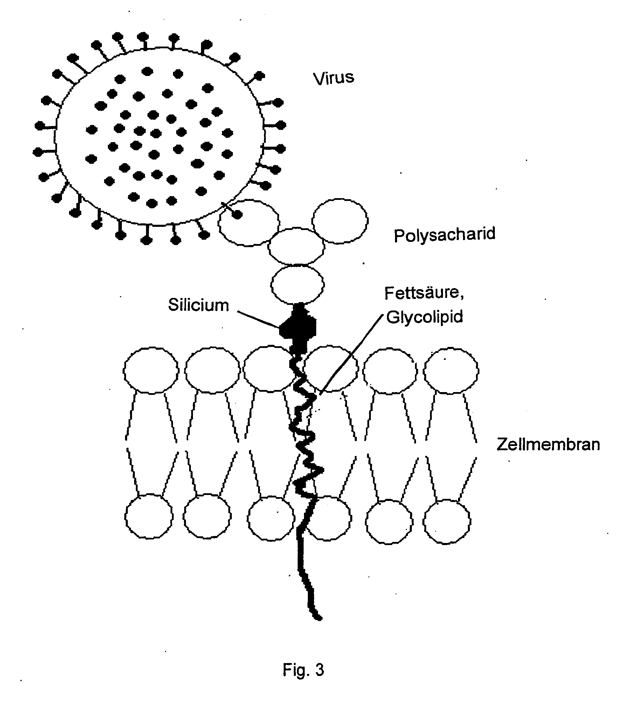

In einer weiteren bevorzugten Ausführungsform der Erfindung wird die zu behandelnde Krankheit im wesentlichen durch Bakterien ausgelöst bzw. durch Bakterien mitausgelöst; bei den Bakterien kann es sich um Legionellen, Streptokokken, Staphylokokken, Klebsiellen, Haemophilis influenzae, Rickettsien (Fleckfieber), Mykobakterien, Mykoplasmen, Ureaplasmen, Neisserien (Meningitis, Waterhouse-Friedrichsen-Syndrom, Gonorrhoe), Pseudomonaden, Bordetellen (Pertussis), Corynobakterien (Diphtherie), Chlamydien, Campylobacter (Durchfall), Escherichia coli, Proteus, Salmonellen, Shigellen, Yersinien, Vibrionen, Enterokokken, Clostridien, Listerien, Borrelien, Treponema pallidum, Brucellen, Francisellen und/oder Leptospira handeln. Eine bevorzugte Form der therapeutischen Anwendung ist in den Fig. 1 bis 3 illustriert. Die Fig. zeigen u.a. die Wechselwirkung von beispielsweise Zuckersilanen mit viralen oder bakteriellen Pathogenen. Diese Art der Wechselwirkung, die auch mit entarteten Zellen möglich ist, stellt eine bevorzugte Art einer Thearpie im Sinne der Erfindung dar. Die Darstellung der einzelenen Komponeneten folgt der Art der Darstellung in Standardwerken auf dem betreffenden Gebiet.

In einer bevorzugten Ausführungsform ist die Krebserkrankung oder der Tumor, die/der behandelt oder verhindert wird, ausgewählt aus der Gruppe von Krebserkrankungen oder Tumorerkrankungen des Hals-Nasen-Ohren-Bereichs, der Lunge, des Mediastinums, des Gastrointestinaltraktes, des Urogenital-Systems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindesalter, Lymphomen, Leukämien, paraneoplastischen Syndromen, Metastasen ohne bekannten Primärtumor (CUP-Syndrom), peritonealen Karzinomastosen, Immunsuppression-bezogenen Malignitäten und/oder Tumor-Metastasen.

Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs; das Melanom; das Hepatom; das Neuroblastom; das Papillom; das Apudom, das Choristom; das Branchiom; das maligne Karzinoid-Syndrom; die Karzinoid-Herzerkrankung; das Karzinom, (zum Beispiel Walker-Karzinom, Basalzellen-Karzinom, basosquamöses Karzinom, Brown-Pearce-Karzinom, duktales Karzinom, Ehrlich-Tumor, in-situ-Karzinom, Krebs-2-Karzinom, Merkel-Zellen-Karzinom, Schleimkrebs, nicht-kleinzelliges Bronchialkarzinom, Haferzellen-Karzinom, papilläres Karzinom, szirrhöses Karzinom, bronchiolo-alveoläres Karzinom, Bronchial-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukämie, Nullzellen-Leukämie, T-Zellen-Leukämie, chronische T-Zellen-Leukämie, HTLV-II-assoziierte Leukämie, akut lymphozytische Leukämie, chronisch-lymphozythische Leukämie, Mastzell-Leukämie und myeloische Leukämie); maligne Histiocytose, Hodgkin-Krankheit, non-Hodgkin-Lymphom, solitärer Plasmazelltumor, Reticuloendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofibrom; Adenolymphom; Karzinosarkom; Chordom; Craniopharyngiom; Dysgerminom; Hamartom; Mesenchymom; Mesonephrom; Myosarkom; Ameloblastom; Cementom; Odontom; Teratom; Thymom; Chorio-blastom; Adenokarzinom; Adenom; Cholangiom; Cholesteatom; Cylindrom; Cystadeno-carcinom; Cystadenom; Granulosa-zelltumor; Gynadroblastom; Hidradenom; Inselzelltumor; Leydig-Zelltumor; Papillom; Sertoli-Zell-Tumor; Thekazelltumor; Leiomyom; Leiomyosarkom; Myoblastom; Myom; Myosarkom; Rhabdomyom; Rhabdomyosarkom; Ependynom; Ganglioneurom; Gliom; Medulloblastom; Meningiom; Neurilemmom; Neuroblastom; Neuroepitheliom; Neurofibrom; Neurom; Paragangliom; nicht-chromaffines Paragangliom; Angiokeratom; angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangio-myom, Lymphangiosarkom; Pinealom; Cystosarkom phyllodes; Hemangiosarkom; Lymphangiosarkom; Myxosarkom; Ovarialkarzinom; Sarkom (zum Beispiel Ewing-Sarkom, experi-mentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Himanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des ürogenitaltrakts); Neurofibromatose und zervikale Plattenepitheldysplasie.

In einer weiter bevorzugten Ausführungsform ist die Krebserkrankung oder der Tumor, die/der behandelt oder verhindert wird, ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Hamleiter, der Blase, der Prostata, der Hamröhre, des Penis und der Hoden, gynäkologische Tumoren umfassend Tumoren des Zervix, der Vagina, der Vulva, Korpuskarzinom, maligne Trophoblastenerkrankung, Övarialkarzinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppression-bezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Transplantations-bezogene Malignitäten, metastasierte Tumoren umfassend Gehimmetastasen, Lungen-metastasen, Lebermetastasen, Knochenmetastasen, pleurale und perikardiale Metastasen und maligne Aszites.

In einer weiter bevorzugten Ausführungsform ist die Krebserkrankung oder der Tumor, die/der behandelt oder verhindert wird, ausgewählt aus der Gruppe umfassend Krebserkrankungen oder Tumorerkrankungen der Mammakarzinome, der Gastrointestinaltumore, einschließlich Kolonkarzinome, Magenkarzinome, Pankreaskarzinome, Dickdarmkrebs, Dünndarmkrebs, der Ovarialkarzinome, der Zervikalkarzinome, Lungen-krebs, Prostatakrebs, Nierenzellkarzinome und/oder Lebermetastasen. Die Auslöser der genannten Krankheiten - d. h. aller im Zusammenhang mit der erfindungsgemäßen Lehre offenbarten bakteriellen, viralen, Tumor- und anderen Erkrankungen - sind im Sinne der Erfindung Pathogene. Pathogene im Sinne der Erfindung sind also alle Objekte, die als Krankheitserreger fungieren können. Hierbei kann es sich sowohl um humanpathogene Objekte wie auch fakultativ pathogene Objekte handeln; z B. um Viren, Bakterien oder entartete Zellen. Diese Pathogene besitzen Merkmale, aus denen sich ihre krankmachenden Eigenschaften herleiten. Diese Krankheiten lassen sich daher durch die pathogen-assoziierten molekularen Muster ihrer Erreger charakterisieren und klassifizieren.

Es war überraschend, dass die Verwendung der Verbindungen gemäß der Formel I zu überraschenden Wirkungen gegen die genannten Krankheiten führt, insbesondere gegen die genannten Virus-, bakteriellen und Tumorerkrankungen. Bei den konkret genannten bakteriellen, Virus- und Tumorerkrankungen zeigt die Anwendung der erfindungsgemäßen Lehre überraschende Resultate. Die überraschenden Resultate werden weiter unten noch einmal gruppiert dargestellt. D. h., wenn eine konkrete Erkrankung im Zusammenhang mit der technischen Lehre offenbart wird, so können für diesen Teilbereich der technischen Lehre auch die überraschenden Vorteile geltend gemacht werden.

Die Erfindung betrifft auch die Verwendung der erfindungsgemäßen Verbindungen in Verfahren zur Prophylaxe und/oder Therapie von Personen, Tieren und/oder Patienten mit pathogenen Modifikationen und/oder Defekten der zellulären Immunität, insbesondere Krebs, Sepsis, allergischen Reaktionen im Zusammenhang mit einer Zytostatika-, Chemo- und/oder Strahlentherapie und/oder als Prophylaxe und/oder Therapie im Zusammenhang mit Unfällen mit atomaren, biologischen, chemischen und/oder radioaktiven Stoffen und/oder Materialien. Entzündungsreaktionen sind alle spezifischen oder unspezifischen Reaktionen des Organismus auf die genannten Krankheiten. Es kann sich beispielsweise um die vom Bindegewebe und den Blutgefäßen getragene Reaktion des Organismus auf einen Entzündungsreiz handeln, mit dem Zweck, diesen zu beseitigen oder zu inaktivieren oder die reizbedingte Gewebsschädigung zu reparieren, wobei der ausgelöste Entzündungsreiz die Interaktion zwischen Virus, Bakterien und entarteten Zellen mit dem Wirtsorganismus darstellt. Demgemäß können bakterielle Toxine oder Allergene, die entartete Zellen produzieren, sowie krankhafte Stoffwechselprodukte wie entgleiste Enzyme von bösartigen Tumoren einen Entzündungsreiz setzen. Das Geschehen kann mit einer kurzen Arteriolenverengung durch Adrenalinwirkung mit Mangeldurchblutung und Gewebsalterationen gefolgt von der Entwicklung der klassischen örtlichen Entzündungszeichen beginnen, d. h. von Rötung, Schwellung, Schmerz und Funktionsstörungen. Dieser Vorgang kann ergänzt werden durch Störungen des Elektrolyt-Haushaltes, Einwanderung neutrophiler Granulozyten und Monozyten durch die Gefäßwände, letzteres mit dem Zweck, den Entzündungsreiz und die geschädigten bis nekrotischen Zellen zu beseitigen; ferner können Lymphozyten-Effektorzellen einwandern, die zur Bildung spezifischer Antikörper gegen den Entzündungsreiz, initiiert durch Bakterien, Viren oder Tumoren, führen, sowie Eosinophile. Durch die bei der Reaktion erfolgende Aktivierung des Komplementsystems können Bruchstücke (C3a und C5a) dieses Systems frei werden, die - wie das Histamin und Bradykinin - als Mediatoren der Entzündung wirken, und zwar im Sinne der Anregung der Chemotaxis der zitierten Blutzellen; ferner wird die Blutgerinnung aktiviert In der Folge kann eine Schädigung (Dystrophie und Koagulationsnekrose) des zugeordneten Organparenchyms eintreten. Der Gesamtorganismus kann je nach Intensität und Art der Entzündung mit Fieber, Stress (s.a. Adaptationssyndrom), Leukozytose und Veränderungen in der Zusammensetzung der Plasmaproteine (Akute-Phase-Reaktion) reagieren, die zu einer beschleunigten Blutkörperchensenkungsreaktion führen. Bevorzugte Entzündungen im Sinne der Erfindung sind die eitrige, die exudative, die fibrinöse, die gangräneszierende, die granulomatöse, die hämorrhagische, die katarrhalische, die nekrotisierende, die proliferative oder produktive, die pseudomembranöse, die seröse, die spezifische und/oder die ulzeröse Entzündungen. Eine eine spezifische Reaktion des Immunsystems, bevorzugt eine Entzündungsreaktion in einem Organismus, gegen Viren, Bakterien und entartete Zellen induzierende Pathogen-assoziierte molekulare Muster Krankheit ist im Sinne der Erfindung jede der genannten Tumorerkrankungen oder Infektionen, hervorgerufen durch einen Virus, oder ein Bakterium, wie sie in der Beschreibung der technischen Lehre offenbart sind. Selbstverständlich kann es möglich sein, dass die Viren, Bakterien oder Tumoren in einem bestimmten Organisums keine Reaktion des Immunsystems induzieren.

Die Erfindung betrifft auch einen Kit und dessen Verwendung in der Medizin. Es ist bevorzugt, die erfindungsgemäßen Verbindungen oder den Kit, der diese umfasst, in einer Kombinationstherapie, insbesondere zur Behandlung von Tumoren, zu verwenden. Besonders bevorzugt ist hierbei, dass die Kombinationstherapie eine Chemotherapie, eine Zytostatikabehandlung und/oder eine Strahlentherapie umfasst. In einer besonders bevorzugten Ausführungsform der Erfindung ist die Kombinationstherapie eine adjuvante biologisch-spezifizierte Therapieform. Ganz besonders bevorzugt ist hierbei, dass diese Therapieform eine Immuntherapie ist Weiterhin ist besonders bevorzugt, dass die Kombinationstherapie eine Gentherapie und/oder eine Therapie mit einer erfindungsgemäßen Verbindung umfasst. Dem Fachmann sind verschiedene Kombinationstherapien, insbesondere zur Behandlung von Tumoren, bekannt. Es kann zum Beispiel vorgesehen sein, dass innerhalb einer Kombinationstherapie eine Zytostatikabehandlung erfolgt oder beispielsweise eine Bestrahlung eines bestimmten Tumorareals, wobei diese Behandlung mit einer Gentherapie kombiniert wird, wobei die erfindungsgemäßen Verbindungen als Antikrebsmittel eingesetzt werden. Demgemäß kann es ganz besonders bevorzugt sein, dass die erfindungsgemäßen Verbindungen zur Erhöhung der Sensitivität von Tumorzellen gegenüber Zytostatika und/oder Strahlen verwendet werden. Weiterhin ist es bevorzugt, dass die erfindungsgemäßen Verbindungen zur Hemmung der Vitalität, der Proliferationsrate von Zellen und/oder zur Induktion von Apoptose und eines Zellzyklus-Anrests verwendet werden. Hierbei können in dem Kit oder der erfindungsgemäßen Verwendung Oxoplatin, die genannten Arsen-Oxide, Foscamet oder die Antisense-Konstrukte als Mittel gegen jede der genannten bevorzugten Krankheiten eingesetzt werden.

Die anmeldungsgemäße Lehre zeichnet sich durch folgende Merkmale aus:
- Abkehr vom technisch Üblichen
- neue Aufgabenstellung
- Vorliegen eines seit langem ungelösten dringenden Bedürfnisses für die Lösung des mit der
- Erfindung gelösten Problems
- bisheriges vergebliches Bemühen der Fachwelt
- die Einfachheit der Lösung spricht für erfinderische Tätigkeit, insbesondere da sie kompliziertere Lehren ersetzt
- Entwicklung der wissenschaftlichen Technik ging in eine andere Richtung
- entwicklungsstraffende Leistung
- Fehlvorstellungen der Fachwelt über die Lösung des entsprechenden Problems (Vorurteil)
- technischer Fortschritt, wie z. B.: Verbesserung, Leistungssteigerung, Verbilligung, Ersparnis an Zeit, Material, Arbeitsstufen, Kosten oder schwer beschaffbaren Rohstoffen, erhöhte Zuverlässigkeit, Beseitigung von Fehlern, Qualitätshebung, Wartungsfreiheit, größere Effektivität, höhere Ausbeute, Vermehrung der technischen Möglichkeiten, Bereitstellung eines weiteren Mittels, Eröffnung eines zweiten Weges, Eröffnung eines neuen Gebietes, erstmalige Lösung einer Aufgabe, Reservemittel, Alternativen, Möglichkeit der Rationalisierung, Automatisierung oder Miniaturisierung oder Bereichung des Arzneimittelschatzes
- glücklicher Griff, da aus einer Vielzahl von Möglichkeiten eine bestimmte gewählt wurde, deren Ergebnis nicht vorausgesagt werden konnte, daher handelt es sich um ein patentwürdigen glücklichen Griff
- Irrtum in Entgegenhaltungen
- junges Gebiet der Technik
- Kombinationserfindung, d.h. mehrere bekannte Elemente werden zu einer Kombination zusammengeführt, die einen überraschenden Effekt aufweist
- Lizenzvergabe
- Lob der Fachwelt und
- wirtschaftlicher Erfolg.

Im Folgenden soll die Erfindung anhand von Beispielen erläutert werden, ohne auf diese Beispiele beschränkt zu sein.

### Beispiele

### Synthese biophiler siliciumorganischer Verbindungen (Zuckersilane)

Äquimolare Mengen pro OH - Gruppe des vorliegenden Silanols, Silandiols oder Silantriols der allgemeinen Formel: 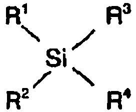 mit
a) R¹ = OH, R², R³, R⁴ = alkyl (methyl bis oktadecyl), phenyl
b) R¹, R² = OH, R³, R⁴ = alkyl (methyl bis oktadecyl), phenyl
c) R¹, R², R³ = OH, R⁴ = alkyl (methyl bis oktadecyl), phenyl
als Aglyca und OH-geschützten Zuckern z. B. azetylgeschützt in Aceton werden wie in der Literatur beschrieben mit der äquivalenten Menge der Lewissäure Trimethylsilyltrifluoromethansulfonat (TMS - Triflat, Me₃SiOSO₂CF₃) in Gegenwart von Säurefängem wie gepulverten Molekularsieb 4 A versetzt und 3 Stunden bei Raumtemperatur gerührt. Die sich bildenden Siliciumzucker (Silazucker) werden durch Säulenchromatografie gereinigt. Ausgangsverbindungen sind weiterhin Mono-, Di- oder Trichlorsilane mit drei, zwei oder einen langkettigen Alkylrest, die aus Grignardierung von Tetrachlorsilan hergestellt worden sind und die leicht zu den entsprechenden Hydroxyverbindungen verseifbar sind, die dann als Aglyca für die Umsetzung mit OH-geschützten Zuckern fungieren können.

**Tabelle: Dargestelle Di(acyloxy)silane * bei Raumtemperatur**

| R¹, R² | R³, R⁴ | Summenformel | M | Schmp. [C°] |
|---|---|---|---|---|
| methyl | octanoyloxy | C₁₈H₃₆O₄Si | 344 | flüssig* |
| methyl | nonanoyloxy | C₂₀H₄₀O₄Si | 372 | " |
| methyl | decanoyloxy | C₂₂H₄₄O₄Si | 400 | " |
| methyl | undecanoyloxy | C₂₄H₄₈O₄Si | 428 | " |
| ethyl | dodecanoyloxy | C₂₈H₅₂O₄Si | 456 | " |
| ethyl | tridecanoyloxy | C₂₈H₅₆O₄Si | 484 | " |
| methyl | tetradecanoyloxy | C₃₀H₆₀O₄Si | 512 | " |
| ethyl | hexadecanoyloxy | C₃₄H₆₈O₄Si | 568 | 24-25 |
| methyl | octadecanoyloxy | C₃₈H₇₆O₄Si | 624 | 33-34,5 |
| methyl | Eicosanoyl | C₄₂H₈₄O₄Si | 680 | 39-42 |
| methyl | Docosanoyl | C₄₆H₉₂O₄Si | 736 | 47-49 |
| ethyl | octanoyloxy | C₂₀H₄₀O₄Si | 382 | flüssig* |
| ethyl | nonanoyloxy | C₂₂H₄₄O₄Si | 400 | " |
| hyl | decanoyloxy | C₂₄H₄₈O₄Si | 428 | " |
| ethyl | undecanoyloxy | C₂₆H₅₂O₄Si | 456 | " |
| ethyl | dodecanoyloxy | C₂₈H₅₆O₄Si | 484 | " |
| ethyl | tridecanoyloxy | C₃₀H₆₀O₄Si | 512 | " |
| methyl | tetradecanoyloxy | C₃₂H₆₄O₄Si | 540 | " |
| ethyl | pentadecanoyloxy | C₃₄H₆₈O₄Si | 568 | " |
| thyl | hexadecanoyloxy | C₃₆H₇₂O₄Si | 596 | 23-25 |
| ethyl | heptadecanoyloxy | C₃₈H₇₆O₄Si | 624 | 26-27 |
| ethyl | octadecanoyloxy | C₄₀H₈₀O₄Si | 652 | 33-34 |
| ethyl | Eicosanoyl | C₄₄H₈₈O4Si | 708 | 40-41,5 |
| ethyl | Docosanoyl | C₄₈H₉₆O₄Si | 764 | 47,5-49 |
| phenyl | octanoyloxy | C₂₈H₄₀O₄Si | 468 | flüssig* |
| phenyl | decanoyloxy | C₃₀H₄₈O₄Si | 524 | " |
| phenyl | dodecanoyloxy | C₃₆H₅₆O₄Si | 581 | " |
| phenyl | tetradecanoyloxy | C₄₀H₆₄O₄Si | 637 | " |
| phenyl | hexadecanoyloxy | C₄₄H₇₂O₄Si | 693 | " |
| phenyl | octadecanoyloxy | C₄₈H₈₀O₄Si | 749 | 33-34 |
| phenyl | Eicosanoyl | C₅₂H₉₆O₄Si | 805 | 40-42 |
| phenyl | Docosanoyl | C₅₆H₁₀₄O₄Si | 861 | 47,5-49 |

| | | | | |
|---|---|---|---|---|
| *Bei Raumtemperatur | | | | |

### Mikrokalorimetrische Untersuchungen von Di(acyloxy)silanen

Die Untersuchungen erfolgten an einem Perkin-Elmer-DSC-7. Die scan-Rate betrug 5 K/min.
Von den untersuchten Verbindungen wurden mittels eines Computerprogramms die Schmelzenthalpien errechnet.

### 1. DES, DPS c ≥ 16 und DMS c ≥ 18

Es stellte sich heraus, dass die thermodynamisch stabilsten Kristalle (= ß₂-Phase) aus der Rekristallisation bzw. nach längerer Lagerung erhalten wurden. Diese ß-Phase schmilzt mit einer einzigen Umwandlung (charakteristisch hohe Temperatur- und Schmelzenthalpiewerte) zur isotropen Flüssigkeit. Beim Abkühlen kristallisieren die Verbindungen DES, DPS ≥ 16 und DMS ≥ 18 zuerst in eine instabile Form (= α-Phase). Mit weiteren Abkühlen kristallisiert die α-Phase in eine stabile ß₂-Phase (DES c16; 18, DPS c16; 18, DMS c18) oder in eine weitere instabile ß₁ Phase (DES c20; 22, DPS c20; 22, DMS c20; 22). Die Stabilität der α-Phase ist abhängig von der Kettenlänge des Fettsäurerestes (kürzerkettigere Verbindungen sind weniger stabil), sowie von der Temperatur der Lagerung. Die Einstellung des thermodynamischen Gleichgewichtes (ß₂-Phase) erfolgt unterschiedlich schnell und kann sich über mehrere Wochen erstrecken. Die Schmelzpunkte der α-Phase liegen nur 2-3 K oberhalb der Kristallisationstemperaturen. Diese nur geringfügige Unterkühlung ist notwendig, um erste Kristallkeime zu bilden, die dann ein schnelles Kristallwachstum ermöglichen (sehr steile Peaks für Kristallisation).

### 2. DES, DPS c < 16 und DMS c < 18

Diese kürzerkettigeren Verbindungen besitzen ein anderes thermisches Verhalten. Beim Aufheizen schmelzen diese Substanzen über einen relativ großen Temperaturbereich (bis zu 25 K) und zeigen so einen lang gestreckten flachen Peak. Beim Abkühlen kristallisieren diese Stoffe in nur eine feste Form. Von besonderem Interesse erscheinen die Verbindungen DES, DPS c = 16 und DMS c = 18. Hier zeigen die homologen Reihen Enthalpieminima.

### Prüfung auf zytotoxisches Potential an Kälber-Aortenendothelzellinie BKEz-7

Als Marker für eine zytotoxische Wirkung wird bei Zellen der charakterisierten Kälber-Aortenendothelzellinie BKEz-7 die Hemmkonzentration ermittelt, bei der eine 50%-ige Hemmung der Zellproliferation erfolgt (im Vergleich zu Kontrollkulturen ohne Zusatz eines Zellinhibitors). Die resultierende Reduzierung der Zellzahl um 50% wird als IC 50 ausgedrückt. Die getesteten Silanverbindungen wurden als Suspensionen auf die Kulturen gegeben und 48 Stunden bei 37°C inkubiert.
Zusätzlich erfolgten morphologische Kontrollen und Vitalitätsbestimmungen mittels dem Neutralrot-Test. Mit Ausnahme des Di(dodecanoyloxy)dimethylsilans standen alle Ergebnisse der morphologischen Untersuchungen in guter Übereinstimmung. Für Di(dodecanoyl-oxy)dimethylsilan ergaben sich im Neutralrot-Test geringere Vitalitätsbeeinflussungen als im Zytotoxizitätstest.

**Tabelle:**

| **Testsubstanz** | **IC50-Wert** | **Bedenkliche Toxizität** |
|---|---|---|
| Di(dodecanoyloxy)diphenylsilan | 0.036 mM | - |
| Di(decanoyloxy)diphenylsilan | 0.014 mM | - |
| Di(octadecanoyloxy)diphenylsilan | 0.084 mM | - |
| Di(octanoyloxy)diphenylsilan | > 0.02 mM | - |
| Di(tetradecanoyloxy)diphenylsilan | 0.046 mM | - |
| Di(dodecanoyloxy)dimethylsilan | 0.129 mM | - |
| Di(octadecanoyloxy)dimethylsilan | 0.495 mM | - |
| Di(tetradecanoyloxy)dimethylsilan | 0.110 mM | - |

### Pharmakologische Untersuchungen an der Maus

Die Verbindungen wurden an der Maus auf zentrale, vegetative und toxische Effekte geprüft. Durch systemische Einzelbeobachtungen sind neben der akuten Toxizität (orienberende letale Dosis, oLD) zentrale und vegetative Wirkungen der Testsubstanzen, insbesondere Effekte auf die Motorik und das Reaktionsvermögen, die Haut- und Rektaltemperatur sowie auf die Pupillenmotorik erfasst worden. Die getesteten Dosierungen betrugen 5 •10⁻⁴, 1 • 10⁻³ und 5 • 10⁻³ mol/kg Körpergewicht Letale Wirkungen konnten bei keiner der Verbindungen beobachtet werden. Die oLD lag für Di(tetradecanoyloxy)dimethylsilan und Di(octadecanoyloxy)dimethylsilan unter 1 • 10⁻³ mol/kg Körpergewicht für alle übrigen Verbindungen unter 5 • 10⁻³ mollkg Körpergewicht.

**Tabelle:**

| **Testsubstanz** | **5•10⁻⁴ mol/kg** | **1•10⁻³ mol/kg** | **5•10⁻³ mol/kg** |
|---|---|---|---|
| Di(dodecanoylaxy)diphenylsila n | keine Verhältensänderungen | keine Verhaltensänderungen | keine Verhaltensänderungen |
| Di(decanoyloxy)diphenylsilan | keine Verhaltensänderungen | keine Verhaltensänderungen | schwache Ataxie |
| Di(hexadecanoyloxy)diphenyls ilan | keine Verhaltensänderungen | keine Verhaltensänderungen | schwache Ataxie, Hyperaktivität und Piloerektion, Straubtail |
| Di(octadecanoyloxy)diphenylsi lan | keine Verhaltensänderungen | schwache Ataxie, Hyperaktivität und Piloerektion | schwache Ataxie |
| Di(octanoyloxy)diphenylsilan | schwache Hyperaktivität | Ataxie, Hyper-aktivität | schwache Ataxie, Hyperaktivität |
| Di(tetradecanoloxy)diphenylsi lan | keine Verhaltensänderungen | Ataxie, Hyper-aktivität | schwache Ataxie, Piloerektion |
| Di(dodecanoyloxy)dimethylsila n | keine Verhaltensänderungen | keine Verhaltensänderungen | Ataxie, Ploerektion, Griffstärkesenkung |
| Di(decanoyloxy)dimethylsilan | keine Verhaltensänderungen | Piloerektion | schwach bis mäßig ausgeprägte Verhaltensänderung en |
| Di(hexadecanoyloxy)dimethyls ilan | keine Verhaltensänderungen | keine Verhaltensänderungen | Ataxie, Piloerektion, Grifistärkesenkung |
| Di(octadecanoyloxy)dimethylsi lan | keine Verhaltensänderungen | keine Verhaltensänderungen | - |
| Di(tetradecanoyloxy)dimethylsi lan | schwache Ataxie, Piloerektion | Ataxie | - |

| | | | |
|---|---|---|---|
| | | | |

### Die zytotoxische Wirkung der beiden Zuckersilane

1-O-Dimethyl(dodecyl)silyl-2,3,4,6-tetra-0-acetyl-ß-D-glucopyrasonid

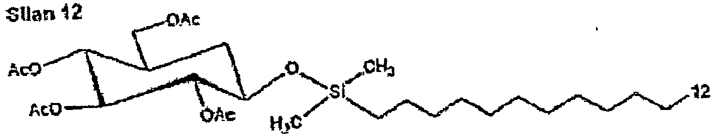

1-O-Dimethyl(octadecyl)silyl-2,3,4,6-tetr'a-0-acteyl-ß-D-glucopyranosid 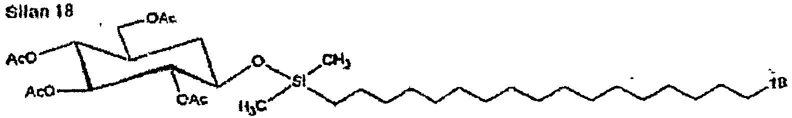 wurde an V79-Zellen im Wachstumshemmtest ermittelt.

Die Zytotoxizität steht in engem Zusammenhang zur akuten Toxizität, die im allgemeinen an Labortieren ermittelt wird.

Ergebnisse aus Zytotoxizitäts-Test ermöglichen eine Abschätzung der LD₅₀ mit retativ hoher Genauigkeit. So ist mittels eines Zytotoxizitäts-Tests eine erste Aussage zur akuten Toxizität möglich.

Bei Silan 12 wurde eine dosisabhängige Hemmung des Wachstums der V79-Zellen in einem Konzentrationsbereich zwischen 32 µg/ml und 200 µg/ml gemessen.

Die Silan wurden in Ethanol aufgelöst und in Zellkulturmedien in Konzentrationen von bis zu 500 µg/ml resuspendiert. Es wurden MTT Tests in dreifacher Ausführung durchgeführt, für welche doppelte Lösungen (7 Schritte) und colo205 (Kofon-Karzinom), BxPC3 (pankreatisches Adenokarzinom) sowie T47D (Brustkrebs) benutzt wurden.

### Zelllinie- IC50 (µg/ml)

| # Silane | Colo205 | BxPC3 | T47D |
|---|---|---|---|
| 1. Di (heptadecanoyloxy) diethylsilan | 125 | 15 | 15 |
| 2. Di (undecanoyloxy) dimethylsilan | 125 | 80 | 125 |
| 3. Di (tridecanoyloxy) dimethylsilan | 230 | 80 | 80 |
| 4. Di (undecanoyloxy) diethylslan | 200 | 100 | 180 |
| 5. Di (pentadecanoyloxy) diphenylsilan | 70 | 60 | 20 |
| 6. Di (tridecanoyloxy) diethylsilan | 240 | 150 | 250 |
| 7. Di (tridecanoyloxy) diphenylslan | 125 | 60 | 80 |
| 8. Di (hexadecanoyloxy) diphenylsilan | 300 | 30 | 20 |
| 9. DPS - C10 = Didecanoyloxy - diethylslan | 60 | 15 | 40 |
| 10. Di (octadecanoyloxy) diphenylsilan | 60 | 15 | 15 |
| 11. Di (hexadecanoyloxy) diethylsilan | 300 | 10 | 7 |
| 12. Di (decanoyloxy) diethylsilan | 300 | 140 | 600 |
| 13. Di (octadecanoyloxy) diethylsilan | 125 | 10 | 8 |
| 14. Di (tetradecanoyloxy) diethylsilan | 250 | 70 | 65 |
| 15. Di (tetradecanoyloxy) diphenylsilan | 62 | 15 | 15 |
| 16. Di (hexadecanoyloxy) dimethylsilan | 500 | 45 | 15 |

Zuckersilane zeigen ebenfalls eine Wirkung auf Krebszellen, Beispiele für solche Verbindungen sind:
1-o-Dimethyl (octadecyl)silyl-2,3,4,6-tetra-0-acetyl-beta-D-Glucopyranosid
1-o-Dimehtyl (dodedecyl)silyi-2,3,4,6-teta-0-acetyl-beta-D-Glucopyranosid

### Silane- Toxizität

### MIT - Assays.

Die Silane 1, 5, 9, 10, 11, 13, 15, 16 wurden in Ethanol gelöst und in MTT Assays in einer anfänglichen Konzentration von 200 µg/ml (5 Lösungsschritte 200 - 12.5 µg/ml) verwendet. Die Platten wurde für eine Dauer.von 4 Tagen inkubiert und die Entwicklungsfähigkeit mithilfe des EZ4U Test bestimmt. Die IC50 Werte wurden ausgehend von Dosierungs-Reaktions-Linien berechnet
Zelllinien: Colo 205 and HT29 - Kolonkarzinom; BxPC3 and MIAPaCa2 - Pankreaskrebs; CRO2B - karzinoid, A431 Epidermalkarzinom; normale Zellen: WI38 - humane embryonale Fibroblasten; NIH3T3 - mausartige Fibroblasten; IEC6 - mausartige Enterozyten.

| *IC50 (µg*/*ml) - Krebszelllinien* | | | | | | |
|---|---|---|---|---|---|---|
| | Colo205 | BxPC3 | CR02B | A431 | HT29 | MlAPaCa2 |
| Si1 | 37.0 | 37.0 | 22.8 | 31.8 | 41.5 | 50.0 |
| Si5 | 43.8 | 25.0 | 35.0 | 36.3 | 40.0 | 38.5 |
| Si9 | 40.6 | 40.0 | 31.5 | 29.4 | 41.5 | 37.5 |
| Si10 | 30.6 | 20.0 | 18.5 | 21.9 | 27.5 | 37.0 |
| Si11 | (200) | 20.0 | 18.9 | 21.9 | 23.1 | 82.5 |
| Si13 | 47.5 | 17.5 | 17.8 | 15.9 | 18.3 | 110 |
| Si15 | 30.0 | 33.8 | 12.5 | 34.5 | 39.4 | 37.0 |
| Si16 | (200) | 12.5 | 31.5 | 34.5 | 34.4 | 400 |

| *IC50 (µg*/*ml) - Normale Zellen* | | | |
|---|---|---|---|
| | WI38 | NIH3T3 | IEC6 |
| Si1 | 26.2 | 36.2 | 28.1 |
| Si5 | 36.5 | 55.0 | 32.5 |
| Si9 | 37.0 | 34.4 | 17.2 |
| Si10 | 21.8 | 22.2 | 17.2 |
| Si11 | 23.7 | 25.0 | 21.2 |
| Si13 | 16.5 | 20.6 | 17.0 |
| Si15 | 35.0 | 29.4 | 19.1 |
| Si16 | 36.0 | 42.5 | 17.2 |

| | | | |
|---|---|---|---|
| | | | |

### Toxizität der Silane - Zusammenfassung:

### Mittelwerte-IC50 für alle Krebs- oder normalen Zelllinien:

| IC50 (µg/ml, Durchschnitt ± SD) | | |
|---|---|---|
| *Krebszellen* | *Normale Zellen* | |
| 33,2 ± 8,0 | 30,1 ± 5,3 | Si1 |
| 34,0 ± 6,4 | 41,3 ± 12,0 | Si5 |
| 35,6 ± 6,0 | 29,5 ± 10,8 | Si9 |
| 21,9 ± 3,9 | 20,4 ± 2,8 | Si10 |
| 21,05 ± 1,9 | 23,3 ± 1,9 | Si11 |
| 17,3 ± 1,03 | 18,0 ± 2,2 | Si13 |
| 30,0 ± 11,9 | 27,8 ± 8,1 | Si15 |
| 28,2 ± 10,5 | 31,9 ± 13,1 | Si16 |

| | | |
|---|---|---|
| Fazit-IC50: Toxizität für Tumorzellen: Si13 > Si11 > Si10 > Si16 > Si15 | | |

### Die subG1 apoptotische Zellteilung:

Die Zellen (IEC6 mausartige Enterozyten; Panc1 Pankreaskrebszellen; Colo205 Kolonkarzinom) wurden mit 50 µg/ml Silanen inkubiert und nach 4-tägiger Aussetzung auf DNA eingefärbt (Propidiumiodid). Die SubG1 Zellteilung wurde mittels Durchflusscytometrie bestimmt.

| IEC6 | Panc1 | Colo205 |
|---|---|---|
| 2,2±0,14 | 2,5 ± 0,07 | 2,1 ± 0,07 |
| 6,55 ± 0,49 | 57,7 ± 0,07 | 3.0 ± 0,14 |
| 6,35 ± 0,63 | 52,8 ± 3,4 | 3,4 ± 0,28 |
| 0,9 ± 0.0 | 42,8 ± 1,6 | 32,1 ± 1,41 |
| 13,4 ± 0,42 | 60,7 ± 0,2 | 43,9 ± 1,41 |
| 7.0 ± 0,14 | 37,7 ± 1,8 | 3,3 ± 0,14 |
| 35,6 ± 0,14 | 36,7 ± 0,5 | 4,8 ± 0,49 |
| 2,9 ± 0,42 | 48,6 ± 0,6 | 37,6 ± 0,21 |
| 2,45±0,21 | 22,2 ± 1,6 | 3,55 ± 0,07 |

### Fazit:

Si13 führte zur höchsten subG1 Teilung bei normalen IEC6 Zellen.

Diese Vorversuche können auch in vivo in Krebs-Ratten reproduziert werden. Weiterhin sind die Versuche in Schweinen reproduzierbar.

### Herstellung der Oxoplatin-Carrier-Systeme

### Vehikelemulsionen:

Grundlage für die Herstellung kolloidaler siliziumhaltiger Trägersysteme für den Arzneistoff (Oxoplatin) ist die Filmmethode, kombiniert mit temperierter Ultraschalldispergierung, die an einem Beispiel illustriert werden soll:

Für die Filmpräparation wird der Trägerstoff [Didodecyfsilyl-bis (2,3,4,6-tetra-O-aceiyl-ß-D-glucopyranosid)] gelöst in Aceton pA. eingesetzt. Die absolute Menge beträgt in jeder Probe 20 mg. Zur Herstellung der Arzneistofflösung werden 125 mg des Cis- / Oxoplatingemisches in 25 ml Pufferlösung pH 7,4 gelöst. Pro Probe werden 2 ml Lösung eingesetzt, dieses entspricht einer Menge von 10 mg Platinsalz.

Anschließen erfolgt eine temperierte Ultraschalldispergierung 30 min bei 30 °C.

Um eine Koagulation bzw. Koaleszenz und damit eine be-schleunigte Sedimentation weitgehend zu vermeiden, wird jede Probe mit einem Disperser 1 min mit durchschnittlich 25000 Umdrehungen / Min dispergiert. Tests haben gezeigt, dass durch diese Verfahrensweise eine deutliche Stabilitätserhöhung der Proben-emulsionen erreicht wird. Eine Überprüfung der Partikelgröße hinsichtlich der Stabilität ergibt einen gemittelten Wert von 300-450 nm.

Die Stabilität dieser Probenemulsionen beträgt je nach Temperatur mehrere Tage oder Wochen.

### Lyophilisate:

Um die Probenemulsionen in stabile Lyophilisate zu überführen, werden diese 2 Stunden auf Trockeneis vorgefroren. Anschließend erfolgt die Gefriertrocknung 3-4 Tage bei -40 °C und einem Vakuum von 0,035 mbar. Die Lyophilisate werden fest verschlossen und bei 2-6°C gelagert

Die Resuspendierung erfolgt durch Zugabe von 2 ml gereinigtes, entionisiertes Wasser sowie kurzes, leichtes Aufschütteln. Im Anschluss wird die Partikelgröße in den resuspendierten Lyophilisaten bestimmt Diese beträgt durchschnitlich 350-400 ± 50nm. Nach der Resuspendierung sind die Emulsionen bis zu 10 Tage lang bei 2-6 °C haltbar. Im weiteren werden Messungen in Abhängigkeit von der Lagerungszeit unter gleichen Bedingungen durchgeführt Die Lyophilisate sind auch nach 8 Wochen problemlos resuspendierbar. Messungen hinsichtlich ihrer Partikelgröße, folglich ihrer Stablität, ergeben keine signifikanten Veränderungen. Die Lyophilisate sind stabile.

### Sterilisation:

Um die Kontaminationsrate der hergestellten Arzneistofflösungen gering wie möglich zu halten, werden ausnahmslos alle verwendeten Lösungen der Filtration (Porendurchmesser 0,2 µm) unterzogen.

Im weiteren werden die Probenemulsionen sofort nach Herstellung durch Dampfsterilisation in einem Autoklaven (121 °C auf 2 bar) sterilisiert.

Anschließende Messungen ergeben eine Partikelgröße von 690 nm, welche sich aber innerhalb von 1-2 Stunden auf 1000-1900 nm erhöht. Offensichtlich sind die Probenemulsionen thermolabil und nicht im Endbehältnis sterilisierbar.

Die Lyophilisate werden in 2 ml gereinigtes, entionisiertes Wasser resuspendiert und unter gleichen Bedingungen einer Dampfdrucksterilisation unterzogen. Messungen der Partikelgröße ergeben einen Größenanstieg auf durchschnittlich 1000 nm. Weitere Tests innerhalb von 24 Stunden ergeben keine derart dramatischen Veränderungen wie die frischen Probenemulsionen, hinsichtlich zunehmender Aggregation und damit verbundene Stabilitätsdefizite.

### Analytik:

Der Gehalt an Platinsalz in den Probenemulsionen sowie in den resuspendierten Lyophilisaten wird mittels Atomabsorptionsspektroskopie ermittelt. Die durchschnittliche Arzneistoffmenge in den Emulsionen beträgt 5 ± 0,5 mg pro Probe. Der Gehalt in den resuspendierten Lyophilisaten liegt bei 5 ± 0,5 mg pro Probe und ist mit dem Gehalt der Emulsionen vergleichbar.

### Fazit:

Die Fämmethode zur Herstellung kolloidaler siliziumhaltiger Trägersysteme für den Arzneistoff Oxoplatin, kombiniert mit temperierter Ultraschalldispergierung lässt sich problemlos durchführen. Auftretende Effekte, wie Koagulation und Koaleszenz können Erfolg versprechend durch die Behandlung der Emulsionen unter Verwendung von einem Disperser und den Einsatz eines Phosphatpuffersystems weitgehend vermieden werden. Die Vehikelstabilität in den Lyophilisaten ist mit der in den frisch hergestellten Emulsionen vergleichbar.

Mit Hilfe dieser Verfahrensweisen ist es uns möglich, die zeitlich noch begrenzte Haltbarkeit der Probenemulsionen in stabile, leicht resuspendierbare Lyophilisaten zu überfuhren. Weiterhin wird die langfristige Lagerung als Lyophilisat empfohlen.

### Chemikalien:

- Cis-/ Oxoplatin gelöst in Pufferlösung pH 7,4
- [Didodecylsilyl-bis (2,3,4,6-tetra-O-acetyl-ß-D-glucopyranosid)] gelöst in Aceton pA
- gereinigtes, entionisiertes Wasser (Membran-gefiltert)

### Geräte:

(1) Disperser von Ika Labortechnik Staufen
(2) Zetamasters S von Malvem Instruments
(3) Gefriertrocknungsanlage Alpha 2-4 LSC von Fisher Scientific
(4) Atomabsorptionsspektrometer der Fa. Zeiss Germany, Gerätetyp AAS 4

### Herstellung der Trinatrium-phosphono-formiat hexahydrat

### (Foscarnet)-Carrier-Systeme

Grundlage für die Herstellung kolloidaler siliziumhaltiger Trägersysteme für den Arzneistoff Trinatrium-phosphono-formiat-hexahydrat (Foscamet) war die Lipidfilmmethode, kombiniert mit temperierter Ultraschalldispergierung.

### Lipidfilmpräparation:

Für die Präparation des Lipidfilmes wurde das Lipid [Didodecylsilyl-bis (2,3,4,6-tetra-O-acetyl-β-D-glucopyranosid)] als Trägerstoff in Aceton pA gelöst und pro Probe/Vial 2 ml eingesetzt, so dass die absolute Menge in jedem Vial 20 mg betrug. Das Aceton wurde anschließend mittels Rotationsverdampfers entfernt.

### Herstellung der Nanovehikelemulsionen:

Zur Herstellung der erforderlichen Foscarnetlösung wurden 2 g Foscarnet in 100 ml isotonischer Sorbitollösung gelöst.

Für jedes Probevial (mit präpariertem Lipidfilm) wurden 2 ml dieser Arzneistofflösung eingesetzt, das entspricht einer theoretischen Absolutmenge von 40 mg Foscarnet.

Anschließend erfolgte eine temperierte Ultraschalldispergierung, 30 min bei 30 °C.

Zur Stabilitätserhöhung wurde jede Probe mit einem Disperser 1 min mit durchschnittlich 25000 Umdrehungen / Min dispergiert. Die durchschnittliche Haltbarkeit der Dispersionen betrug 7 Tage.

Eine Überprüfung der Partikelgröße hinsichtlich der Stabilität ergab einen gemittelten Wert von 400-450 nm

### Lyophilisate:

Für die Überführung der Foscarnet-Nanovehikelemulsionen in stabile Lyophilisate, wurden die Probevials ca. 2 Stunden auf Trockeneis bzw. 4 Stunden im Eisschrank vorgefroren. Anschließend erfolgte die Gefriertrocknung 3-4 Tage bei -40 °C und einem Vakuum von 0,035 mbar, wobei diese Zeitangabe von 4 Tagen auf einer Probenanzahl von ca. 30 Stück bezogen ist Die Kapazität der von uns verwendeten Gefriertrocknungsanlage liegt bei maximal 50 Proben.

Die erhaltenden Lyophilisate wurden fest verschlossen und bei 2-6 °C gelagert, unter diesen Lagerbedingungen ist eine langfristige Lagerung problemlos möglich.

Die Resuspendierung erfolgte durch Zugabe von 2 ml gereinigtes, entionisiertes Wasser. Durch kurzes, leichtes Aufschütteln wurden resuspendierte Lyophlisat-Emulsionen erhalten.

Die Partikefgrößen in den resuspendierten Lyophilisaten betrugen durchschnittlich 400-450 ± 40 nm.

Die resuspendierten Emulsionen sind bis zu 5 Tage lang bei 2-6 °C haltbar und für den baldigen Einsatz bestimmt

### Analytik:

Der Gehalt an Foscamet in den Probenemulsionen sowie in den resuspendierten Lyophilisaten wurde mittels Atomabsorptionsspektroskopie ermittelt. Die durchschnittliche Arzneistoffmenge an Foscarnet in den Emulsionen und in den resuspendierten Lyophilisaten betrug 27 ± 3 mg pro Probe. Die Foscamet Gehalte der Probenemulsionen unterscheiden sich kaum mit denen der resuspendierten Lyophilisaten, sie sind nahezu gleich.

### Fazit:

Die bereits beim Oxoplatin angewendete Filmmethode lässt sich problemlos auf Foscamet übertragen. Unterschiede gibt es nur aufgrund unterschiedlicher Löslichkeiten in der Auswahl des Lösungsmittels. Die Verwendung eines Dispersers wurde ebenfalls auf Foscamet übertragen, so dass hinsichtlich der Stabilität der erhaltenen Emulsionen und Lyophilisate, sehr gute Ergebnisse erzielt werden konnten. Die verschlossenen Lyophilisate können mehrere Wochen bei 2-6 °C problemlos gelagert werden.

### Chemikalien:

- Trinatrium-phosphono-formiat-hexahydrat (Foscamet) in isotonischer Sorbitollösung (2g/100ml)
- [Didodecylsilyl-bis(2,3,4,6-tetra-O-acetyl-ß-D-glucopyranosid)] gelöst in Aceton p.A. (500mg/50ml)
- gereinigtes, entionisiertes Wasser (Membran-gefiltert)

### Geräte:

(1) Disperser von Ika Labortechnik Staufen
(2) Zetamasters S von Malvern Instruments
(3) Gefriertrocknungsanlage Alpha 2-4 LSC von Martin Christ, Osterode
(4) Atomabsorptionsspektrometer der Fa. Zeiss Germany, Gerätetyp AAS 4

Die Verbindungen Dimethyl bis(trialanyl)sillylester C₂₀H₃₈O₈N₆Si, Dimethyl bis(tetraalanyl)silylester C₂₆H₄₈O₁₀N₈Si, Dimethyl bis(pentaalanyl)silylester C₃₂H₅₈O₁₂N₁₀Si, Dimethyl bis(hexaalanyl)silylester C₃₈H₆₈O₁₄N₁₂Si, Dimethyl bis(heptaalanyl)silylester C₄₄H₇₈O₁₆N₁₄Si und Dimethyl bis(octaalanyl)silylester C₅₀H₈₈O₁₈N₁₆Si zeigen eine Wirkung auf Mäuse, Osteosarkom bzw. Adenokarzinome. Die Verbindungen inhibieren auch die Vermehrung von Medulloblastom-Zellen und Zellen des Basatzellkarzinoms. Wenn die Verbindungen zur Bildung von Vesikel verwendet werden, die Foscamet oder Oxoplatin umfassen, ist eine stärkere Wirkung auf Angiosarkome nachweisbar als bei einer Verwendung von Oxoplatin oder Foscamet ohne die Vesikel bzw. mit Phospholipid-Vesikel (50 Gew.% Phosphatidylcholin und 50 Gew.% Cholesterol).

Die Verbindungen Diethyl bis(decagtycyl)silylester C₄₄H₇₂O₂₂N₂₀Si, Dipropyl bis(triglycyl)silylester C₁₈H₃₄O₈N₆Si, Dipropyl bis(tetraglycyl)silylester C₂₂H₄₀O₁₀N₈Si und Dipropyl bis(pentaglycyl)silylester C₂₆H₄₆O₁₂N₁₀Si zeigen eine Wirkung auf Nephroblastome und Hepatobfastome. Wiederum werden Versuche durchgeführt, bei denen Vesikel aus den genannten Verbindungen bereitgestellt werden, die CH₃AsO(OH)₂, HAsO₂ und/oder H₃[As(OH)₆] enthalten.

Die Verbindungen Di(undecanoyloxy)dimethylsilan C₂₄H₄₈O₄Si, Di(tridecanoyloxy)dimethytsilan C₂₈H₅₆O₄Si, Di(pentadecanoyloxy)dimethylsilan C₃₂H₆₄O₄Si, Di(heptadecanoyloxy)dimethylslan C₃₆H₇₂O₄Si, Di(octanoyloxy)diethylsilan C₂₀H₄₀O₄Si, Di(nonanoyloxy)diethyisilan C₂₂H₄₄O₄Si, Di(decanoyloxy)diethylsilan C₂₄H₄₈O₄Si und Di(undecanoyloxy)diethylsilan C₂₆H₅₂O₄Si bzw. Vesikel, die aus diesen Verbindungen gebildet werden, zeigen eine Wirkung gegen die Metastasierung von Plattenepihtelkarzinomen oder Plasmozytomen in Ratten, wobei diese Wirkung verbessert werden kann, wenn die Vesikel Oxoplatin oder Foscamet beinhalten. Vergleichbare Vesikel auf Phospholipid-Basis haben eine geringere Wirkung.

Die Verbindungen Di(dodecanoyloxy)diisopropylsilan C₃₀H₆₀C₄Si, Di(tridecanoyloxy)diisopropylsilan C₃₂H₆₄O₄Si, Di(tetradecanoyloxy)diisopropylsilan C₃₄H₆₈O₄Si, Di(pentadecanoyloxydiisopropylsilan C₃₆H₇₂O₄Si und Di(hexadecanoyloxy)diisopropylsilan C₃₈H₇₆O₄Si sowie Vesikel, die aus diesen gebldet werden, haben eine Wirkung gegen Adenokarzinom-Gewebe, insbesondere eine inhibierende Wirkung auf die Metastasierung. Diese Wirkung ist bei siliziumhaltigen Glykolipiden, die mit anderen Verbindungen als der gemäß der allgemeinen Formel I hergestellt werden, bei allen getesteten Krankheiten nicht nachweisbar.

Die Verbindungen Di(nonanoyloxy)diisopropylsilan C₂₄H₄₈O₄Si, Di(decanoyloxy)diisopropylsilan C₂₆H₅₂O₄Si und Di(undecanoyloxy)diisopropysilan C₂₈H₅₆O₄Si und Vesikel, die aus diesen gebildet werden, zeigen eine antimetastasierende Wirkung bei Osteosarkomen in Ratten.

Die Verbindungen Diisopropyl bis(hexaalanyl)silylester C₄₂H₇₆O₁₄N₁₂Si, Diisopropyl bis(heptaalanyl)silylester C₄₈H₈₆O₁₆N₁₄Si und Diisopropyl bis(octaalanyl)silylester C₅₄H₉₆O₁₈N₁₆Si und Vesikel, die aus diesen gebildet werden, zeigen eine Wirkung auf Osteosarkome.

Die Verbindungen Dimethyl bis(heptaphenylalanyl) silylester C₁₂₈H₁₃₄O₁₆N₁₄Si, Dimethyl bis(octaphenylalanyl)silylester C₁₄₆H₁₅₂O₁₈N₁₆Si, Dimethyl bis(nonaphenylalanyl)silylester C₁₆₄H₁₇₀O₂₀N₁₈Si, Dimethyl bis(decaphenylalanyl)silylester C₁₈₂H₁₈₈O₂₂N₂₀Si, Diethyl bis(biphenylalanyl)silylester C₅₈H₆₆O₈N₆Si, Diethyl bis(tetraphenylalnyl)silylester C₇₆H₈₄O₁₀N₈Si und Diethyl bis(pentaphenylalanyl)silylester C₉₄H₁₀₂O₁₂N₁₀Si und Vesikel, die aus diesen gebildet werden, zeigen eine Wirkung auf Lymphome.

Die Verbindungen Dimethyl bis(nonavalyl)silylester C₉₂H₁₇₀O₂₀N₁₈Si, Dimethyl bis(decavalyl)silylester C₁₀₂H₁₈₈O₂₂N₂₀Si, Diethyl bis(trivalyl)silylester C₃₄H₈₆O₈N₆Si, Diethyl bis(tetravalyl)silylester C₄₄H₈₄O₁₀N₈Si, Diethyl bis(pentavalyl)silylester C₅₄H₁₀₂O₁₂N₁₀Si, Diethyl bis(hexavalyl)silylester C₆₄H₁₂₀O₁₄N₁₂Si und Diethyl bis(heptavalyl)silylester C₇₄H₁₃₈O₁₆N₁₄Si und Vesikel, die aus diesen gebildet werden, zeigen eine Wirkung auf Seminome.

Die Verbindungen Diisopropyl bis(nonaalanyl)silylester C₆₀H₁₀₆O₂₀N₁₈Si, Diisopropyl bis(decaalanyl)silylester C₆₆H₁₁₆O₂₂N₂₀Si, Dipropyl bis(tetraalanyl)silylester C₃₀H₅₆ON₈Si, Dipropyl bis(pentaalanyl)silylester C₃₆H₆₆O₁₂N₁₀Si, Dipropyl bis(hexaalanyl)silylester C₄₂H₇₆O₁₄N₁₂Si, Dipropyl bis(heptaalanyl)silylester C₄₈H₈₆O₁₆N₁₄Si, Dipropyl bis(octaalanyl)silylester C₅₄H₉₆O₁₈N₁₆Si und Dipropyl bis(nonaalanyl)silylester C₆₀H₁₀₆O₂₀N₁₈Si und Vesikel, die aus diesen gebildet werden, zeigen eine Wirkung gegen Plattenepithelkarzinome.

Die Verbindungen Diphenyl bis(decaalanyl)silylester C₅₈H₈₄O₂₂N₂₀Si, Diphenyl bis(trivalyl)silylester C₄₂H₆₆O₈N₆Si, Diphenyl bis(tetravalyl)silylester C₄₆H₇₂ON₈Si, Diphenyl bis(pentavalyl)silylester C₅₀H₇₈O₁₂N₁₀Si, Diphenyl bis(hexavalyl)silylester C₅₄H₈₄O₁₄N₁₂Si, Diphenyl bis(heptavalyl)silylester C₅₈H₉₀O₁₆N₁₄Si, Diphenyl bis(octavalyl)silylester C₆₂H₉₆O₁₈N₁₆Si und Diphenyl bis(nonavalyl)silylester C₆₆H₁₀₂O₂₀N₁₈Si und Vesikel, die aus diesen gebildet werden, zeigen eine Wirkung gegen Angiosarkome.

Die Verbindungen Diphenyl bis(triglycyl)silylester C₂₄H₃₀O₈N₆Si, Diphenyl bis(tetraglycyl)silylester C₂₈H₃₆O₁₀NeSi, Diphenyl bis(pentaglycyl)silylester C₃₂H₄₂O₁₂N₁₀Si, Diphenyl bis(hexaglycyl)silylester C₃₆H₄₈O₁₄N₁₂Si, Diphenyl bis(heptaglycyl)silylester C₄₀H₅₄O₁₆N₁₄Si, Diphenyl bis(octaglycyl)silylester C₄₄H₆₀O₁₈N₁₆Si, Diphenyl bis(nonaglycyl)silylester C₄₈H₆₆O₂₀N₁₈Si und Diphenyl bis(decaglycyl)silylester C₅₂H₇₂O₂₂N₂₀Si und Vesikel, die aus diesen gebildet werden, zeigen eine Wirkung auf Plasmozytome.

Die Verbindungen Dipropyl bis(nonavalyl)silylester C₉₆H₁₇₈O₂₀N₁₈Si, Dipropyl bis(decavalyl)silylester C₁₀₆H₁₉₆O₂₂N₂₀Si, Dimethyl bis(triphenylalanyl)silylester C₅₆H₆₂O₈N₆Si, Dimethyl bis(tetraphenylalanyl)slylester C₇₄H₈₀O₁₀N₈Si, Dimethyl bis(pentaphenylalanyl)silylester C₉₂H₉₈O₁₂N₁₀Si und Dimethyl bis(hexaphenylalanyl)silylester C₁₁₀H₁₁₆O₁₄N₁₂Si und Vesikel, die aus diesen gebildet werden, zeigen eine Wirkung auf Basalzellkarzinome.

Die Verbindungen Dimethyl bis(nonaalanyl)silylester C₅₆H₉₈0₂₀N₁₈Si, Dimethyl bis(decaalanyl)siylester C₆₂H₁₀₈0₂₂N₂₀Si, Diethyl bis(trialanyl)silylester C₂₂H₄₂0₈N₆Si, Diethyl bis(tetraatanyl)saylester C₂₈H₅₂O₁₀N₈Si, Diethyl bis(pentaalanyl)silylester C₃₄H₆₂O₁₂N₁₀Si, Diethyl bis(hexaalanyl)silylester C₄₀H₇₂O₁₄N₁₂Si und Diethyl bis(heptaalanyl)silylester C₄₆H₈₂O₁₆N₁₄Si und Vesikel, die aus diesen gebildet werden, zeigen eine Wirkung auf Adenokarzinome.

Die Verbindungen Dimethyl bis(nonaglycyl)silylester C₃₈H₆₂ON₂₀N₁₈Si, Dimethyl bis(decaglycyl)silylester C₄₂H₆₈O₂₂N₂₀Si, Diethyl bis(triglycyl)silylester C₁₆H₃₀O₈N₆Si und Diethyl bis(tetraglycyl)silylester C₂₀H₃₆O₄N₈Si und Vesikel, die aus diesen gebildet werden, zeigen eine Wirkung gegen Medulloblastome.

Die Verbindungen Di(octanoyloxy)dimethylsilan C₁₈H₃₆O₄Si, Di(decanoyloxy)dimethylsilan C₂₂H₄₄O₄Si, Di(dodecanoyloxy) dimethylslan C₂₆H₅₂O₄Si, Di(tetradecanoyloxy) dimethylsilan C₃₀H₆₀O₄Si, Di(hexadecanoyloxy)dimethylslan C₃₄H₆₈OSi, Di(octadecanoloxy)dimethylsilan C₃₈H₇₆O₄Si und Di(nonanoyloxy)dimethylsilan C₂₀H₄₀O₄Si und Vesikel, die aus diesen gebildet werden, zeigen eine Wirkung auf Osteosarkome.

Die Verbindungen Di(heptadecanoyloxy)diisopropylsilan C₄₀H₈₀O₄Si, Di(octadecanoyloxy)diisopropylsilan C₂₂H₄₄O₄Si, Di(linoylooxy)dimethylsilan C₃₈H₆₈O₄Si, Di (lino-lenoylooxy)dimethylsilan C₃₈H₆₄O₄Si, Di(oleylooxy) dimethylsilan C₃₈H₇₂O₄Si und Di(linoylooxy)diethylsilan C₄₀H₇₂O₄Si und Vesikel, die aus diesen gebildet werden, zeigen eine Wirkung auf Lymphome.

Die Verbindungen Di(nonanoyloxy)dipropylsilan C₂₄H₄₈O₄Si, Di(decanoyloxy)dipropylsilan C₂₆H₅₂O₄Si, Di(undecanoyloxy) dipropylsilan C₂₈H₅₆O₄Si, Di(dodecanoyloxy)dipropylsilan C₃₀H₆₀O₄Si, Di(tridecanoyloxy)dipropylsilan C₃₂H₆₄O₄Si, Di(tetradecanoyloxy)dipropylsilan C₃₄H₆₈O₄Si und Di(pentadecanoyloxy)dipropylsilan C₃₆H₇₂O₄Si und Vesikel, die aus diesen hergestellt werden, zeigen eine Wirkung gegen Seminome.

Die Verbindungen Diethyl bis(octaalanyl)silylester C₅₂H₉₂O₁₈N₁₆Si, Diethyl bis(nonaalanyl)silylester C₅₈H₁₀₂0₂₀N₁₈Si, Diethyl bis(decaalanyl)silylester C₆₄H₁₁₂O₂₂N₂₀Si, Dipropyl bis(trialanyl)silylester Q₂₄H₄₆0₈N₆Si, Diisopropyl bis(tetraalanyl)silylester C₃₀H₅₅0₁₀N₈Si und Diisopropyl bis(pentaalanyl)silylester C₃₆H₆₆0₁₂N₁₀Si und Vesikel, die aus diesen hergestellt werden, zeigen eine Wirkung auf Plattenepithelkarzinome.

Die Verbindungen Diethyl bis(pentaglycyl)silylester C₂₄H₄₂O₁₂N₁₀Si, Diethyl bis(hexaglycyl)Silylester C₂₄H₄₈O₁₄N₁₂Si, Diethyl bis(heptaglycyl)silylester C₃₂H₅₄O₁₆N₁₄Si, Diethyl bis(octablycyl)silylester C₃₆H₆₀C₁₈N₁₆Si und Diethyl bis(nonaglycyl)silylester C₄₀H₆₆O₂₀N₁₈Si und Vesikel, die aus diesen hergestellt werden, zeigen eine Wirkung auf Angiosarkome.

Die Verbindungen Diphenyl bis(decavalyl)silylester C₇₀H₁₀₈O₂₂N₂₀Si, Diphenyl bis(triphenylalanyl)silylester C₉₄H₉₀O₈N₆Si, Diphenyl bis(tetraphenylalanyl)silylester C₉₈H₉₆O₁₀N₈Si, Diphenyl bis(pentaphenylalanyl)silylester C₁₀₂H₁₀₂O₁₂N₁₀Si, Diphenyl bis(hexaphenylalanyl)silylester C₁₀₆H₁₀₈O₁₄N₁₂Si, Diphenyl bis(heptaphenylalanyl)silylester C₁₁₀H₁₁₄O₁₆N₁₄Si, Diphenyl bis(octaphenylalanyl)silylester C₁₁₄H₁₂₀O₁₈N₁₆Si, Diphenyl bis(nonaphenylalanyl)silylester C₁₁₈H₁₂₆O₂₀N₁₈Si und Diphenyl bis(decaphenylalanyl) silylester C₁₂₂H₁₃₂O₂₂N₂₀Si und Vesikel, die aus diesen gebildet werden, zeigen eine Wirkung gegen Plasmozytome.

Die Verbindungen Di(octanoyloxy)diphenylsilan C₂₈H₄₀O₄Si, Di(decanogoxy)diphenylsilan C₃₂H₄₈O₄Si, Di(dodecanoyloxy)diphenylsilan C₃₆H₅₆O₄Si, Di (tetradecanoyloxy)diphenylsilan C₄₀H₆₄O₄Si, Di(hexadecanoyloxy)diphenylsilan C₄₄H₇₂O₄Si und Di (octadecanoyloxy)diphenylsilan C₄₈H₈₀O₄Si und Vesikel, die aus diesen gebildet werden, zeigen eine Wirkung auf Basalzellkarzinome.

Die Verbindungen Dipropyl bis(decaalanyl)silylester C₆₆H₁₁₆0₂₂N₂₀Si, Dimethyl bis(trivalyl)silylester C₃₂H₆₂O₈N₆Si, Dimethyl bis(tetravalyl)silylester C₄₂H₈₀O₁₀N₈Si, Dimethyl bis(pentavalyl)silylester C₅₂H₉₈O₁₂N₁₀Si, Dimethyl bis(hexavalyl)silylester C₆₂H₁₁₆O₁₄N₁₂Si, Dimethyl bis(heptavalyl)silylester C₇₂H₁₃₄O₁₆N₁₄Si und Dimethyl bis(octavalyl)silylester C₈₂H₁₅₂O₁₆N₁₆Si und Vesikel, die aus diesen hergestellt werden, zeigen eine Wirkung gegen Adenokarzinome.

Die Verbindungen Diethyl bis(hexaphenylalanyl)silylester C₁₁₂H₁₂₀O₁₄N₁₂Si, Diethyl bis(heptaphenylalanyl)silylester C₁₃₀H₁₃₈O₁₆N₁₄Si, Diethyl bis(octaphenylalanyl)silylester C₁₄₈H₁₅₆O₁₈N₁₆Si, Diethyl bis(nonaphenylalanyl)silylester C₁₆₈H₁₇₄O₂₀N₁₈Si, Diethyl bis(decaphenylalanyl)silylester C₁₈₄H₁₉₂O₂₂N₂₀Si, Dipropyl bis(triphenylalanyl)silylester C₆₀H₇₀O₈N₆Si, Dipropyl bis(tetraphenylalanyl)silylester C₇₈H₈₈O₁₀N₈Si und Dipropyl bis(pentaphenylalanyl)silylester C₉₆H₁₀₆O₁₂N₁₀Si und Vesikel, die aus diesen gebildet werden, zeigen eine Wirkung auf Medulloblastome.

Die Verbindungen Diisopropyl bis(tetraglycyl)silylester C₂₂H₄₀O₁₀N₈Si, Diisopropyl bis(pentaglycyl)silylester C₂₆H₄₆O₁₂N₁₀Si, Diisopropyl bis(hexaglycyl)silylester C₃₀H₅₂0₁₄N₁₂Si, Diisopropyl bis(heptaglycyl)silylester C₃₄H₅₈0₁₆N₁₄Si, Disopropyl bis(octaglycyl)silylester C₃₈H₆₄0₁₈N₁₆Si, Disopropyl bis(nonaglycyl)silylester C₄₂H₇₀0₂₀N₁₈Si und Disopropyl bis(decaglycyl)silylester C₄₆H₇₆0₂₂N₂₀Si und Vesikel, die aus diesen gebildet werden, zeigen eine Wirkung gegen Osteosarkome.

Die Verbindungen Di (oleylooxy)diisopropylsilan C₄₂H₈₀O₄Si, Di(linoylooxy)diisopropylsilan C₄₂H₇₆O₄Si und Di(linolenoylooxy) diisopropylsilan C₄₂H₇₂O₄Si und Vesikel, die aus diesen gebildet werden, zeigen eine Wirkung auf Lymphome.

Die Verbindungen Di(linolenoylooxy)diethylsilan C₄₀H₆₈O₄Si, Di (oleylooxy)diethylsilan C₄₀H₇₆O₄Si, Di(linoylooxy)dipropylsilan C₄₂H₇₆O₄Si und Di(linolenoylooxy)dipropylsilan C₄₂H₇₂O₄Si und Vesikel, die aus diesen gebildet werden, zeigen eine Wirkung auf Seminome.

Die Verbindungen Dipropyl bis(hexaglycyl)silylester C₃₀H₅₂0₁₄N₁₂Si, Dipropyl bis(heptaglycyl)silylester C₃₄H₅₈0₁₆N₁₄Si, Dipropyl bis(octaglycyl)silylester C₃₈H₆₄0₁₈N₁₆Si, Dipropyl bis(nonaglycyl)silylester C₄₂H₇₀0₂₀N₁₈Si, Dipropyl bis(decaglycyl)silylester C₄₆H₇₆0₂₂N₂₀Si und Diisopropyl bis(triglycyl)silylester C₁₈H₃₄O₈N₆Si und Veskel, die aus diesen gebildet werden, zeigen eine Wirkung gegen Plattenepithelkarzinome.

Die Verbindungen Di(dodecanoyloxy)dipropylsilan C₃₀H₆₀O₄Si, Di(tridecanoyloxy)dipropylsilan C₃₂H₆₄O₄Si, Di(tetradecanoyloxy)dipropylsilan C₃₄H₆₈O₄Si, Di(penta-decanoyloxy)dipropylsilan C₃₆H₇₂O₄Si, Di(hexadecanoyloxy) dipropylsilan C₃₈H₇₆O₄Si, Di(heptadecanoyloxy)dipropyl-silan C₄₀H₈₀O₄Si, Di(odadecanoyloxy)dipropylsilan C₂₂H₄₄O₄Si und Di(octanoyloxy)diisopropylsilan C₂₂H₄₄O₄Si und Vesikel, die aus diesen gebildet werden, zeigen eine Wirkung auf Angiosarkome.

Die Verbindungen Dipropyl bis(hexaphenylalanyl)silylester C₁₁₄H₁₂₄O₁₄N₁₂Si, Dipropyl bis(heptaphenylalanyl)silylester C₁₃₂H₁₄₂O₁₆N₁₆Si, Dipropyl bis(octaphenylalanyl)silylester C₁₅₀H₁₆₀O₁₈N₁₆Si, Dipropyl bis(nonaphenylalanyl)silylester C₁₆₈H₁₇₈O₂₀N₁₈Si und Dipropyl bis(decaphenylalanyl) silylester C₁₈₆H₁₉₆O₂₂N₂₀Si und Vesikel, die aus diesen gebildet werden, zeigen eine Wirkung gegen Plasmozyten.

Die Verbindungen Di(hexadecanoyloxy)dipropylsilan C₃₈H₇₆O₄Si, Di(heptadecanoyloxy)dipropylsilan C₄₀H₈₀O₄Si, Di(octadecanoyloxy)dipropylsilan C₂₂H₄₄O₄Si und Di(octanoyloxy)diisopropylsilan C₂₂H₄₄O₄Si und Vesikel, die aus diesen gebildet werden, zeigen eine Wirkung auf Basalzellkarzinome.

Die Verbindungen Diethyl bis(octavalyl)silylester C₈₄H₁₅₆O₁₈N₁₆Si, Diethyl bis(nonavalyl)silylester C₉₄H₁₇₄O₂₀N₁₈Si, Diethyl bis(decavalyl)silylester C₁₀₄H₁₉₂O₂₂N₂₀Si, Dipropyl bis(trivalyl)silylester C₃₆H₇₀O₈N₆Si, Dipropyl bis(tetravalyl)silylester C₄₆H₈₈O₁₀N₈Si, Dipropyl bis(pentavalyl)silylester C₅₆H₁₀₆O₁₂N₁₀Si, Dipropyl bis(hexavalyl)silylester C₆₆H₁₂₄O₁₄N₁₂Si, Dipropyl bis(heptavalyl)silylester C₇₆H₁₄₂O₁₆N₁₄Si und Dipropyl bis(octavalyl)silylester C₈₆H₁₆₀O₁₈N₁₆Si und Vesikel, die aus diesen gebildet werden, zeigen eine Wirkung auf Adenokarzinome.

Die Verbindungen Di(oleylooxy)diisopropylsilan C₄₂H₈₀O₄Si, Dimethyl bis(triglycyl)silylester C₁₄H₂₆O₄Si, Dimethyl bis(tetraglycyl)silylester C₁₈H₃₂O₁₀N₈Si, Dimethyl bis(pentaglycyl)silylester C₂₂H₃₈O₁₂N₁₀Si, Dimethyl bis(hexaglycyl)silylester C₂₆H₄₄O₁₄N₁₂Si, Dimethyl bis(heptaglycyl)silylester C₃₀H₅₀O₁₆N₁₄Si und Dimethyl bis(octaglycyl)silylester C₃₄H₅₆O₁₈N₁₆Si und Vesikel, die aus diesen gebildet werden, zeigen eine Wirkung gegen Medulloblastome.

Die Verbindungen Di(nonanoyloxy)diphenylsilan C₃₀H₄₄O₄Si, Di(undecanoyloxy)diphenylsilan C₃₄H₅₂O₄Si, Di(trideca-noyloxy)diphenylsilan C₃₈H₆₀O₄Si, Di(pentadecanoyloxy)diphenylsilan C₄₂H₆₈O₄Si, Di(heptadecanoyloxy)diphenylsilan C₄₆H₇₆O₄Si, Di (linoylooxy)diphenylsilan C₄₈H₇₂O₄Si, Di(linolenoylooxy)diphenylsilan C₄₈H₆₈O₄Si und Di(oleylooxy)diphenylsilan C₄₈H₇₆O₄Si und Vesikel, die aus diesen gebildet werden, zeigen eine Wirkung gegen Osteosarkome.

Die Verbindungen Diphenyl bis(trialanyl)silylester C₃₀H₄₂O₈N₆Si, Diphenyl bis(tetraalanyl)silylester C₃₄H₄₈O₁₀N₈Si, Diphenyl bis(pentaalanyl)silylester C₃₈H₅₄O₁₂N₁₀Si, Diphenyl bis(hexaalanyl)silylester C₄₂H₆₀0₁₄N₁₂Si, Diphenyl bis(heptaalanyl)silylester C₄₆H₆₆O₁₆N₁₄Si, Diphenyl bis(octaalanyl)silylester C₅₀H₇₂O₁₈N₁₆Si und Diphenyl bis(nonaalanyl)silylester C₅₄H₇₈O₂₀N₁₈Si und Vesikel, die aus diesen gebildet werden, zeigen eine Wirkung auf Lymphome.

Vergleichbare Phospholipid-Vesikel haben diese Wirkungen nicht. Auch bei dem Vergleich von Phospholipid-Vesikeln, die Foscamet, Oxoplatin oder CH₃AsO(OH)₂, HAsO₂ und/oder H₃[As(OH)₆] umfassen - d. h., dass diese genannten Antikrebsmittel, Antivirus- oder antibakteriellen Verbindungen von Phospholipid-Vesikeln umschlossen sind -, zeigt sich, dass verbesserte Resultate erzielt werden, wenn statt der Phospholipid-Vesikel Vesikel verwendet werden, die aus den oben genannten langkettigen siliziumhaltigen Glykolipiden gebildet werden.

Die Verbindungen Di(oleylooxy)diisopropylsilan C₄₂H₆₀O₄Si, Di(linoylooxy)diisopropylsilan C₄₂H₇₆O₄Si und Di(linolenoylooxy) diisopropylsilan C₄₂H₇₂O₄Si zeigen eine Wirkung gegen HIV-Vren, insbesondere wenn die Verbindungen Vesikel bilden, die Antisense-Konstrukte umfassen, die spezifisch mit HIV-Viren interagieren.

Die Verbindungen Di(linolenoylooxy)diethysilan C₄₀H₆₈O₄Si, Di (oleylooxy)diethylsilan C₄₀H₇₆O₄Si, Di(linoylooxy)dipropylsilan C₄₂H₇₆O₄Si und Di(linolenoylooxy)dipropylsilan C₄₂H₇₂O₄Si zeigen eine Wirkung gegen Hepatitis C-Viren, insbesondere wenn die Verbindungen Vesikel bilden, die Antisense-Konstrukte umfassen, die spezifisch mit Hepatitis C-Viren interagieren.

Die Verbindungen Dipropyl bis(hexaglycyl)silylester C₃₀H₅₂0₁₄N₁₂Si, Dipropyl bis(heptaglycyl)silylester C₃₄H₅₈0₁₆N₁₄Si, Dipropyl bis(octaglycyl)silylester C₃₈H₆₄O₁₈N₁₆Si, Dipropyl bis(nonaglycyl)silylester C₄₂H₇₀0₂₀N₁₈Si, Dipropyl bis(decaglycyl)silylester C₄₆H₇₆0₂₂N₂₀Si und Diisopropyl bis(triglycyl)silylester C₁₈H₃₄O₈N₆Si zeigen eine Wirkung gegen Influenza-Viren, insbesondere wenn die Verbindungen Vesikel bilden, die Antisense-Konstrukte umfassen, die spezifisch mit Influenza-Viren interagieren.

Die Verbindungen Di(dodecanoyloxy)dipropylsilan C₃₀H₆₀O₄Si, Di(tridecanoyloxy)dipropylsilan C₃₂H₆₄O₄Si, Di(tetradecanoyloxy)dipropylsilan C₃₄H₆₈O₄Si, Di(penta-decanoyloxy)dipropylsilan C₃₆H₇₂O₄Si, Di(hexadecanoyloxy) dipropylsilan C₃₈H₇₆O₄Si, Di(heptadecanoyloxy)dipropyl-silan C₄₀H₈₀O₄Si, Di(octadecanoyloxy)dipropylsilan C₂₂H₄₄O₄Si und Di(octanoyloxy)diisopropylsilan C₂₂H₄₄O₄Si zeigen eine Wirkung gegen Hepatitis B-Viren, insbesondere wenn die Verbindungen Vesikel bilden, die Antisense-Konstrukte umfassen, die spezifisch mit Hepatitis B-Viren interagieren.

Die Verbindungen Dipropyl bis(hexaphenylalanyl)silylester C₁₁₄H₁₂₄O₁₄N₁₂Si, Dipropyl bis(heptaphenylalanyl)slylester C₁₃₂H₁₄₂O₁₆N₁₆Si, Dipropyl bis(octaphenylalanyl)silylester C₁₅₀H₁₆₀O₁₈N₁₆Si, Dipropyl bis(nonaphenylalanyl)silylester C₁₆₈H₁₇₈O₂₀N₁₈Si und Dipropyl bis(decaphenylalanyl) silylester C₁₈₆H₁₉₆O₂₂N₂₀Si zeigen eine Wirkung gegen Hepatitis A-Viren, insbesondere wenn die Verbindungen Vesikel bilden, die Antisense-Konstrukte umfassen, die spezifisch mit Hepatitis A-Viren interagieren.

Die Verbindungen Di(hexadecanoyloxy)dipropylsilan C₃₈H₇₆O₄Si, Di(heptadecanoyloxy)dipropylsilan C₄₀H₈₀O₄Si, Di(octadecanoyloxy)dipropylsilan C₂₂H₄₄O₄Si und Di(octanoyloxy)diisopropylsilan C₂₂H₄₄O₄Si zeigen eine Wirkung gegen Sendai-Viren, insbesondere wenn die Verbindungen Vesikel bilden, die Antisense-Konstrukte umfassen, die spezifisch mit Sendai-Viren interagieren.

Die Verbindungen Diethyl bis(octavalyl)silylester C₈₄H₁₅₆O₁₈N₁₆Si, Diethyl bis(nonavalyl)silylester C₉₄H₁₇₄O₂₀N₁₈Si, Diethyl bis(decavalyl)silylester C₁₀₄H₁₉₂O₂₂N₂₀Si, Dipropyl bis(trivalyl)silylester C₃₆H₇₀O₈N₆Si, Dipropyl bis(tetravalyl)silylester C₄₆H₈₈O₁₀N₈Si, Dipropyl bis(pentavalyl)silylester C₅₆H₁₀₆O₁₂N₁₀Si, Dipropyl bis(hexavalyl)silylester C₆₆H₁₂₄O₁₄N₁₂Si, Dipropyl bis(heptavalyl)silylester C₇₆H₁₄₂O₁₆N₁₄Si und Dipropyl bis(octavalyl)silylester C₈₆H₁₆₀O₁₈N₁₆Si zeigen eine Wirkung gegen Herpes-Viren, insbesondere wenn die Verbindungen Vesikel bilden, die Antisense-Konstrukte umfassen, die spezifisch mit Herpes-Viren interagieren.

Die Verbindungen Di(oleylooxy)diisopropylsilan C₄₂H₈₀O₄Si, Dimethyl bis(triglycyl)silylester C₁₄H₂₆O₄Si, Dimethyl bis(tetraglycyl)silylester C₁₈H₃₂O₁₀N₈Si, Dimethyl bis(pentaglycyl)silylester C₂₂H₃₈O₁₂N₁₀Si, Dimethyl bis(hexaglycyl)silylester C₂₆H₄₄O₁₄N₁₂Si, Dimethyl bis(heptaglycyl)silylester C₃₀H₅₀O₁₆N₁₄Si und Dimethyl bis(octaglycyl)silylester C₃₄H₅₆O₁₈N₁₆Si zeigen eine Wirkung gegen Entero-Viren, insbesondere wenn die Verbindungen Vesikel bilden, die Antisense-Konstrukte umfassen, die spezifisch mit Entero-Viren interagieren.

Die Verbindungen Di(nonanoyloxy)diphenylsilan C₃₀H₄₄O₄Si, Di(undecanoyloxy)diphenylsilan C₃₄H₅₂O₄Si, Di(trideca-noyloxy)diphenylsilan C₃₈H₆₀O₄Si, Di(pentadecanoyloxy) diphenylsilan C₄₂H₆₈O₄Si, Di(heptadecanoyloxy) diphenylsilan C₄₆H₇₆O₄Si, Di (linoylooxy)diphenylsilan C₄₈H₇₂O₄Si, Di(linolenoylooxy)diphenylsilan C₄₈H₆₈O₄Si und Di(oleylooxy)diphenylsilan C₄₈H₇₆O₄Si zeigen eine Wirkung gegen Rhino-Viren, insbesondere wenn die Verbindungen Vesikel bilden, die Antisense-Konstrukte umfassen, die spezifisch mit Rhino-Viren interagieren.

Die Verbindungen Diphenyl bis(trialanyl)silylester C₃₀H₄₂O₈N₆Si, Diphenyl bis(tetraalanyl)silylester C₃₄H₄₈O₁₀N₈Si, Diphenyl bis(pentaalanyl)silylester C₃₈H₅₄O₁₂N₁₀Si, Diphenyl bis(hexaalanyl)silylester C₄₂H₆₀0₁₄N₁₂Si, Diphenyl bis(heptaalanyl)silylester C₄₆H₆₆O₁₆N₁₄Si, Diphenyl bis(octaalanyl)silylester C₅₀H₇₂O₁₈N₁₆Si und Diphenyl bis(nonaalanyl)silylester C₅₄H₇₈O₂₀N₁₈Si zeigen eine Wirkung gegen Hepatitis-Viren, insbesondere wenn die Verbindungen Vesikel bilden, die Antisense-Konstrukte umfassen, die spezifisch mit Hepatitis-Viren interagieren. Die Verwendung der konkreten Verbindungen ist aber nicht auf die genannten Krankheiten beschränkt, sondern sie können bei allen weiteren genannten Erkrankungen verwendet werden. Wenn die Antisense-Konstrukte in übliche Phospholipid-Vesikel eingeschlossen werden, ist die Wirkung gegen die genannten Viren überraschend geringer.

## Patentansprüche

1. Verwendung von Tetraorganosilizium-Verbindungen der allgemeinen Formel I 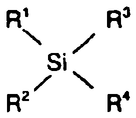 in der R¹, R², R³ und/oder R⁴, die gleich oder verschieden sein können oder Teile eines zwei- oder mehrzähnigen Liganden, durch den ein Chelatkomplex gebildet wird, jeweils Alkylreste, Alkoxyreste, Acyloxyreste der Formel R-COO-, Glukosidreste wie Mono-, Di-, Polysaccaride oder einen Peptidrest der nachstehenden Formel 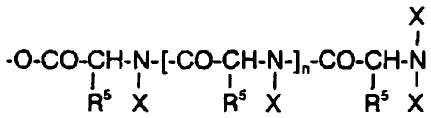 bedeuten, wobei R⁵ einen unverzweigten oder verzweigten Alkyl-, Alkenyl-, Alkinylrest, Arylrest oder Heterocyclus oder einen cyclischen oder aliphatischen Rest oder einen heterocyclischen oder heteroaliphatischen Rest bedeutet, der durch ein bis drei Halogenatome, eine oder mehrere Hydroxygruppen, eine oder mehrere Carboxyl- oder Carboxylatgruppen, Alkoxyreste, Aminogruppen, die substituiert oder unsubstituiert sind oder als Ammoniumsalz vorliegen, eine oder mehrere Ketogruppen, Aldehydgruppen, eine oder mehrere Estergruppen substituiert sein kann, die Reste R⁵, die gleich oder verschieden sein können, den nach Entfernung der Gruppe 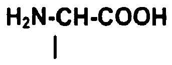 von einer in der Natur vorkommenden Aminosäure und/oder synthetisierte Aminosäure verbleibenden Rest darstellen, die Reste X, die gleich oder verschieden sein können, ein Wasserstoffatom oder eine in der Peptidchemie übliche Aminoschutzgruppe darstellen und n eine ganze Zahl von 0 bis 12 bedeutet, zur Herstellung eines Medikaments zur Behandlung von Tumor-, Bakterien- und/oder Viruserkrankungen.

2. Verwendung nach Anspruch 1, einer Verbindung der allgemeinen Formel I 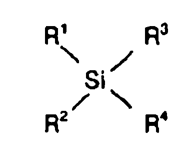 in der R¹ und R², die gleich oder verschieden sein können, jeweils einen Acyloxyrest der Formel R-COO- oder einen Peptidrest der Formel 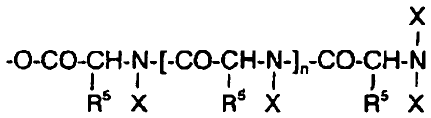 bedeuten, wobei R einen unverzweigten oder verzweigten Alkyl-, Alkenyl- oder Alkinylrest mit 5 bis 29 C-Atomen bedeutet, die durch ein bis drei Halogenatome, Alkoxyreste mit 1 bis 18 C-Atomen oder Aminogruppen substituiert sein können, die Reste R⁵, die gleich oder verschieden sein können, den nach der Entfernung der Gruppe 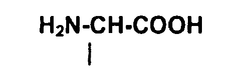 von einer in der Natur vorkommenden Aminosäure verbleibenden Rest darstellen, die Reste X, die gleich oder verschieden sein können, ein Wasserstoffatom oder eine in der Peptidchemie übliche Aminoschutz-gruppe darstellen und n eine ganze Zahl von 1 bis 12 bedeutet, und R³ und R⁴, die gleich oder verschieden sein können, jeweils einen Alkylrest mit 1 bis 6 C-Atomen, eine Arylgruppe mit 3 bis 14 C-Atomen, beispielsweise eine Phenylgruppe, den nach Entfernung eines Wasserstoffatoms verbleibenden Rest eines Monosaccharids, Disaccharids, Aminozuckers oder einer Hydroxycarbonsäure, einen Alkoxyrest mit 1 bis 5 C-Atomen, einen Acylrest, einer in der Natur vorkommenden Aminosäure mit freier oder geschützter Aminogruppe oder einen Di-, Tri- oder Tetrapeptidrest aus einer in der Natur vorkommenden Aminosäure mit freien oder geschützten Aminogruppen darstellen oder die Bedeutung R¹ und R² haben, wobei R einen unverzweigten oder verzweigten Alkyl-, Alkenyl-, oder Alkinylrest darstellt, zur Herstellung eines Medikaments zur Behandlung von Tumor-, Bakterien- und/oder Viruserkrankungen.

3. Verwendung nach einem der vorhergehenden Ansprüche,
**dadurch gekennzeichnet, dass**
die Erkrankungen in Zusammenhang mit pathogenen Modifikationen und/oder Defekten der zellulären Immunität stehen, insbesondere Krebs, Sepsis oder allergische Reaktionen im Zusammenhang mit einer Zytostatika-, Chemo- und/oder Strahlentherapie.

4. Verwendung nach einem der vorhergehenden Ansprüche,
**dadurch gekennzeichnet, dass**
die Behandlung von Tumorerkrankungen eine Hemmung oder Verhinderung eines Tumorwachstums, einer Tumorausbreitung, einer Tumor-Angiogenese, einer Tumor-Invasion, einer Tumor-Infiltration und/oder einer Tumor-Metastasierung ist.

5. Verwendung nach einem der vorhergehenden Ansprüche,
**dadurch gekennzeichnet, dass**
die Behandlung von Tumor-, Bakterien- und/oder Viruserkrankungen zur Aktivierung von Thymozytenpopulationen oder zur Freisetzung von Zytokinen und/oder Interleukinen führt.

6. Verwendung nach einem der vorhergehenden Ansprüche,
**dadurch gekennzeichnet, dass**
die Verbindungen der allgemeinen Formel gemäß 1 und/oder Vesikel die diese umfassen, mit einem Antisensemolekül, Oxoplatin, Arsenoxide und/oder Foscarnet assoziiert sind.

7. Verwendung nach einem der vorhergehenden Ansprüche,
**dadurch gekennzeichnet, dass**
die Verbindungen der allgemeinen Formel gemäß des Anspruch 1 ausgewählt sind aus der Gruppe umfassend Di(dodecanoyloxy)diphenylsilan, Di(decanoyloxy)diphenylsilan, Di(hexadecanoyloxy)diphenylsilan, Di(octadecanoyloxy)diphenylsilan, Di(octanoyloxy)diphenylsilan,
Di(tetradecanoyloxy)diphenylsilan, Di(dodecanoyloxy)dimethylsilan, Di(decanoyloxy)dimethylsilan, Di(hexadecanoyloxy)dimethylsilan, Di(octadecanoyloxy)dimethylsilan, Di(tetradecanoyloxy)dimethylsilan, Di (heptadecanoyloxy) diethylsilan, Di (undecanoyloxy) dimethylsilan, Di (tridecanoyloxy) dimethylsilan, Di (undecanoyloxy) diethylsilan, Di (pentadecanoyloxy) diphenylsilan, Di (tridecanoyloxy) diethylsilan, Di (tridecanoyloxy) diphenylsilan, DPS - C10 = Didecanoyloxy - diethylsilan, Di (hexadecanoyloxy) diethylsilan, Di (decanoyloxy) diethylsilan, Di (octadecanoyloxy) diethylsilan und/oder Di (tetradecanoyloxy) diethylsilan.

8. Verwendung nach einem der vorhergehenden Ansprüche,
**dadurch gekennzeichnet, dass**
die Behandlung eine prophylaktische und/oder eine therapeutische Behandlung ist.

9. Verwendung nach einem der vorhergehenden Ansprüche,
**dadurch gekennzeichnet, dass**
die Verbindungen gemäß der allgemeinen Formel I die Blut-/Hirnschranke überwinden.

10. Verwendung nach einem der vorhergehenden Ansprüche,
**dadurch gekennzeichnet, dass**
die Verbindungen gemäß der allgemeinen Formel I nach Anspruch 1 oder 2 eine immunmodulatorische Aktivität aufweisen.

11. Verwendung nach einem der vorhergehenden Ansprüche,
**dadurch gekennzeichnet, dass**
die Verbindungen gemäß Anspruch 1 oder 2 in Vesikel-Form vorliegen.

## Claims

1. Use of tetra-organosilicon compounds of the general formula I 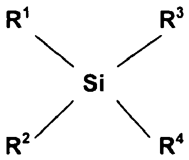 wherein R¹, R², R³ and/or R⁴, which may be the same or different or may be part of a twodentate or polydentate ligand, by which a chelate complex is formed, each represent alkyl radicals, alkoxy radicals, acyloxy radicals of the formula R-COO-, glycoside radicals such as monosaccharides, disaccharides, polysaccharides or a peptide radical of the following formula 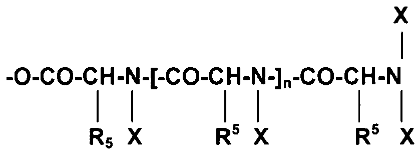 wherein R⁵ represents a non-branched or branched alkyl radical, alkenyl radical, alkinyl radical, aryl radical or heterocyle or a cyclic or aliphatic radical or a heterocyclic or heteroaliphatic radical, which may be substituted by one to three halogen atoms, one or several hydroxyl groups, one or several carboxyl or carboxylate groups, alkoxy radicals, amino groups, which are substituted or unsubstituted or exist in the form of ammonium salt, one or several keton groups, aldehyde groups, one or several ester groups, the R⁵ radicals, which can be the same or different, that represent the remaining residue depending on the distance from the group 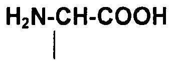 of a naturally occurring amino acid and/or of on a synthesized amino acid, the X radicals, which can be the same or different, that represent a hydrogen atom or an amino protecting group common in peptide chemistry, and n represents a whole number of 1 to 12, for the production of a drug for the treatment of tumours, bacterial and/or viral diseases.

2. Use in accordance with claim 1 of a compound of the general formula I 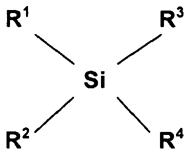 wherein R¹ and R², which can be the same or different, each represent an acyloxy radical of the formula R-COO- or a peptide radical of the formula 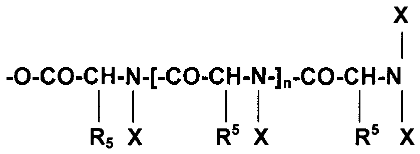 wherein R represents a non-branched or branched alkyl radical, alkenyl radical or alkinyl radical with 5 to 29 atoms of C which may be substituted by one to three halogen atoms, alkoxy radicals with 1 to 18 atoms of C or amino groups, the R⁵ radicals, which can be the same or different, that represent the remaining residue depending on the distance from the group 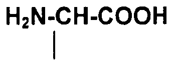 of a naturally occurring amino acid, the X radicals, which can be the same or different, that represent a hydrogen atom or an amino protecting group common in peptide chemistry, and n represents a whole number of 0 to 12, and R³ and R⁴, which can be the same or different, that each represent an alkyl radical with 1 to 6 atoms of C, an aryl group with 3 to 14 atoms of C, for example a phenyl group, the remaining residue depending on the distance from a hydrogen atom of a monosaccharide, disaccharide, amino sugar or a hydroxycarboxylic acid, an alkoxy radical with 1 to 5 atoms of C, an acyl radical, a naturally occurring amino acid with an unprotected or protected amino group or a dipeptide, tripeptide or tetrapeptide radical of a naturally occurring amino acid with unprotected or protected amino groups or have the meaning of R¹ and R², wherein R represents a non-branched or branched alkyl radical, alkenyl radical, alkinyl radical for the production of a drug for the treatment of tumours, bacterial and/or viral diseases.

3. Use in accordance with one of the preceding claims,
**characterised in that**
the diseases are associated with pathogenic modifications and/or defects of the cellular immunity, in particular cancer, sepsis or allergic reactions associated with a cytostatic therapy, chemotherapy and/or radiotherapy.

4. Use in accordance with one of the preceding claims,
**characterised in that**
the treatment of tumours is an inhibition or prevention of a tumour growth, a tumour spread, a tumour angiogenesis, a tumour invasion, a tumour infiltration and/or a tumour metastasis.

5. Use in accordance with one of the preceding claims,
**characterised in that**
the treatment of tumours, bacterial and/or viral diseases results in the activation of thymocyte populations or in the release of cytokines and/or interleukins.

6. Use in accordance with one of the preceding claims,
**characterised in that**
the compounds of the general formula pursuant to 1 and/or vesicles comprising such compounds are associated with an antisense molecule, Oxoplatin, arsenic oxides and/or Foscarnet.

7. Use in accordance with one of the preceding claims,
**characterised in that**
the compounds of the general formula pursuant to claim 1 are chosen from the group comprising Di(dodecanoyloxy)diphenylsilan, Di(decanoyloxy)diphenylsilan, Di(hexadecanoyloxy)diphenylsilan, Di(octadecanoyloxy)diphenylsilan, Di(octanoyloxy)diphenylsilan,
Di(tetradecanoyloxy)diphenylsilan, Di(dodecanoyloxy)dimethylsilan, Di(decanoyloxy)dimethylsilan, Di(hexadecanoyloxy)dimethylsilan, Di(octadecanoyloxy)dimethylsilan, Di(tetradecanoyloxy)dimethylsilan, Di(heptadecanoyloxy) diethylsilan, Di(undecanoyloxy) dimethylsilan, Di(tridecanoyloxy) dimethylsilan, Di (undecanoyloxy) diethylsilan, Di(pentadecanoyloxy) diphenylsilan, Di(tridecanoyloxy) diethylsilan, Di(tridecanoyloxy) diphenylsilan, DPS - C10 = Didecanoyloxy - diethylsilan, Di(hexadecanoyloxy) diethylsilan, Di(decanoyloxy) diethylsilan, Di(octadecanoyloxy) diethylsilan and/or Di(tetradecanoyloxy) diethylsilan.

8. Use in accordance with one of the preceding claims,
**characterised in that**
the treatment is a prophylactic and/or a therapeutic treatment.

9. Use in accordance with one of the preceding claims,
**characterised in that**
the compounds enter the blood-brain barrier in accordance with the general formula I.

10. Use in accordance with one of the preceding claims,
**characterised in that**
the compounds have an immunomodulatory activity in accordance with the general formula I pursuant to claim 1 or 2.

11. Use in accordance with one of the preceding claims,
**characterised in that**
the compounds are in a vesicle form in accordance with claim 1 or 2.

## Revendications

1. Utilisation de composés tétraorganiques de silicium de formule générale I 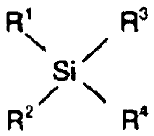 dans laquelle R¹, R², R³ et/ou R⁴, pouvant être identiques ou différents ou faire partie d'un ligand à deux ou plusieurs dents à partir duquel se forme un complexe chélate, représentent des restes alkyles, des restes alcoxyles, des restes acyloxyles de formule R-COO, des restes glucosides tels que monosaccharide, disaccharide, polysaccharide ou un reste peptide de formule 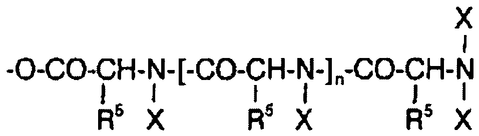 R⁵ représentant un reste alkyle, alcényle, alkinyle, aryle linéaire ou ramifié ou un hétérocycle ou un reste cyclique ou aliphatique ou un reste hétérocyclique ou hétéroaliphatique pouvant être substitué par un ou trois atomes d'halogène, un ou plusieurs groupes hydroxyles, un ou plusieurs groupes carboxyles ou carboxylates, des restes alcoxyles, des groupes d'acides aminés substitués ou non substitués ou présents sous forme de sel d'ammonium, un ou plusieurs groupes cétones, groupes aldéhydes, un ou plusieurs groupes esters, les restes R⁵, pouvant être identiques ou différents, représentant le reste d'un acide aminé présent dans la nature et/ou d'acides aminés synthétisés après retrait du groupe 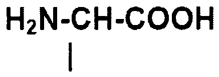 les restes X, pouvant être identiques ou différents, représentant un atome d'hydrogène ou un groupement protecteur d'acides aminés usuel dans la chimie des peptides et n équivalant à un nombre entier de 0 à 12, pour la fabrication d'un médicament destiné au traitement d'affections tumorales, bactériennes et/ou virales.

2. Utilisation selon la revendication 1 d'un composé de formule générale I 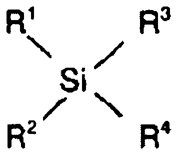 dans laquelle R¹ et R², pouvant être identiques ou différents, représentent un reste acyloxyle de formule R-COO ou un reste peptide de formule 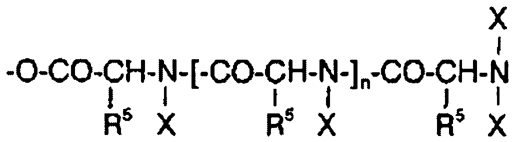 R représentant un reste alkyle, alcényle ou alkinyle linéaire ou ramifié comportant 5 à 29 atomes C pouvant être substitués par un à trois atomes d'halogène, des restes alcoxyles comportant 1 à 18 atomes C ou groupes d'acides aminés, les restes R⁵, pouvant être identiques ou différents, représentant le reste d'un acide aminé présent dans la nature après retrait du groupe 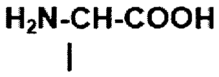 les restes X, pouvant être identiques ou différents, représentant un atome d'hydrogène ou un groupement protecteur d'acides aminés usuel dans la chimie des peptides et n équivalant à un nombre entier de 1 à 12, et R³ et R⁴ pouvant être identiques ou différents, représentant un reste alkyle comportant 1 à 6 atomes C, un groupe aryle comportant 3 à 14 atomes C, par exemple un groupe phényle, le reste d'un monosaccharide, d'un disaccharide, d'un sucre aminé ou d'un acide hydroxycarboxylique après retrait d'un atome d'hydrogène, un reste alcoxyle comportant 1 à 5 atomes C, un reste acyle, un acide aminé présent dans la nature avec un groupe aminé libre ou protégé ou un reste dipeptide, tripeptide ou tétrapeptide extrait d'un acide aminé présent dans la nature avec des groupes aminés libres ou protégés ou ayant la signification R¹ et R², R représentant un reste alkyle, alcényle ou alkinyle linéaire ou ramifié, pour la fabrication d'un médicament destiné au traitement d'affections tumorales, bactériennes et/ou virales.

3. Utilisation selon l'une des revendications précédentes,
caractérisée en ceci que
les affections ont un lien avec des modifications pathogènes et/ou anomalies de l'immunité cellulaire, en particulier le cancer, le spesis ou les réactions allergiques liées à un traitement cytostatique, une chimiothérapie et/ou une thérapie par rayons.

4. Utilisation selon l'une des revendications précédentes,
caractérisée en ceci que
le traitement d'affections tumorales consiste à inhiber ou empêcher une croissance tumorale, une extension tumorale, une angiogénèse tumorale, une invasion tumorale,
une infiltration tumorale et/ou une métastase tumorale.

5. Utilisation selon l'une des revendications précédentes,
caractérisée en ceci que
le traitement d'affections tumorales, bactériennes et/ou virales provoque l'activation de populations de thymocytes ou la libération de cytokines et/ou d'interleukines.

6. Utilisation selon l'une des revendications précédentes,
caractérisée en ceci que
les composés de formule générale selon la renvendication 1 et/ou les vésicules qu'ils renferment sont associés à une molécule antisens, l'oxoplatine, l'oxyde d'arsenic et/ou le foscarnet.

7. Utilisation selon l'une des revendications précédentes,
caractérisée en ceci que
les composés de formule générale selon la revendication 1 sont sélectionnés du groupe comprenant le di(dodécanoyloxy)diphénylsilane, di(décanoyloxy)diphénylsilane, di(héxadécanoyloxy)diphénylsilane, di(octadécanoyloxy)diphénylsilane, di(octanoyloxy)diphénylsilane,
di(tétradécanoyloyxy)diphénylsilane, di(dodécanoyloxy)diméthylsilane, di(décanoyloxy)diméthylsilane, di(héxadécanoyloxy)diméthylsilane, di(octadécanoyloxy)diméthylsilane, di(tétradécanoyloxy)diméthylsilane, di(heptadécanoyloxy)diéthylsilane, di(undécanoyloxy)diméthylsilane, di(tridécanoyloxy)diméthylsilane, di(undécanoyloxy)diéthylsilane, di(pentadécanoyloxy)diphénylsilane, di(tridécanoyloxy)diéthylsilane, di(tridécanoyloxy)diphénylsilane, DPS - C10 = didécanoyloxy - diéthylsilane, di(héxadécanoyloxy)diéthylsilane, di(décanoyloxy)diéthylsilane, di(octadécanoyloxy)diéthylsilane et/ou di(tétradécanoyloxy)diéthylsilane.

8. Utilisation selon l'une des revendications précédentes,
caractérisée en ceci que
le traitement est un traitement prophylactique et/ou thérapeutique.

9. Utilisation selon l'une des revendications précédentes,
caractérisée en ceci que
les composés de formule générale I franchissent la barrière hémato-encéphalique.

10. Utilisation selon l'une des revendications précédentes,
caractérisée en ceci que
les composés de formule générale I selon la revendication 1 ou 2 présentent une activité immunomodulatoire.

11. Utilisation selon l'une des revendications précédentes,
caractérisée en ceci que
les composés selon la revendication 1 ou 2 existent sous forme de vésicules.
